# EUROPEAN PATENT SPECIFICATION

(11) **EP 3 201 327 B1**
(45) Date of publication and mention of the grant of the patent: **03.10.2018**
(21) Application number: 15775198.3
(22) Date of filing: 05.10.2015
(51) Int. Cl.: C12N 9/22, C12N 15/82

(54) **FUNCTIONAL MUTANT ALLELES OF EIN5 FOR YIELD INCREASE**
FUNKTIONELLE MUTANTE ALLELE VON EIN5 ZUR ERTRAGSSTEIGERUNG
ALLÈLES MUTANTS FONCTIONNELS DE L'EIN5 POUR L'AUGMENTATION DU RENDEMENT

(30) Priority: 03.10.2014 EP 14187614
(43) Date of publication of application: 09.08.2017
(73) Proprietor: VIB vzw, 9052 Ghent (BE); Universiteit Gent, 9000 Gent (BE)
(72) Inventor: INZÉ, Dirk, Gustaaf, 9310 Moorsel - Aalst (BE); NELISSEN, Hilde, 9000 Gent (BE); DUBOIS, Marieke, 9840 Zevergem (BE)
(86) International application number: PCT/EP2015/072924
(87) International publication number: WO 2016/050984

(56) References cited:
- WO-A1-2012/143545
- WO-A2-2009/067580
- A. SKIRYCZ ET AL: "Pause-and-Stop: The Effects of Osmotic Stress on Cell Proliferation during Early Leaf Development in Arabidopsis and a Role for Ethylene Signaling in Cell Cycle Arrest", THE PLANT CELL ONLINE, vol. 23, no. 5, 1 May 2011 (2011-05-01), pages 1876-1888, XP055045867, ISSN: 1040-4651, DOI: 10.1105/tpc.111.084160
- GABRIELA OLMEDO ET AL: "ETHYLENE-INSENSITIVE5 encodes a 5' -> 3' exoribonuclease required for regulation of the EIN3-targeting F-box proteins EBF1͞2", PROCEEDINGS OF THE NATIONAL ACADEMY OF SCIENCES OF THE UNITED STATES OF AMERICA, vol. 103, no. 36, 5 September 2006 (2006-09-05), pages 13286-13293, XP055232358, cited in the application

## Description

### Field of the invention

The present invention relates to the field of plant molecular biology. More specifically the present invention provides mutant alleles, chimeric genes comprising said mutant alleles and plants comprising said mutant alleles. Plant comprising said mutant alleles are characterized by having an increase in yield compared to wild type plants.

### Introduction to the invention

Leaf growth is a complex process, integrating genetically programmed processes and environmental signals. These environmental signals can trigger inhibition of leaf growth which under drought stress for example, forms a mechanism to limit water evaporation from the leaf surface. During the past decades, extensive research has been performed on *Arabidopsis thaliana* to study plant behavior under extreme environmental conditions such as severe drought (Xiong et al., 2002; Verslues et al., 2006; Seki et al., 2007; Schachtman and Goodger, 2008). These studies mainly scored plant survival or used final rosette area as a measure for plant tolerance to drought (Umezawa et al., 2006). However, it became clear that the often rather harsh conditions used in these studies are not very representative for natural conditions, in which the drought stress is generally milder and does not always threaten survival (Skirycz and Inzé, 2010). Moreover, it was shown that tolerance to severe drought stress and the ability of plants to continue to grow under mild stress conditions are very different traits mediated by different molecular processes (Skirycz et al., 2011a; Claeys and Inzé, 2013). Furthermore, recent studies pointed out the importance of evaluating stress responses at organ or tissue level, as the response to osmotic stress is highly dependent on the organ and its developmental stage (Dinneny, 2008; Harb et al., 2010; Skirycz et al., 2010; Baerenfaller et al., 2012). Thus, in order to understand the specific mechanisms involved in growth inhibition under water deprivation, it is important to study growth response specifically in actively growing leaves.

Leaf growth in Arabidopsis is consisting of three major developmental phases (Anastasiou and Lenhard, 2007; Andriankaja et al., 2012; Gonzalez et al., 2012). First, growth of very young leaves emerging from the shoot apical meristem is driven exclusively by cell proliferation. Next, cell division starts to cease at the tip of the leaf and gradually all cells in the leaf exit the cell cycle and start to expand (Donnelly et al., 1999; Andriankaja et al., 2012; Gonzalez et al., 2012). Finally, cell division in the developing leaves has stopped and further leaf growth mainly occurs by cell expansion (Vlieghe et al., 2005). Under mild drought conditions, studied *in vitro* using osmotic stress as a proxy, both cell proliferation and cell expansion are affected resulting in a final leaf size reduction of about 50% (Skirycz et al., 2010; Skirycz et al., 2011b; Claeys et al., 2012).

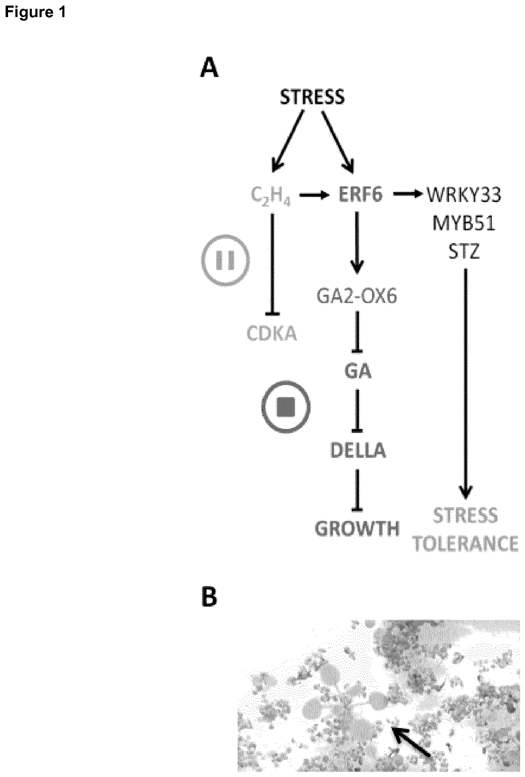

When mild stress occurs during the proliferation phase of early leaf development (leaves <0.1mm² in size), cell cycle progression is affected in a two-step process denominated the "Pause-and-Stop" mechanism, involving crosstalk between the plant hormones ethylene and gibberillic acid (GA)(Figure 1a)( Skirycz et al., 2011b; Claeys et al., 2012). In the first step of mild stress response, the ethylene precursor ACC, which accumulates specifically in growing leaves, inactivates the Cyclin Dependent Kinase A (CDKA), thereby transiently blocking further cell cycle progression ("Pause"). In parallel, the accumulation of ACC induces the expression of the downstream ETHYLENE RESPONSE FACTOR 6 (ERF6). This transcription factor occupies a central role in this pathway as on the one hand it activates the stress tolerance mechanisms, while on the other hand it further converts the paused cell cycle into a definitive cell cycle exit ("Stop") (Dubois et al., 2013). Molecularly, the latter occurs through transcriptional activation of the GA2-OX6 gene, which stimulates breakdown of GAs. Decreased GA levels further result in stabilization of DELLA proteins, which push cells into cell expansion and endoreduplication (Claeys et al., 2012; Achard et al., 2008).

It is however very unlikely that this rather simple linear pathway on its own is the sole regulator of leaf growth under stress. Considering the complexity of regulation of leaf growth under normal conditions (Gonzalez et al., 2012), and the numerous pathways that are responding to changing environments (Skirycz an Inzé, 2010), one can imagine that the integration of environmental signals into leaf growth adaptation is an extremely complex response involving different molecular processes such as signal detection, post-transcriptional regulation, protein complex formation, etc... To identify new genes involved in leaf growth regulation under adverse conditions without restricting our windows towards certain molecular processes or biological functions, we performed a forward genetic screen. As a starting point, we used the easily screenable severe dwarfed phenotype of an inducible ERF6-overexpression line, mutagenized it with EMS, and screened for suppressor mutants. In the present invention we have identified different alleles from EIN. These alleles encode for mutant, functional proteins which when present in a homozygous state in a plant lead to an increased leaf biomass.

### Figures

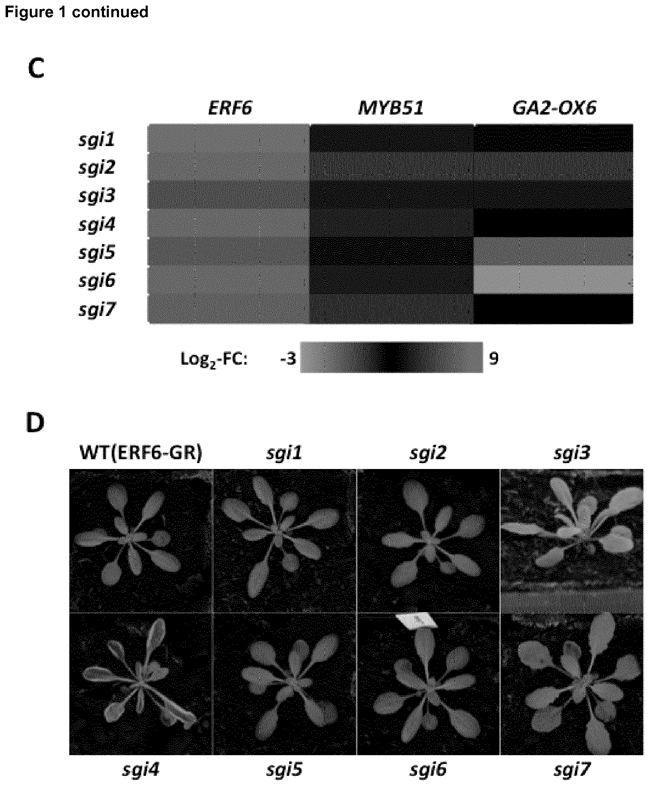

Figure 1: Principle of the EMS-screen and general characteristics of the 7 identified mutants.
   (A) ERF6-centered pathway of growth-inhibition and stress tolerance activation under osmotic stress. Upon activation of ERF6, the GA levels are reduced due to induction of *GA2-OX6*, resulting in a growth inhibition. (B) As a consequence of the pathway in (A), plants overexpressing *ERF6* are extremely dwarfed. Upon EMS-mutagenesis, the indicated mutant suppresses the ERF6-induced dwarfism. The *in vitro* growth medium contains Kanamycin to select for seedlings containing the ERF6-GR construct, and Dexamethasone to induce the overexpression of *ERF6* and the accompanied dwarfism. (C) Expression analysis of the mutants upon transfer to Dexamethasone for the overexpression of *ERF6*, the stress-related target gene *MYB51* and the growth-related target gene *GA2-OX6.* Mutants *sgi1* - *sgi4* and *sgi7* show no longer induction of *GA2-OX6* but still induction of *MYB51*, while *sgi5* and *sgi6* mutants do not induce *MYB51* but have reduced levels of *GA2-OX6.* (D) Rosette phenotypes of the 7 selected mutants, grown in soil in the absence of Dexamethasone. Pictures are taken 22 days after stratification.

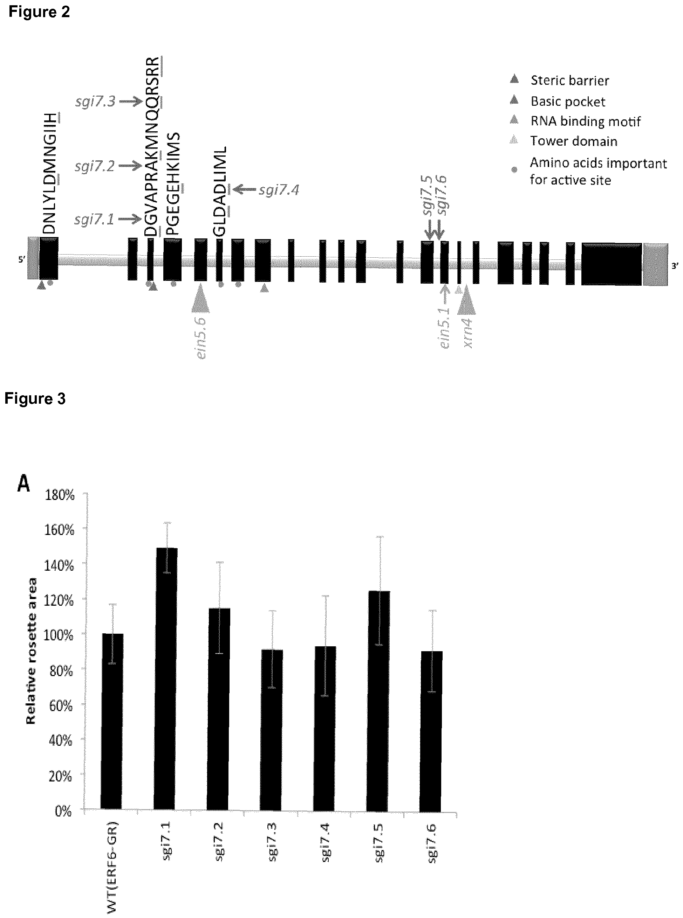

Figure 2: Structure of the XRN4 protein with location of the identified mutations.
   Based on structure predicted by PLAZA (Proost et al., 2009) and on the observations of Nagarajan et al. (2013). Underlined residues indicate key residues for active site function surrounded by conserved stretches of amino acids. Mutants obtained with the EMS-screen are indicated in red, mutants used in previous studies in green (Potuschak et al., 2006). Small arrows indicate point mutants, large triangles indicate T-DNA insertions.

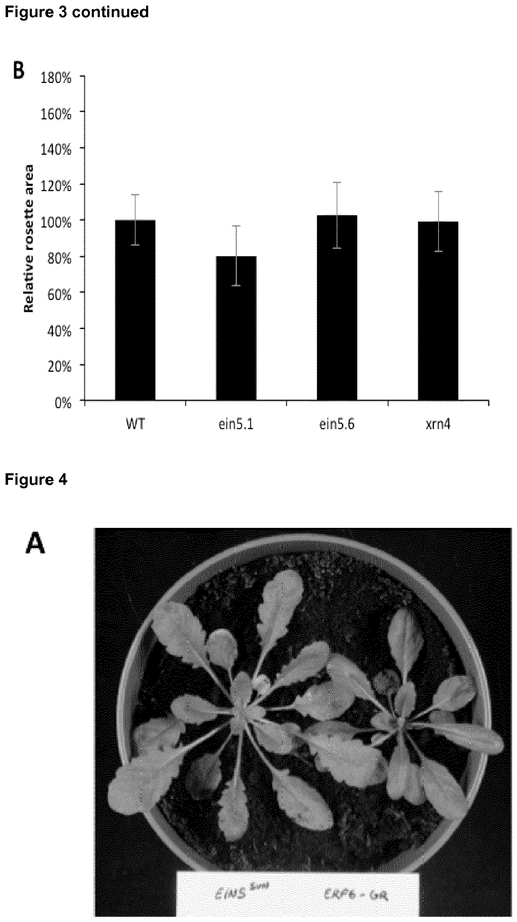

Figure 3: Rosette size of the EMS-mutants and the previously described *ein5* mutants
   (A) Rosette size of the EMS-mutants at 22 days after stratification (DAS) upon growth in soil. Values were normalized to the wild type size. (B) Rosette size of the previously characterized *ein5* mutants at 22 days after stratification (DAS) upon growth in soil. Values were normalized to the wild type size. Error bars represent standard error.

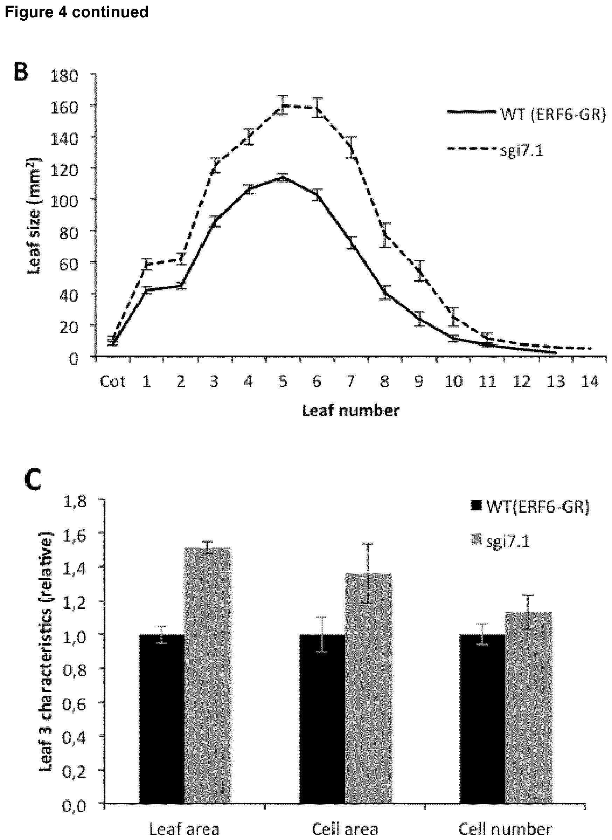

Figure 4: Individual leaf size and cellular measurements of *sgi7.1*
   (A) Picture of a representative *sgi7.1* rosette (left) and appropriate WT (ERF6-GR) (right) after 25 days of growth in soil. (B) Size per leaf of the *sgi7.1* mutant and wild type at 22 days after stratification (DAS) upon growth in soil. Error bars represent standard deviation. Four biological repeats were done. (C) Cellular measurements of the abaxial epidermal cell layer of representative third leaves from the plants in (B). Error bars represent standard error.

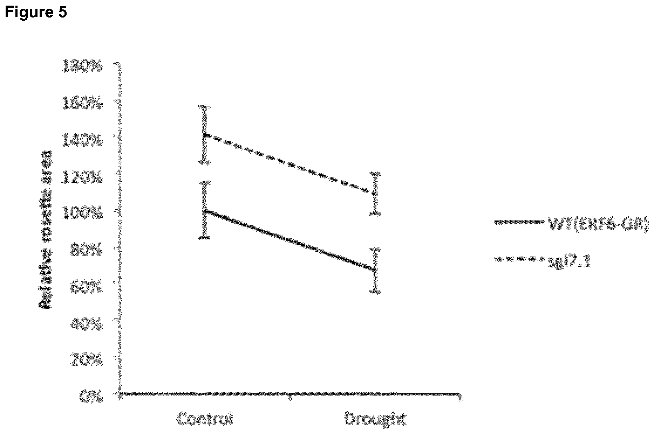

Figure 5 : Rosette size of *sgi7.1* under control and drought stress conditions
   Rosette size of the *sgi7.1* mutant at 22 days after stratification (DAS) when grown on the Weighing Imaging and Watering Machine under either well-watered conditions or mild drought stress. Values were normalized to the wild type size.

### Detailed description of the invention

To facilitate the understanding of this invention a number of terms are defined below. Terms defined herein (unless otherwise specified) have meanings as commonly understood by a person of ordinary skill in the areas relevant to the present invention. As used in this specification and its appended claims, terms such as "a", "an" and "the" are not intended to refer to only a singular entity, but include the general class of which a specific example may be used for illustration, unless the context dictates otherwise. The terminology herein is used to describe specific embodiments of the invention, but their usage does not delimit the invention, except as outlined in the claims. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., current Protocols in Molecular Biology (Supplement 100), John Wiley & Sons, New York (2012), for definitions and terms of the art. The definitions provided herein should not be construed to have a scope less than understood by a person of ordinary skill in the art.

The present invention is derived from the unexpected findings that the homozygous presence of specific EIN5 alleles, encoding mutant, functional EIN5 proteins, in a plant which does not express a wild type EIN5 protein, leads to an increased yield of at least 20% with respect to the wild type plant not comprising the specific EIN5 alleles. Specific EIN5 alleles which are functional and encode for mutant EIN5 proteins are selected from the list consisting of: i) a protein having at least one amino acid change in the sequence DGVAPRAKMNQQRSRR (SEQ ID NO: 15) present in SEQ ID NO: 1 or in a corresponding plant orthologous sequence thereof, ii) a protein having at least one amino sequence change in the sequence GLDADLIML (SEQ ID NO: 16) present in SEQ ID NO: 1 or in a corresponding plant orthologous sequence thereof, iii) a protein having an amino acid change at position 526 or in a corresponding plant orthologous sequence thereof. Yet another specific EIN5 allele which is functional has its splice acceptor site mutated at the nucleotide position 1603 in SEQ ID NO: 2 or in a corresponding plant orthologous sequence thereof.

The term "at least one amino acid change in a sequence" means that at least one of the amino acids present in the sequence can be changed (e.g. by mutation) towards any possible amino acid.

In a specific embodiment the functional mutant EIN5 allele encodes for a functional protein selected from the list consisting of: i) a protein having an amino acid change of G to E in position 105 of SEQ ID NO: 1, or in a corresponding position in a plant orthologous sequence thereof, ii) a protein having an amino acid change of A to V in position 110 of SEQ ID NO: 1, or in a corresponding position in a plant orthologous sequence thereof, iii) a protein having an amino acid change of Q to STOP in position 115 of SEQ ID NO: 1, or in a corresponding position in a plant orthologous sequence thereof, iv) a protein having an amino acid change of D to N in position 236 of SEQ ID NO: 1, or in a corresponding position in a plant orthologous sequence thereof, v) a protein having an amino acid change of Q to STOP in position 526, or in a corresponding position in a plant orthologous sequence thereof or the functional allele of EIN5 has its splice acceptor site mutated at the nucleotide position 1603 in SEQ ID NO: 2 from G to A, or in a corresponding position of a plant orthologous sequence thereof.

The EIN5 gene is sometimes referred to in the art also as the AIN1 or XRN4 gene. Thus a representative of the plant EIN5 gene in *Arabidopsis thaliana* is AT1G54490 (TAIR accession, www.arabidopsis.org), which genome sequence is depicted in SEQ ID NO: 2 and its protein coding sequence is depicted in SEQ ID NO: 1.

The term "plant yield" or "yield" as used herein generally refers to a measurable product from a plant, particularly a crop. Yield and yield increase (in comparison to a wild-type plant) can be measured in a number of ways, and it is understood that a skilled person will be able to apply the correct meaning in view of the particular embodiments, the particular crop concerned and the specific purpose or application concerned. The terms "improved yield" or "increased yield" can be used interchangeable. As used herein, the term "improved yield" or the term "increased yield" means any improvement in the yield of any measured plant product, such as biomass (e.g. leaf biomass), grain, fruit, leaf, root, or fiber. In accordance with the invention, changes in different phenotypic traits may improve yield. For example, and without limitation, parameters such as floral organ development, root initiation, root biomass, seed number, seed weight, harvest index, leaf formation, phototropism, apical dominance, and fruit development, are suitable measurements of improved yield. Increased yield includes higher fruit yields, higher seed yields, higher fresh matter production, higher leaf biomass and/or higher dry matter production. Any increase in yield is an improved yield in accordance with the invention. For example, the improvement in yield can comprise a 0.1%, 0.5%, 1%, 3%, 5%, 10%, 15%, 20%, 30%, 40%, 50%, 60%, 70%, 80%, 90% or greater increase in any measured parameter. For example, an increase in the bu/acre yield of soybeans derived from a soy bean plant which does not express wild type EIN5 protein but comprises and expresses allelic variants of EIN5 in a homozygous state, as specified herein before, as compared with the bu/acre yield from wild type soybeans cultivated under the same conditions, is an improved yield in accordance with the invention. The increased or improved yield can be achieved in the absence or presence of stress conditions. For example, enhanced or increased "yield" refers to one or more yield parameters selected from the group consisting of biomass yield, dry biomass yield, aerial dry biomass yield, underground dry biomass yield (e.g. root biomass), fresh-weight biomass yield, aerial fresh-weight biomass yield (e.g. leaf biomass), underground fresh-weight biomass yield; enhanced yield of harvestable parts, either dry or fresh-weight or both, either aerial or underground or both; enhanced yield of crop fruit, either dry or fresh-weight or both, either aerial or underground or both; and enhanced yield of seeds, either dry or fresh-weight or both, either aerial or underground or both. "Crop yield" is defined herein as the number of bushels of relevant agricultural product (such as grain, forage, or seed) harvested per acre. Crop yield is impacted by abiotic stresses, such as drought, heat, salinity, and cold stress, and by the size (biomass) of the plant. The yield of a plant can depend on the specific plant/crop of interest as well as its intended application (such as food production, feed production, processed food production, biofuel, biogas or alcohol production, or the like) of interest in each particular case. Thus, in one embodiment, yield can be calculated as harvest index (expressed as a ratio of the weight of the respective harvestable parts divided by the total biomass), harvestable parts weight per area (acre, square meter, or the like); and the like. The harvest index is the ratio of yield biomass to the total cumulative biomass at harvest. Harvest index is relatively stable under many environmental conditions, and so a robust correlation between plant size and grain yield is possible. Measurements of plant size in early development, under standardized conditions in a growth chamber or greenhouse, are standard practices to measure potential yield advantages conferred by the presence of the EIN5 alleles of the invention in a homozygous state in a background with no wild type EIN5 expression.

There are two major options to produce a plant expressing the mutant, functional EIN5-alleles of the invention: i) introducing (e.g. by breeding or by genetic transformation) the mutant, functional EIN5-alleles in a plant which does not express wild type alleles of EIN5 and ii) modifying the wild type EIN5-alleles in a plant by introducing targeted mutations in the plant so that the mutant, functional EIN5 alleles are present in a homozygous state. The various steps to generate plants expressing a functional, mutant EIN5-allele of the invention are hereunder discussed.

### Methods for generating a plant with knock-out alleles of EIN5

As used herein a "an EIN5 knock-out allele" is an EIN5 allele which is mutated as compared to the wild type EIN5 allele (i.e. it is a non-functional EIN5 mutant allele) and which encodes a non-functional EIN5 protein or results in a significantly reduced amount of EIN5 protein (by introducing for example a mutation in a regulatory region such as a promoter), or which encodes a protein with significantly reduced activity. Said "EIN5 knock-out allele" may encode no EIN5 protein, or which may encode a non-functional EIN5 protein or an EIN5 protein with significantly reduced function. Such an EIN5 allele may also be referred as an inactivated EIN5 allele. A wild type EIN5 allele is the form of the EIN5 allele as it occurs in nature.

In a specific embodiment, the activity of an EIN5 protein may be reduced or eliminated by disrupting the genes encoding EIN5. The disruption inhibits expression or activity of EIN5 protein compared to a corresponding control plant cell lacking the disruption. A specific embodiment, the disruption step comprises insertion of one or more transposons, where the one or more transposons are inserted into the endogenous EIN5 genes. The disruption can be a homozygous disruption in the EIN5 gene. Alternatively, the disruption is a heterozygous disruption in the EIN5 gene. In certain embodiments, when more than one EIN5 gene is involved, there is more than one disruption, which can include homozygous disruptions, heterozygous disruptions or a combination of homozygous disruptions and heterozygous disruptions. Methods for the transposon tagging of specific genes in plants are well known in the art. See, for example, Meissner, et al (2000) Plant J. 22:265-21. In addition, the TUSC process for selecting Mu insertions in selected genes has been described in US5962764.

In a specific embodiment, a gene encoding for a zinc finger protein that binds to a gene encoding an EIN5 polypeptide is introduced into a plant or plant cell, resulting in a reduced expression of the EIN5 gene. In particular embodiments, the zinc finger protein binds to a regulatory region of an EIN5 gene. In other embodiments, the zinc finger protein binds to a messenger RNA encoding an EIN5 polypeptide and prevents its translation. Methods of selecting sites for targeting by zinc finger proteins have been described, for example, in US6453242, and methods for using zinc finger proteins to inhibit the expression of genes in plants are described, for example, in US2003/0037355.

In another specific embodiment, a gene encoding a TALE protein, that binds to a gene encoding an EIN5 polypeptide, is introduced into a plant or plant cell which results in a reduced expression of the EIN5 gene. In particular embodiments, the TALE protein binds to a regulatory region of an EIN5 gene. In other embodiments, the TALE protein binds to a messenger RNA encoding an EIN5 polypeptide and prevents its translation. Methods of selecting sites for targeting by zinc finger proteins have been described in e.g. Moscou MJ, Bogdanove AJ (2009) (A simple cipher governs DNA recognition by TAL effectors. Science 326:1501) and Morbitzer R, Romer P, Boch J, Lahaye T (2010) (Regulation of selected genome loci using de novo-engineered transcription activator-like effector (TALE)-type transcription factors. Proc Natl Acad Sci USA 107:21617-21622.)

Additional methods for decreasing or eliminating the expression of endogenous genes in plants are also known in the art and can be similarly applied to the instant invention. These methods include other forms of mutagenesis, such as ethyl methanesulfonate-induced mutagenesis, deletion mutagenesis and fast neutron deletion mutagenesis used in a reverse genetics sense (with PCR) to identify plant lines in which the endogenous gene has been deleted. For examples of these methods see, Ohshima, et al, (1998) Virology 243:472-481; Okubara, et al, (1994) Genetics 137:867-874 and Quesada, et al, (2000) Genetics 154:421-436. In addition, a fast and automatable method for screening for chemically induced mutations, TILLING (Targeting Induced Local Lesions in Genomes), using denaturing HPLC or selective endonuclease digestion of selected PCR products is also applicable to the instant invention. See, McCallum, et al, (2000) Nat. Biotechnol 18:455-457. Mutations that impact gene expression or that interfere with the function of the encoded protein are well known in the art. Insertional mutations in gene exons usually result in null-mutants. Mutations in conserved residues are particularly effective in inhibiting the activity of the encoded protein. Conserved residues of plant EIN5 polypeptides suitable for mutagenesis with the goal to eliminate EIN5 activity have been described. Such mutants can be isolated according to well-known procedures, and mutations in different EIN5 loci can be stacked by genetic crossing. See, for example, Gruis, et al (2002) Plant Cell 14:2863-2882.

### Methods for producing a plant having knock-out alleles of EIN5 further comprising specific EIN5 alleles of the invention

In a specific embodiment a plant in which no EIN5 expression occurs (i.e. a plant having knock-out alleles of EIN5) is transformed with a chimeric gene comprising a plant-expressible promoter operably linked to an EIN5 allele of the invention and a suitable plant terminator sequence.

A chimeric gene, as used herein, refers to a gene that is made up of heterologous elements that are operably linked to enable expression of the gene, whereby that combination is not normally found in nature. As such, the term "heterologous" refers to the relationship between two or more nucleic acid or protein sequences that are derived from different sources. For example, a promoter is heterologous with respect to an operably linked nucleic acid sequence, such as a coding sequence, if such a combination is not normally found in nature. In addition, a particular sequence may be "heterologous" with respect to a cell or organism into which it is inserted (i.e. does not naturally occur in that particular cell or organism).

The expression "operably linked" means that said elements of the chimeric gene are linked to one another in such a way that their function is coordinated and allows expression of the coding sequence, i.e. they are functionally linked. By way of example, a promoter is functionally linked to another nucleotide sequence when it is capable of ensuring transcription and ultimately expression of said other nucleotide sequence. Two proteins encoding nucleotide sequences, e.g. a transit peptide encoding nucleic acid sequence and a nucleic acid sequence encoding a protein according to the invention, are functionally or operably linked to each other if they are connected in such a way that a fusion protein of first and second protein or polypeptide can be formed.

A gene, e.g. a chimeric gene comprising a specific EIN5 allele of the invention, is said to be expressed when it leads to the formation of an expression product. An expression product denotes an intermediate or end product arising from the transcription and optionally translation of the nucleic acid, DNA or RNA, coding for such product. During the transcription process, a DNA sequence under control of regulatory regions, particularly the promoter, is transcribed into an RNA molecule.

As the skilled person will be well aware, various promoters may be used to promote the transcription of the nucleic acids encoding the functional EIN5 alleles of the invention. Such promoters include for example constitutive promoters, inducible promoters (e.g. stress-inducible promoters, drought-inducible promoters, hormone-inducible promoters, chemical-inducible promoters, etc.), tissue-specific promoters, developmentally regulated promoters and the like.

The chimeric gene of the invention may also comprise a 3' end region, i.e. a transcription termination or polyadenylation sequence, operable in plant cells. As a transcription termination or polyadenylation sequence, use may be made of any corresponding sequence of bacterial origin, such as for example the nos terminator of Agrobacterium tumefaciens, of viral origin, such as for example the CaMV 35S terminator, or of plant origin, such as for example a histone terminator as described in published Patent Application EP 0 633 317 A1. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

### Methods for producing plants having functional, mutant EIN5 alleles in plants

In specific embodiments the introduction of the functional mutant EIN5 allele of the invention occurs in a plant having wild type EIN5 alleles.

In a specific embodiment the functional mutant EIN5 alleles of the invention can be introduced into said plant e. g. through mutagenesis. "Mutagenesis", as used herein, refers to the process in which plant cells are subjected to a technique which induces mutations in the DNA of the cells, such as contact with a mutagenic agent, such as a chemical substance (such as ethylmethylsulfonate (EMS), ethylnitrosourea (ENU), etc.) or ionizing radiation (neutrons (such as in fast neutron mutagenesis, etc.), alpha rays, gamma rays (such as that supplied by a Cobalt 60 source), X-rays, UV-radiation, etc.), T-DNA insertion mutagenesis (Azpiroz-Leehan et al. (1997) Trends Genet 13:152-156), transposon mutagenesis (McKenzie et al. (2002) Theor Appl Genet 105:23-33), or tissue culture mutagenesis (induction of somaclonal variations), or a combination of two or more of these. Thus, the desired mutagenesis of one or more EIN5 genes or EIN5 alleles may be accomplished by one of the above methods. While mutations created by irradiation are often large deletions or other gross lesions such as translocations or complex rearrangements, mutations created by chemical mutagens are often more discrete lesions such as point mutations. For example, EMS alkylates guanine bases, which results in base mispairing: an alkylated guanine will pair with a thymine base, resulting primarily in G/C to A/T transitions. Following mutagenesis, plants are regenerated from the treated cells using known techniques. For instance, the resulting seeds may be planted in accordance with conventional growing procedures and following pollination seed is formed on the plants. Additional seed that is formed as a result of such pollination in the present or a subsequent generation may be harvested and screened for the presence of mutant functional EIN5 alleles of the invention. Several techniques are known to screen for specific mutant alleles, e.g., DeleteageneTM (Delete-a-gene; Li et al., 2001, Plant J 27: 235-242) uses polymerase chain reaction (PCR) assays to screen for deletion mutants generated by fast neutron mutagenesis, TILLING (targeted induced local lesions in genomes; McCallum et al., 2000, Nat Biotechnol 18:455-457) identifies EMS-induced point mutations, etc.

In yet another embodiment the mutant functional EIN5 alleles can also be introduced via gene targeting techniques. The term "gene targeting" refers herein to directed gene modification that uses mechanisms such as double stranded DNA break repair via non-homologous end-joining, homologous recombination, mismatch repair or site-directed mutagenesis. The method can be used to replace, insert and delete endogenous sequences or sequences previously introduced in plant cells. Methods for gene targeting can be found in, for example, WO 2006/105946 or WO2009/002150. Double stranded DNA breaks can be induced in a targeted manner using custom designed sequence specific nucleases and nuclease systems such as meganucleases/homing endonucleases, zinc finger nucleases, TALENs or CRISPR/CAS (for a review see Gaj et al., 2013, Trends Biotechnol 31: 397-405). In addition to making mutant alleles, such techniques can also be used to delete entire genes encoding proteins having the activity of a protein having any of the above amino acid sequences.

A mutant functional EIN5 allele of the invention can also be introduced through introgression of a generated mutant functional EIN5 allele into said plant.

### Isolation of orthologous plant sequences of the identified EIN5 alleles

Based on the identified functional EIN5 allelic sequences of the invention, the skilled person can isolate homologues sequences or functional variants of the sequences disclosed herein using methods well known in the art, e.g., alignments, either manually or by using computer programs such as BLAST (Altschul et al. (1990), Journal of Molecular Biology, 215, 403-410), which stands for Basic Local Alignment Search Tool or ClustalW (Thompson et al. (1994), Nucleic Acid Res., 22, 4673-4680) or any other suitable program which is suitable to generate sequence alignments. Homologous sequences as described above can comprise orthologous or paralogous sequences. Several different methods are known by those of skill in the art for identifying and defining these functionally homologous sequences. Three general methods for defining orthologous and paralogous genes are described; an ortholog, paralog or homolog may be identified by one or more of the methods described below. Orthologs and paralogs are evolutionarily related genes that have similar sequence and similar functions. Orthologs are structurally related genes in different species that are derived by a speciation event. Paralogs are structurally related genes within a single species that are derived by a duplication event. Within a single plant species, gene duplication may result in two copies of a particular gene, giving rise to two or more genes with similar sequence and often similar function known as paralogs. A paralog is therefore a similar gene formed by duplication within the same species. Paralogs typically cluster together or in the same clade (a group of similar genes) when a gene family phylogeny is analyzed using programs such as CLUSTAL (Thompson, et ah, (1994) Nucleic Acids Res. 22:4673-4680; Higgins, et al, (1996) Methods Enzymol. 266:383-402). Groups of similar genes can also be identified with pairwise BLAST analysis (Feng and Doolittle, (1987) J. Mol. Evol. 25:351-360). Orthologous sequences can also be identified by a reciprocal BLAST strategy. Once an orthologous sequence has been identified, the function of the ortholog can be deduced from the identified function of the reference sequence. Orthologous genes from different organisms have highly conserved functions, and very often essentially identical functions (Lee, et al, (2002) Genome Res. 12:493-502; Remm, et al, (2001) J. Mol. Biol. 314:1041-1052). Paralogous genes, which have diverged through gene duplication, may retain similar functions of the encoded proteins. In such cases, paralogs can be used interchangeably with respect to certain embodiments of the instant invention (for example, transgenic expression of a coding sequence).

Functional variants or homologues of the sequences disclosed herein may also be identified and isolated by hybridization under stringent conditions using as probes identified nucleotide sequences or fragments thereof. For example, homologous sequences from other monocot species than the specific sequences disclosed herein are said to be substantially identical or essentially similar if they can be detected by hybridization under stringent, preferably highly stringent conditions. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridizes to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridizations (Northern blots using a probe of e.g. 100nt) are for example those which include at least one wash in 0.2X SSC at 63°C for 20min, or equivalent conditions.

"High stringency conditions" can be provided, for example, by hybridization at 65°C in an aqueous solution containing 6x SSC (20x SSC contains 3.0 M NaCl, 0.3 M Nacitrate, pH 7.0), 5x Denhardt's (100X Denhardt's contains 2% Ficoll, 2% Polyvinyl pyrollidone, 2% Bovine Serum Albumin), 0.5% sodium dodecyl sulphate (SDS), and 20 µg/ml denaturated carrier DNA (single-stranded fish sperm DNA, with an average length of 120 - 3000 nucleotides) as non-specific competitor. Following hybridization, high stringency washing may be done in several steps, with a final wash (about 30 min) at the hybridization temperature in 0.2-0.1 × SSC, 0.1% SDS. "Moderate stringency conditions" refers to conditions equivalent to hybridization in the above described solution but at about 60-62°C. Moderate stringency washing may be done at the hybridization temperature in 1x SSC, 0.1% SDS. "Low stringency" refers to conditions equivalent to hybridization in the above described solution at about 50-52°C. Low stringency washing may be done at the hybridization temperature in 2x SSC, 0.1% SDS.
Example 8 depicts plant orthologs and EIN5 alleles.

In a specific embodiment the chimeric genes comprising a functional, mutated EIN5-allele of the invention are incorporated in recombinant vectors. Typically such recombinant vectors are plant transformation vectors further comprising a selectable marker or screenable marker gene.
"Selectable or screenable marker", "selectable or screenable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β- galactosidase with its coloured substrates, for example X-Gal), luminescence (such as the luciferin/luciferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Similarly, marker genes can be excised using one or more rarecleaving double strand break inducing enzyme such as meganucleases (naturally occurring or engineered to recognize a specific DNA sequence), zinc finger nucleases, TALE nucleases and the like, if recognition sites for such enzymes are present in the vicinity of the marker gene. Excision can occur via homologous recombination if homology regions flank the marker gene, or via non-homologous end-joining with two recognition sites flanking the marker gene,

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention. The term "nucleic acid molecule" as used interchangeably with the term "polynucleotide" in accordance with the present invention, includes DNA, such as cDNA or genomic DNA, and RNA.

A transgenic plant for the purposes of the invention is thus understood as meaning, as above, that the nucleic acids used in the method of the invention (e.g. the chimeric genes) are not present in, or originating from, the genome of said plant, or are present in the genome of said plant but not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, heterologous expression of the nucleic acids takes place. Preferred transgenic plants are mentioned herein.

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

The term "introduction" or "transformation" as referred to herein encompass the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, mega-gametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363- 373); electroporation of protoplasts (Shillito R.D. et al. (1985) Bio/Technol 3, 1099-1 102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation in planta. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP1198985, Aldemita and Hodges (Planta 199: 612-617, 1996); Chan et al. (Plant Mol Biol 22 (3): 491 -506, 1993), Hiei et al. (Plant J 6 (2): 271 -282, 1994). In the case of corn transformation, the preferred method is as described in either Ishida et al. (Nat. Biotech. 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1 , Engineering and Utilization, eds. S.D. Kung and R. Wu, Academic Press (1993) 128-143 and in Potrykus Annu. Rev. Plant Physiol. Plant Mol. Biol. 42 (1991) 205-225). The nucleic acids or the construct to be expressed is preferably cloned into a vector, which is suitable for transforming *Agrobacterium tumefaciens*, for example pBin19 (Bevan et al (1984) Nucl. Acids Res. 12-8711). Agrobacteria transformed by such a vector can then be used in known manner for the transformation of plants, such as plants used as a model, like Arabidopsis or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Hofgen and Willmitzer in Nucl. Acid Res. (1988) 16, 9877 or is known inter alia from F.F. White, Vectors for Gene Transfer in Higher Plants; in Transgenic Plants, Vol. 1 , Engineering and Utilization, eds. S.D. Kung and R. Wu, Academic Press, 1993, pp. 15-38.
In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic [Feldman, KA and Marks MD (1987). Mol Gen Genet 208:1 -9; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551 -558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension [Bechthold, N (1993). CR Acad Sci Paris Life Sci, 316: 1 194-1 199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Hofgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

In some embodiments, the plant cell according to the invention is non-propagating or cannot be regenerated into a plant.

Plants that are particularly useful in the methods of the invention include in particular monocotyledonous and dicotyledonous plants including fodder or forage legumes, ornamental plants, food crops, trees or shrubs selected from the list comprising Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. [canola, oilseed rape, turnip rape]), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp. (e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium spp., Vicia spp., Vigna spp., Viola odorata, Vitis spp., Zea mays, Zizania palustris, Ziziphus spp., amongst others.

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

The term "expression cassette" refers to any recombinant expression system for the purpose of expressing a nucleic acid sequence of the invention in vitro or in vivo, constitutively or inducibly, in any cell, including, in addition to plant cells, prokaryotic, yeast, fungal, insect or mammalian cells. The term includes linear and circular expression systems. The term includes all vectors. The cassettes can remain episomal or integrate into the host cell genome. The expression cassettes can have the ability to self-replicate or not (i.e., drive only transient expression in a cell). The term includes recombinant expression cassettes that contain only the minimum elements needed for transcription of the recombinant nucleic acid.

The following non-limiting Examples describe methods and means according to the invention. Unless stated otherwise in the Examples, all techniques are carried out according to protocols standard in the art. The following examples are included to illustrate embodiments of the invention. Those of skill in the art should, in light of the present disclosure, appreciate that many changes can be made in the specific embodiments which are disclosed and still obtain a like or similar. More specifically, it will be apparent that certain agents which are both chemically and physiologically related may be substituted for the agents described herein while the same or similar results would be achieved.

### Examples and materials and methods

### 1. Screen for mutants suppressing ERF6-induced dwarfism

It has been shown in the art that leaf growth under stress is regulated by the ERF6-centered pathway in a GA/DELLA-dependent way (Dubois et al., 2013; Claeys et al., 2012)(Figure 1a). Consistently, plants highly overexpressing the transcription factor *ERF6* are extremely dwarfed, and show dark green, down curling leaves. We used this easily detectable phenotype as a starting point for a forward genetics screen and subjected 10000 seeds of an ERF6-GR line (M1) to treatment with the mutagen 1-Methylsulfonyloxyethane (EMS). This commonly used mutagen introduces random and stable point mutations in the genome (Weigel and Glazebrook, 2006). Mutagenized seeds were selfed to obtain M2 mutants homozygous for both the ERF6-GR construct and for the introduced mutations, enabling the identification of recessive mutations. To screen for mutants suppressing the ERF6-induced phenotype 20 000 Arabidopsis (M2) seeds were grown *in vitro* on medium containing dexamethasone (DEX) to induce the ERF6-overexpression, and on Kanamycin (Km). As the ERF6-GR construct contains a Kanamycin resistance marker, this step enabled removal of wild type seeds that possibly contaminated the seed stock. In the presence of the inducing compound (DEX), the non-mutated plants and those that were mutated in a gene unrelated to growth were extremely dwarfed (Figure 1b). In contrast, 12 plants able to repress the ERF6-phenotype because they were mutated in a gene related to ERF6-mediated growth regulation, grew normally. They were selected and selfed to obtain large amounts of non-segregating M3 seeds. The mutants were further crossed with each other to enable identification of allelic groups. In total, the 12 mutants were classified in 7 different allelic groups: 6 groups containing each only 1 mutant, and 1 group containing 6 allelic mutants. For this last group of 6 mutants, a representative mutant was chosen for further analysis. The 7 non-allelic mutants were named *sgi1* - *sgi7* (Suppressor of ERF6-mediated Growth Inhibition).

To validate the mutants, we first checked by PCR and sequencing for the presence of an intact ERF6-GR construct. Two mutants, named *sgi1* and *sgi2* were found to be mutated in the ERF6-domain of the ERF6-GR construct. This provoked missense mutations, respectively A270T and R285Q which are situated in the DNA-binding AP2-domain of the ERF6 protein. However, because upon crossing with a wild type the progeny of both mutants segregated according to the 2^{nd} Mendelian law for 2 independent alleles, we concluded that the mutation in *ERF6* itself is not responsible for the suppression of the ERF6-induced dwarfism.

Secondly, we investigated whether the suppression of the ERF6-induced dwarfism is a specific suppression of the growth-regulatory pathway controlled by ERF6, or a more general abolishment of all functions of ERF6. As we reported previously, ERF6 plays a dual role under stress with, next to the activation of the growth-inhibitory pathway, the induction of several stress tolerance-related transcription factors such as *MYB51*, *STZ* and *WRKY33* (Dubois et al., 2013; Sakamoto et al., 2000, 2004; Gigolashvili et al., 2007; Jiang and Deyholos, 2009; Birkenbihl et al., 2012; Li et al., 2012; Niu et al., 2012; Dubois et al., 2013). We thus performed expression analysis by qRT-PCR to measure the capacity of ERF6 to induce the stress tolerance-related targets, and to confirm the inability to activate the growth-related target gene *GA2-OX6.* To induce the overexpression of ERF6, 15-days old seedlings were exposed to dexamethasone-containing medium and RNA was extracted from the actively expanding third true leaf. We observed that in 5 of the 7 mutants (*sgi1*-*sgi4* and *sgi7*) the induction of *GA2-OX6* by ERF6 does not longer occur, while the stress tolerance genes were still induced (Figure 1c). Amongst these 5 mutants are the 2 mutants *sgi1* and *sgi2* with mutated ERF6, confirming that the functionality of ERF6 was not affected by the *in cis* mutation. The other 2 mutants, *sgi5* and *sgi6* were also affected in the induction of the tolerance-related genes, but showed a remarkable down-regulation of the growth-inhibitory *GA2-OX6* gene and were for that reason kept in the screen (Figure 1c).

Interestingly, when growing the 7 different mutants in soil, under non-DEX conditions, we observed that several mutants show additional phenotypes, on top of the capacity to suppress the ERF6-GR phenotype (Figure 1d). For example, the *sgi3* mutant exhibits a clear shade avoidance phenotype accompanied by early flowering. The *sgi4* mutant has a very striking epinastic phenotype and shows trichomes at both sides of the rosette leaves, while these are generally only observed on the adaxial leaf size. This mutant also shows defective formation of siliques, which often remain very small and contain only few seeds. Importantly, this *sgi7* mutant showed visible increase in rosette size. Larger rosettes were sometimes also observed for *sgi1* and *sgi6*, although this was not reproducible. We thus concluded that 7 non-allelic mutants are able to suppress ERF6-induced dwarfism through different molecular mechanisms and that some of these mutants show interesting rosette phenotypes on top of the capacity to suppress the ERF6-phenotype.

### 2. Identification of the causal genes

To identify the gene mutated in each of the 7 mutants, each mutant (in Col-0 background) was outcrossed with the Arabidopsis Ler-1 accession. About 3000 F2 seeds were screened *in vitro* on growth medium containing both Km and DEX. Upon segregation, 400 mutants showing a normal growth phenotype, and thus containing at least a single copy of the ERF6-GR and being homozygous for the causal mutation were selected and pooled for bulk segregant analysis using Shoremap (Schneeberger et al., 2009). Following genes were found to carry the causal mutation in the respective mutants (see Table 1).

The mutants *sgi1* and *sgi2* were mutated in, respectively, AT1G54820 and AT2G36350. Both genes encode putative kinases without apparent homology, but both have previously been associated with the response in Arabidopsis leaves upon infection by the Gemini virus (Ascencio-Ibanez et al., 2008). Because ERF6 C-terminally possesses a phosphorylation site necessary for proper ERF6 function (Nakano et al., 2006; Meng et al., 2013; Wang et al., 2013), the fact that the 2 mutants mutated in the ERF6-domain of the ERF6-GR both show a causal mutation in a kinase enzyme raises the question whether an impaired post-translational modification of ERF6 by these mutants kinases and/or by the mutant ERF6 could explain the suppression of the ERF6-induced dwarfism. This is rather unlikely as the mutations are not situated in the putative phosphorylation site, but the exact molecular mechanism and putative connection between ERF6 and these 2 kinases certainly deserves further consideration.

The mutation in *sgi3* still remains to be identified. The clear shade avoidance phenotype and the early flowering could suggest a mutation in a gene involved in GA-biosynthesis or signalling, as for example overexpression the *GA20*-*OX1* GA-biosynthetic enzyme shows a comparable phenotype. The same hold for mutant *sgi5*, in which the causal mutation still remains unidentified.

The mutant *sgi4* appeared to be mutated in AT3G05040, a gene encoding the HASTY protein. A G -> A mutation in this gene generates a premature stop codon and probably a truncated protein. The HASTY protein is a nuclear membrane-located protein from the importin/exportin family which imports/exports different substrates such as mRNA or small proteins to or out of the nucleus (Telfer and Poethig, 1998; Bollman et al., 2003). Importantly, the HASTY gene has already been identified in multiple EMS-screens including some based on GR-lines (Allen et al., 2013; REFs). As the functionality of GR lines is relying on proper transport of the constitutively overexpressed fusion protein to the nucleus, it can be speculated that HASTY is involved in this translocation and that truncated HASTY proteins fail in this process. However, it then remains elusive how induction of the stress-related ERF6 target genes still occurs in *sgi4.* We might speculate that few ERF6-GR proteins sporadically reach the nucleus through other transporters, which can be sufficient for the activation of some but not all ERF6 targets. Another hypothesis might involve putative protein-interaction partners of ERF6, which could be necessary to activate a subset of the ERF6-targets (such as the GA2-OX6) and of which the transport to the nucleus is HASTY-dependent. This is however completely speculative. Mutants in the HASTY gene have previously been reported to exhibit the same phenotype as observed for *sgi4*, including the highly pronounced epinasty and the trichomes on the lower side of the leaf, indicating a faster maturation of the shoot (Bollman et al., 2003).

In the mutant *sgi6*, the mutated gene AT3G27670 encodes the transmembrane receptor-like protein *RESURRECTION1 (RST1)*. This gene has previously been reported to negatively affect the cuticular wax biosynthesis, as mutants in *RST1* have increased levels of cuticular wax with an altered fatty acid composition (Chen et al., 2005). As mutants produce 70% of shrunken and unviable seeds (which was also observed for the *sgi6* mutant), the *RST1* gene is also thought to play a role in embryo development (Chen et al., 2005). Finally, the *RST1* protein is a positive regulator of plant defence against biotrophic pathogens, and a negative regulator of defence against necrotrophes (Mang et al., 2009). In *sgi6*, the mutation in the splice acceptor site of the first intron generates a postponed splicing engendering a frameshift and thus likely results in loss-of-function *of RST1.*

Finally, the *sgi7* mutant carried a mutation in the gene encoding EIN5, also named XRN4. EIN5 is a 5' -> 3' exoribonuclease and degrades transcripts carrying preferential EIN5 binding sites (Souret et al., 2004; Rymarquis et al., 2011). The EIN5 enzyme has been involved in the ethylene signalling pathways as reverse genetic mutants of EIN5 are ethylene insensitive, and do not longer show the typical triple response of ethylene-treated seedlings grown in the dark (Van der Straeten et al., 1993; Roman et al., 1995; Olmedo et al., 2006; Potuschak et al., 2006). Consistently with this identification, the *sgi7* mutant did not show triple response upon germination in the dark on medium containing the ethylene precursor 1-aminocyclopropane-1-carboxylic acid (ACC). The *sgi7* mutant looked particularly interesting as it showed a visible increase in leaf size and as it was the representative mutant of the group of 6 allelic mutants, further called *sgi7.1* - *sgi7.6.* We therefore chose to characterize these mutants in more detail.

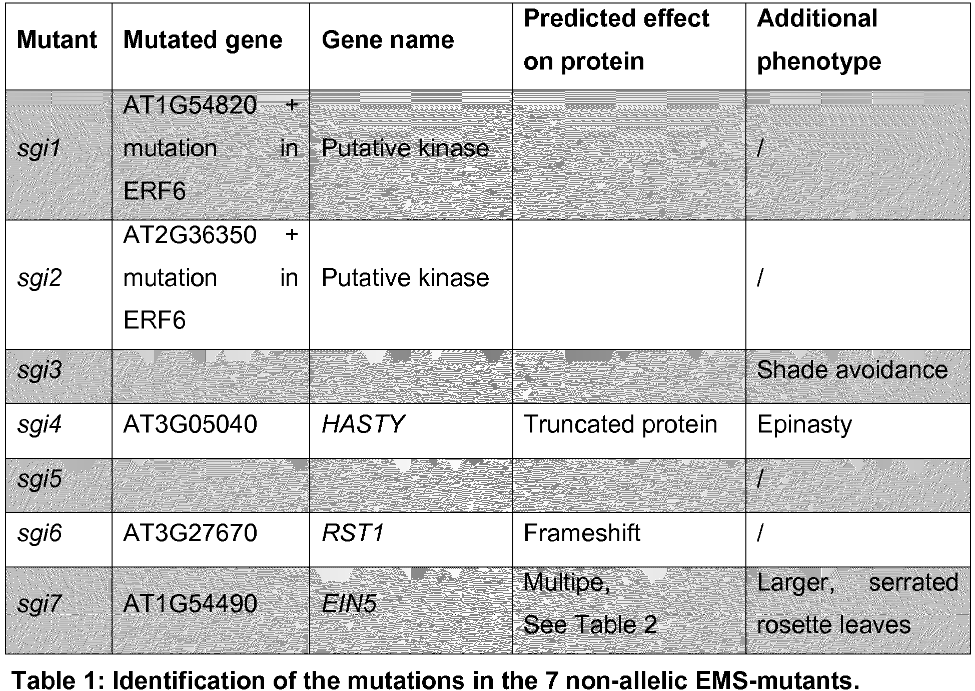

### 3. Multiple mutations identified in conserved domains of EIN5

As the promising *sgi7* mutant (from now on named *sgi7.1*) was the representative of an allelic group of 6 mutants, we also identified the exact mutation in each of the 5 other mutants (see Table 2). The mutations mapped on 6 different sites on the coding sequence of EIN5 and had predicted different effects on the protein sequence: in *sgi7.1*, *sgi7.2* and *sgi7.4*, the point mutation could result in an amino acid substitution, while in *sgi7.3* and *sgi7.5*, the mutation is likely to generate a nonsense mutation. Finally, in *sgi7.6*, the mutation was situated in the splice acceptor site of the 14^{th} intron, which could generate incorrect splicing and finally result in a frameshift.

The EIN5/XRN4 protein is a rather huge protein of 947 amino acids with relatively well-conserved orthologs in Drosophila and yeast (Nagarajan et al., 2013). The protein structure of the yeast XRN1 has recently been described, and the N-terminal half of the protein, containing the most important functional domains, is thought to be conserved between the orthologs. At its N-terminal, the exoribonuclease EIN5 contains several small well-conserved domains, such as a steric barrier to prevent penetrance of dsRNA, a basic pocket for substrate stabilisation and a RNA binding motif (Figure 2). Next to this, the N-terminal side contains several extremely well-conserved amino acids which are thought to be crucial for proper functionality of the active site and which are situated in short stretches of very well-conserved amino acids. Interestingly, several of the identified mutations are localized within these very conserved and thus potentially important motifs (Figure 2).

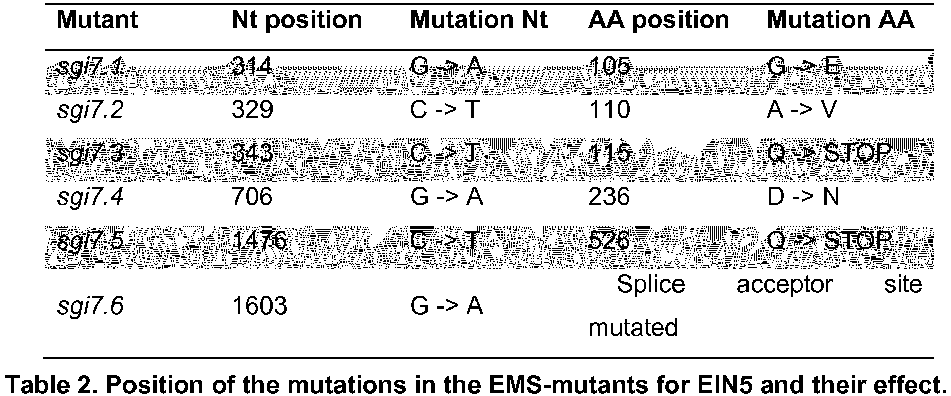

Six mutations were identified on different positions of the EIN5 coding sequence. Based on the validated mutations, the effect on protein level was predicted: three of them generate a missense mutation, two a nonsense mutation and one generates an incorrect splicing variant probably resulting in a frameshift. Nt = nucleotide, AA = amino acid.

### 4. Different alleles with different effects on leaf growth

All identified mutants are able to suppress ERF6-induced dwarfism but interestingly, some of them show additional phenotypes under non-dex conditions (Figure 1d). To investigate whether all identified mutations in *EIN5* had a similar or different effect on Arabidopsis leaf growth, all mutants were simultaneously grown in soil for 22 days and rosette size was measured by making leaf series. Interestingly, the six *sgi7* alleles showed different levels of increase in leaf size (Figure 3a). The most pronounced increase in leaf size was observed for the *sgi7.1* mutant, with an average increase of 49% (p = 2E-9). The mutants *sgi7.5* and *sgi7.2* also showed a significant increase in final rosette area of respectively 26% and 15%. In contrast, the mutants *sgi7.3* and *sgi7.6* were not larger than the WT (ERF6-GR). To further evaluate these growth phenotypes, 3 independent previously used mutants of *EIN5* (*ein5.1*, *ein5.6* and *xrn4*) were phenotyped in a similar way. These three mutants are confirmed loss-of-function mutants as they carry a 1-bp deletion causing a frameshift, a T-DNA insertion in the fifth exon, and a T-DNA insertion in an intron, respectively (Figure 2) (Olmedo et al., 2006). Although these mutants have been extensively studied, their leaf growth has never been characterized in detail. At 22 days after stratification (DAS), the final rosette area was unaltered as compared to wild-type rosette size (Figure 3b). The observation that in these loss-of-function mutants the leaf size is not increased reinforces our hypothesis that the mutations in *sgi7.1*, *sgi7.2* and *sgi7.5* generate EIN5 alleles likely to alter the activity of this protein.

### 5. The EIN5^{G105E} allele of sgi7.1 enhances leaf growth

To unravel the mechanisms behind the stimulation of leaf growth in the *sgi7* mutants, we further focussed on the mutant with the largest rosette area, *sgi7.1.* Detailed analysis showed that this mutant is on average 53% larger resulting from an increased size of all leaves (Figure 4a and 4b). To uncover the cellular mechanism behind it, cellular drawings of the abaxial epidermal layer were made to determine whether increased leaf growth results from more and/or larger cells. We observed a pronounced increase in cell area, with an average increase of 36% (Figure 4c). The increase in cell area was however not sufficient to explain the increased leaf size (51% for the third leaf), but the cell number was only slightly and not significantly increased in all biological repeats. We thus conclude that the leaf size increase in the *sgi7.1* mutant mainly results from an increase in cell area and that the EIN5^{G105E} allele mainly affects the expansion phase of leaf development, although small effects on cell division cannot entirely be excluded.

### 6. The sgi7.1 mutant is more tolerant to mild drought stress

Because the growth-inhibitory pathway on which the forward genetics screen is based is mainly active under adverse environmental conditions reducing rosette growth, we exposed the *sgi7.1* mutant to mild drought stress. Mutant and control were grown on the Weighing Imaging and Watering Machine (WIWAM) under a well-watered regime for 11 days and then exposed to a mild drought stress regime reducing wild-type rosette size by about 30-40% (Skirycz et al., 2011). Rosette size was measured at 22 DAS. In three independent biological repeats, the drought-induced growth inhibition was less pronounced in the *sgi7.1* mutant (-23% in *sgi7.1* compared to -33% in WT) (Figure 5). As this mutant already shows a growth advantage under normal conditions, this resulted in *sgi7.1* mutants being under mild drought equally large as wild type under control conditions. We thus conclude that the EIN5^{G105E} allele not only positively affects growth under control conditions, but also under mild drought stress.

### 7. Introduction of an EIN5 mutant functional allele in Zea mays

The maize homolog of EIN5 is GRMZM2G099630 and the nucleotide sequence is depicted in SEQ ID NO: 14 (see example 8). In order to introduce mutations in the DNA coding for the conserved sequence (GDVAP) (SEQ ID NO: 17) we use the CRISPR technology by stably transforming plants with a construct containing UBI::CAS9 and U6::gRNA, with gRNA containing the sequence targeting the conserved box (gatggtgttgctccaaggg) (SEQ ID NO: 18). The gRNA will guide the CAS9 to the target sequence which will be cleaved. NHEJ will repair the nick while causing insertions, deletions, substitutions. The resulting lines will be genotyped and the lines with no frame shift will be further analyzed and phenotyped.

### 8. Plant ortholoques of EIN5 genes

SEQ ID NO: 1: wild type EIN5 protein sequence of *Arabidopsis thaliana*
   Conserved sequences between different plant orthologs of EIN5 are underlined. The positions of the sgi7.1-sgi7.5 allelic variants in the sequence are marked as [ ].
   The position of the identified functional mutant alleles of EIN5 in A. thaliana is shown in Table 3 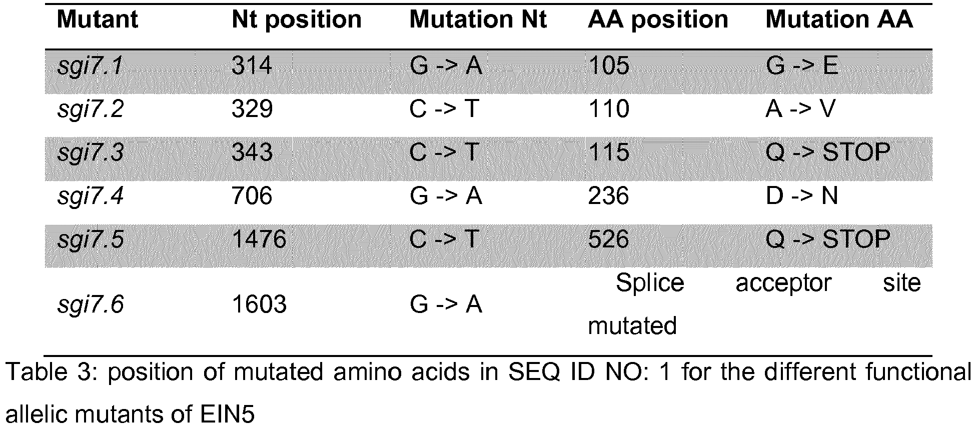
SEQ ID NO: 2 (nucleotide sequence of the *Arabidopsis thaliana* wild type EIN5 gene)
SEQ ID NO: 3 (Glycine max) amino acid sequence of EIN5 protein
SEQ ID NO: 4 (Glycine max) nucleotide sequence of EIN5 gene
SEQ ID NO: 5 Malus domesticus - amino acid sequence of EIN5 protein
SEQ ID NO: 6 Malus domesticus - nucleotide sequence of EIN5 gene
SEQ ID NO: 7 Oryza sativa japonica amino acid sequence of EIN5 protein
SEQ ID NO: 8 Oryza sativa japonica nucleotide sequence of the EIN5 gene
SEQ ID NO: 9 Oryza sativa indica amino acid sequence of EIN5 protein
SEQ ID NO: 10 Oryza sativa indica nucleotide sequence of EIN5 gene
SEQ ID NO: 11 Populus trichocarpa protein sequence of EIN5
SEQ ID NO: 12 Populus trichocarpa nucleotide sequence of EIN5 gene
SEQ ID NO: 13 amino acid sequence of Zea mays EIN5 protein
SEQ ID NO: 14 nucleotide sequence of Zea mays EIN5 gene

### References

Achard, P., F. Gong, S. Cheminant, M. Alioua, P. Hedden and P. Genschik (2008). "The cold-inducible CBF1 factor-dependent signaling pathway modulates the accumulation of the growth-repressing DELLA proteins via its effect on gibberellin metabolism." Plant Cell 20(8): 2117-2129.
Allen, R. S., K. Nakasugi, R. L. Doran, A. A. Millar and P. M. Waterhouse (2013). "Facile mutant identification via a single parental backcross method and application of whole genome sequencing based mapping pipelines." Frontiers in Plant Science 4.
Anastasiou, E. and M. Lenhard (2007). "Growing up to one's standard." Current Opinion in Plant Biology 10(1): 63-69.
Andriankaja, M., S. Dhondt, S. De Bodt, H. Vanhaeren, F. Coppens, L. De Milde, P. Muhlenbock, A. Skirycz, N. Gonzalez, G. T. S. Beemster and D. Inze (2012). "Exit from Proliferation during Leaf Development in Arabidopsis thaliana: A Not-So-Gradual Process." Developmental Cell 22(1): 64-78.
Ascencio-Ibanez, J. T., R. Sozzani, T. J. Lee, T. M. Chu, R. D. Wolfinger, R. Cella and L. Hanley-Bowdoin (2008). "Global analysis of Arabidopsis gene expression uncovers a complex array of changes impacting pathogen response and cell cycle during geminivirus infection." Plant Physiology 148(1): 436-454.
Baerenfaller, K., C. Massonnet, S. Walsh, S. Baginsky, P. Buhlmann, L. Hennig, M. Hirsch-Hoffmann, K. A. Howell, S. Kahlau, A. Radziejwoski, D. Russenberger, D. Rutishauser, I. Small, D. Stekhoven, R. Sulpice, J. Svozil, N. Wuyts, M. Stitt, P. Hilson, C. Granier and W. Gruissem (2012). "Systems-based analysis of Arabidopsis leaf growth reveals adaptation to water deficit." Molecular Systems Biology 8.
Birkenbihl, R. P., C. Diezel and I. E. Somssich (2012). "Arabidopsis WRKY33 Is a Key Transcriptional Regulator of Hormonal and Metabolic Responses toward Botrytis cinerea Infection." Plant Physiology 159(1): 266-285.
Bollman, K. M., M. J. Aukerman, M. Y. Park, C. Hunter, T. Z. Berardini and R. S. Poethig (2003). "HASTY, the Arabidopsis ortholog of exportin 5/MSN5, regulates phase change and morphogenesis." Development 130(8): 1493-1504.
Chen, X. B., S. M. Goodwin, X. L. Liu, X. L. Chen, R. A. Bressan and M. A. Jenks (2005). "Mutation of the RESURRECTION1 locus of Arabidopsis reveals an association of cuticular wax with embryo development." Plant Physiology 139(2): 909-919.
Claeys, H. and D. Inze (2013). "The Agony of Choice: How Plants Balance Growth and Survival under Water-Limiting Conditions." Plant Physiology 162(4): 1768-1779.
Claeys, H., A. Skirycz, K. Maleux and D. Inze (2012). "DELLA Signaling Mediates Stress-Induced Cell Differentiation in Arabidopsis Leaves through Modulation of Anaphase-Promoting Complex/Cyclosome Activity." Plant Physiology 159(2): 739-+.
Dinneny, J. R. (2008). "Cell identity mediates the response of Arabidopsis roots to abiotic stress (vol 320, pg 942, 2008)." Science 322(5898): 44-44.
Donnelly, P. M., D. Bonetta, H. Tsukaya, R. E. Dengler and N. G. Dengler (1999). "Cell cycling and cell enlargement in developing leaves of Arabidopsis." Developmental Biology 215(2): 407-419.
Dubois, M., A. Skirycz, H. Claeys, K. Maleux, S. Dhondt, S. De Bodt, R. Vanden Bossche, L. De Milde, T. Yoshizumi, M. Matsui and D. Inze (2013). "ETHYLENE RESPONSE FACTOR6 Acts as a Central Regulator of Leaf Growth under Water-Limiting Conditions in Arabidopsis." Plant Physiology 162(1): 319-332.
Gigolashvili, T., B. Berger, H. P. Mock, C. Muller, B. Weisshaar and U. I. Fluegge (2007). "The transcription factor HIG1/MYB51 regulates indolic glucosinolate biosynthesis in Arabidopsis thaliana." Plant Journal 50(5): 886-901.
Gonzalez, N., H. Vanhaeren and D. Inze (2012). "Leaf size control: complex coordination of cell division and expansion." Trends in Plant Science 17(6): 332-340.
Harb, A., A. Krishnan, M. M. R. Ambavaram and A. Pereira (2010). "Molecular and Physiological Analysis of Drought Stress in Arabidopsis Reveals Early Responses Leading to Acclimation in Plant Growth." Plant Physiology 154(3): 1254-1271.
Jiang, Y. Q. and M. K. Deyholos (2009). "Functional characterization of Arabidopsis NaCl-inducible WRKY25 and WRKY33 transcription factors in abiotic stresses." Plant Molecular Biology 69(1-2): 91-105.
Li, G. J., X. Z. Meng, R. G. Wang, G. H. Mao, L. Han, Y. D. Liu and S. Q. Zhang (2012). "Dual-Level Regulation of ACC Synthase Activity by MPK3/MPK6 Cascade and Its Downstream WRKY Transcription Factor during Ethylene Induction in Arabidopsis." Plos Genetics 8(6).
Mang, H. G., K. A. Laluk, E. P. Parsons, D. K. Kosma, B. R. Cooper, H. C. Park, S. AbuQamar, C. Boccongelli, S. Miyazaki, F. Consiglio, G. Chilosi, H. J. Bohnert, R. A. Bressan, T. Mengiste and M. A. Jenks (2009). "The Arabidopsis RESURRECTION1 Gene Regulates a Novel Antagonistic Interaction in Plant Defense to Biotrophs and Necrotrophs." Plant Physiology 151(1): 290-305.
Meng, X. Z., J. Xu, Y. X. He, K. Y. Yang, B. Mordorski, Y. D. Liu and S. Q. Zhang (2013). "Phosphorylation of an ERF Transcription Factor by Arabidopsis MPK3/MPK6 Regulates Plant Defense Gene Induction and Fungal Resistance." Plant Cell 25(3): 1126-1142.
Nagarajan, V. K., C. I. Jones, S. F. Newbury and P. J. Green (2013). "XRN 5' -> 3' exoribonucleases: Structure, mechanisms and functions." Biochimica Et Biophysica Acta-Gene Regulatory Mechanisms 1829(6-7): 590-603.
Nakano, T., K. Suzuki, T. Fujimura and H. Shinshi (2006). "Genome-wide analysis of the ERF gene family in Arabidopsis and rice." Plant Physiology 140(2): 411-432.
Niu, C. F., W. Wei, Q. Y. Zhou, A. G. Tian, Y. J. Hao, W. K. Zhang, B. A. Ma, Q. Lin, Z. B. Zhang, J. S. Zhang and S. Y. Chen (2012). "Wheat WRKY genes TaWRKY2 and TaWRKY19 regulate abiotic stress tolerance in transgenic Arabidopsis plants." Plant Cell and Environment 35(6): 1156-1170.
Olmedo, G., H. W. Guo, B. D. Gregory, S. D. Nourizadeh, L. Aguilar-Henonin, H. J. Li, F. Y. An, P. Guzman and J. R. Ecker (2006). "ETHYLENE-INSENSITIVE5 encodes a 5 '-> 3 ' exoribonuclease required for regulation of the EIN3-targeting F-box proteins EBF1/2." Proceedings of the National Academy of Sciences of the United States of America 103(36): 13286-13293.
Potuschak, T., A. Vansiri, B. M. Binder, E. Lechner, R. D. Vierstra and P. Genschik (2006). "The exoribonuclease XRN4 is a component of the ethylene response pathway in Arabidopsis." Plant Cell 18(11): 3047-3057.
Roman, G., B. Lubarsky, J. J. Kieber, M. Rothenberg and J. R. Ecker (1995). "Genetic-Analysis of Ethylene Signal-Transduction in Arabidopsis-Thaliana - 5 Novel Mutant Loci Integrated into a Stress-Response Pathway." Genetics 139(3): 1393-1409.
Rymarquis, L. A., F. F. Souret and P. J. Green (2011). "Evidence that XRN4, an Arabidopsis homolog of exoribonuclease XRN1, preferentially impacts transcripts with certain sequences or in particular functional categories." Rna-a Publication of the Rna Society 17(3): 501-511.
Sakamoto, H., T. Araki, T. Meshi and M. Iwabuchi (2000). "Expression of a subset of the Arabidopsis Cys(2)/His(2)-type zinc-finger protein gene family under water stress." Gene 248(1-2): 23-32.
Sakamoto, H., K. Maruyama, Y. Sakuma, T. Meshi, M. Iwabuchi, K. Shinozaki and K. Yamaguchi-Shinozaki (2004). "Arabidopsis Cys2/His2-type zinc-finger proteins function as transcription repressors under drought, cold, and high-salinity stress conditions." Plant Physiology 136(1): 2734-2746.
Schachtman, D. P. and J. Q. D. Goodger (2008). "Chemical root to shoot signaling under drought." Trends in Plant Science 13(6): 281-287.
Schneeberger, K., S. Ossowski, C. Lanz, T. Juul, A. H. Petersen, K. L. Nielsen, J. E. Jorgensen, D. Weigel and S. U. Andersen (2009). "SHOREmap: simultaneous mapping and mutation identification by deep sequencing." Nature Methods 6(8): 550-551.
Seki, M., A. Matsui, J. M. Kim, J. Ishida, M. Nakajima, T. Morosawa, M. Kawashima, M. Satou, T. K. To, Y. Kurihara, E. Kaminuma, T. Endo, Y. Mochizuki, N. Kobayashi, T. Toyoda and K. Shinozaki (2007). "Arabidopsis whole-genome transcriptome analysis under drought, cold, high-salinity, and ABA treatment conditions using tiling array and 454 sequencing technology." Plant and Cell Physiology 48: S8-S8.
Skirycz, A., H. Claeys, S. De Bodt, A. Oikawa, S. Shinoda, M. Andriankaja, K. Maleux, N. B. Eloy, F. Coppens, S. D. Yoo, K. Saito and D. Inze (2011). "Pause-and-Stop: The Effects of Osmotic Stress on Cell Proliferation during Early Leaf Development in Arabidopsis and a Role for Ethylene Signaling in Cell Cycle Arrest." Plant Cell 23(5): 1876-1888.
Skirycz, A., S. De Bodt, T. Obata, I. De Clercq, H. Claeys, R. De Rycke, M. Andriankaja, O. Van Aken, F. Van Breusegem, A. R. Fernie and D. Inze (2010). "Developmental Stage Specificity and the Role of Mitochondrial Metabolism in the Response of Arabidopsis Leaves to Prolonged Mild Osmotic Stress." Plant Physiology 152(1): 226-244.
Skirycz, A. and D. Inze (2010). "More from less: plant growth under limited water." Current Opinion in Biotechnology 21(2): 197-203.
Skirycz, A., K. Vandenbroucke, P. Clauw, K. Maleux, B. De Meyer, S. Dhondt, A. Pucci, N. Gonzalez, F. Hoeberichts, V. B. Tognetti, M. Galbiati, C. Tonelli, F. Van Breusegem, M. Vuylsteke and D. Inze (2011). "Survival and growth of Arabidopsis plants given limited water are not equal." Nature Biotechnology 29(3): 212-214.
Souret, F. F., J. P. Kastenmayer and P. J. Green (2004). "AtXRN4 degrades mRNA in Arabidopsis and its substrates include selected miRNA targets." Molecular Cell 15(2): 173-183.
Telfer, A. and R. S. Poethig (1998). "HASTY: a gene that regulates the timing of shoot maturation in Arabidopsis thaliana." Development 125(10): 1889-1898.
Umezawa, T., M. Fujita, Y. Fujita, K. Yamaguchi-Shinozaki and K. Shinozaki (2006). "Engineering drought tolerance in plants: discovering and tailoring genes to unlock the future." Current Opinion in Biotechnology 17(2): 113-122.
Vanderstraeten, D., A. Djudzman, W. Vancaeneghem, J. Smalle and M. Vanmontagu (1993). "Genetic and Physiological Analysis of a New Locus in Arabidopsis That Confers Resistance to 1-Aminocyclopropane-1-Carboxylic Acid and Ethylene and Specifically Affects the Ethylene Signal-Transduction Pathway." Plant Physiology 102(2): 401-408.
Verslues, P. E., M. Agarwal, S. Katiyar-Agarwal, J. Zhu and J. K. Zhu (2006). "Methods and concepts in quantifying resistance to drought, salt and freezing, abiotic stresses that affect plant water status. (vol 45, pg 523, 2006)." Plant Journal 46(6): 1092-1092.
Vlieghe, K., V. Boudolf, G. T. S. Beemster, S. Maes, Z. Magyar, A. Atanassova, J. D. Engler, R. De Groodt, D. Inze and L. De Veylder (2005). "The DP-E2F-like gene DEL1 controls the endocycle in Arabidopsis thaliana." Current Biology 15(1): 59-63.
Wang, P. C., Y. Y. Du, X. L. Zhao, Y. C. Miao and C. P. Song (2013). "The MPK6-ERF6-ROS-Responsive cis-Acting Element7/GCC Box Complex Modulates Oxidative Gene Transcription and the Oxidative Response in Arabidopsis." Plant Physiology 161(3): 1392-1408.
Weigel, D., J., Glazebrook (2006). Adapted from "Obtaining Mutants," Chapter 2 in Arabidopsis by Detlef Weigel and Jane Glazebrook. Cold Spring Harbor Laboratory Press, Cold Spring Harbor, NY, USA, 2002.
Xiong, L. M., K. S. Schumaker and J. K. Zhu (2002). "Cell signaling during cold, drought, and salt stress." Plant Cell 14: S165-S183.

## Claims

1. A functional allele of EIN5 encoding for a mutant protein selected from the list consisting of:
i. a protein having at least one amino acid change in the sequence DGVAPRAKMNQQRSRR present in SEQ ID NO: 1 or in a corresponding plant orthologous sequence thereof,
ii. a protein having at least one amino sequence change in the sequence GLDADLIML present in SEQ ID NO: 1 or in a corresponding plant orthologous sequence thereof,
iii. a protein having an amino acid change at position 526 in SEQ ID NO: 1 or in a corresponding plant orthologous sequence thereof,
or a functional allele of EIN5 having its splice acceptor site mutated at the nucleotide position 1603 in SEQ ID NO: 2 or in a corresponding plant orthologous sequence thereof.

2. The functional allele according claim 1 encoding for a functional protein selected from the list consisting of:
i. a protein having an amino acid change of G to E in position 105 of SEQ ID NO: 1, or in a corresponding position in a plant orthologous sequence thereof,
ii. a protein having an amino acid change of A to V in position 110 of SEQ ID NO: 1, or in a corresponding position in a plant orthologous sequence thereof,
iii. a protein having an amino acid change of Q to STOP in position 115 of SEQ ID NO: 1, or in a corresponding position in a plant orthologous sequence thereof,
iv. a protein having an amino acid change of D to N in position 236 of SEQ ID NO: 1, or in a corresponding position in a plant orthologous sequence thereof,
v. a protein having an amino acid change of Q to a STOP-codon in position 526 of SEQ ID NO: 1, or in a corresponding position in a plant orthologous sequence thereof,
or a functional allele of EIN5 having its splice acceptor site mutated at the nucleotide position 1603 in SEQ ID NO: 2 from G to A, or in a corresponding position of a plant orthologous sequence thereof.

3. A chimeric gene comprising a plant expressible promoter, a mutant, functional EIN5 allele according to claims 1 or 2 and a functional terminator sequence.

4. A mutant plant, or a cell, part, seed or progeny thereof, having knock-out EIN5 genes, comprising a chimeric gene according to claim 3.

5. A mutant plant, or a cell, part, seed or progeny thereof, having its wild type EIN5 genes replaced by mutant, functional EIN5 alleles in a homozygous state wherein said alleles are defined according to claims 1 or 2, wherein the plant, part, seed or progeny are not exclusively obtained by means of an essentially biological process.

6. The plant according to claims 4 or 5 wherein the yield is increased by at least 20% compared to the plant having wild type EIN5 alleles.

7. The plant according to claims 4 or 5 wherein the drought tolerance is increased compared to the plant having wild type EIN5 alleles.

## Patentansprüche

1. Funktionelles Allel von EIN5, das für ein mutiertes Protein kodiert, das aus der Liste bestehend aus folgenden ausgewählt ist:
i. einem Protein mit mindestens einer Aminosäurenveränderung in der Sequenz DGVAPRAKMNQQRSRR, die in SEQ ID Nr. 1 oder in einer entsprechenden pflanzlichen orthologen Sequenz davon vorliegt,
ii. einem Protein mit mindestens einer Aminosäurenveränderung in der Sequenz GLDADLIML, die in SEQ ID Nr. 1 oder in einer entsprechenden pflanzlichen orthologen Sequenz davon vorliegt,
iii. einem Protein mit mindestens einer Aminosäurenveränderung an Position 526 in SEQ ID Nr. 1 oder in einer entsprechenden pflanzlichen orthologen Sequenz davon vorliegt,
oder funktionelles Allel von EIN5, das seine Spleißakzeptorstelle an der Nukleotidposition 1603 in SEQ ID Nr. 2 oder in einer entsprechenden pflanzlichen orthologen Sequenz davon mutiert aufweist.

2. Funktionelles Allel nach Anspruch 1, das für ein funktionelles Protein kodiert, das aus der Liste bestehend aus folgenden ausgewählt ist:
i. einem Protein mit mindestens einer Aminosäurenveränderung von G zu E in Position 105 von SEQ ID Nr. 1 oder in einer entsprechenden Position in einer pflanzlichen orthologen Sequenz davon,
ii. einem Protein mit mindestens einer Aminosäurenveränderung von A zu V in Position 110 von SEQ ID Nr. 1 oder in einer entsprechenden Position in einer pflanzlichen orthologen Sequenz davon,
iii. einem Protein mit mindestens einer Aminosäurenveränderung von Q zu STOP in Position 115 von SEQ ID Nr. 1 oder in einer entsprechenden Position in einer pflanzlichen orthologen Sequenz davon,
iv. einem Protein mit mindestens einer Aminosäurenveränderung von D zu N in Position 236 von SEQ ID Nr. 1 oder in einer entsprechenden Position in einer pflanzlichen orthologen Sequenz davon,
v. einem Protein mit mindestens einer Aminosäurenveränderung von Q zu einem STOP-Codon in Position 526 von SEQ ID Nr. 1 oder in einer entsprechenden Position in einer pflanzlichen orthologen Sequenz davon,
oder funktionelles Allel von EIN5, das seine Spleißakzeptorstelle an der Nukleotidposition 1603 in SEQ ID Nr. 2 von G zu A der in einer entsprechenden Position in einer pflanzlichen orthologen Sequenz davon mutiert aufweist.

3. Chimäres Gen, das einen pflanzlichen exprimierbaren Promotor, ein mutiertes, funktionelles EIN5-Allel nach den Ansprüchen 1 oder 2 und eine funktionelle Terminatorsequenz umfasst.

4. Mutierte Pflanze oder Zelle, Teil, Saat oder Abkömmling davon mit Knock-out-EIN5-Genen, die bzw. der ein chimäres Gen nach Anspruch 3 umfasst.

5. Mutierte Pflanze oder Zelle, Teil, Saat oder Abkömmling, die bzw. der ihre bzw. seine Wildtyp-EIN5-Gene durch mutierte, funktionelle EIN5-Allele in einem homozygoten Zustand ersetzt aufweist, wobei die Allele nach den Ansprüchen 1 oder 2 definiert sind, wobei die Pflanze, der Teil, die Saat oder der Abkömmling nicht ausschließlich mittels eines im Wesentlichen biologischen Vorgangs erhalten wird.

6. Pflanze nach den Ansprüchen 4 oder 5, wobei die Ausbeute im Vergleich zu der Pflanze mit Wildtyp-EIN5-Allelen um mindestens 20 % erhöht ist.

7. Pflanze nach den Ansprüchen 4 oder 5, wobei die Trockenheitstoleranz im Vergleich zu der Pflanze mit Wildtyp-EIN5-Allelen erhöht ist.

## Revendications

1. Allèle fonctionnel de EIN5 codant pour une protéine mutante sélectionnée parmi la liste constituée de :
i. une protéine ayant au moins un changement d'acide aminé dans la séquence DGVAPRAKMNQQRSRR présente dans SEQ ID N° : 1 ou dans une séquence orthologue végétale correspondante de celle-ci,
ii. une protéine ayant au moins un changement de séquence d'acides aminés dans la séquence GLDADLIML présente dans SEQ ID N° : 1 ou dans une séquence orthologue végétale correspondante de celle-ci,
iii. une protéine ayant un changement d'acide aminé à la position 526 dans SEQ ID N° : 1 ou dans une séquence orthologue végétale correspondante de celle-ci,
ou allèle fonctionnel de EIN5 ayant son site accepteur d'épissage muté à la position nucléotidique 1603 dans SEQ ID N° : 2 ou dans une séquence orthologue végétale correspondante de celle-ci.

2. Allèle fonctionnel selon la revendication 1 codant pour une protéine fonctionnelle sélectionnée parmi la liste constituée de :
i. une protéine ayant un changement d'acide aminé de G à E à la position 105 de SEQ ID N° : 1, ou à une position correspondante dans une séquence orthologue végétale de celle-ci,
ii. une protéine ayant un changement d'acide aminé de A à V à la position 110 de SEQ ID N° : 1, ou à une position correspondante dans une séquence orthologue végétale de celle-ci,
iii. une protéine ayant un changement d'acide aminé de Q à STOP à la position 115 de SEQ ID N° : 1, ou à une position correspondante dans une séquence orthologue végétale de celle-ci,
iv. une protéine ayant un changement d'acide aminé de D à N à la position 236 de SEQ ID N° : 1, ou à une position correspondante dans une séquence orthologue végétale de celle-ci,
v. une protéine ayant un changement d'acide aminé de Q à un codon STOP à la position 526 de SEQ ID N° : 1, ou à une position correspondante dans une séquence orthologue végétale de celle-ci,
ou allèle fonctionnel de EIN5 ayant son site accepteur d'épissage muté à la position nucléotidique 1603 dans SEQ ID N° : 2 de G à A, ou à une position correspondante dans une séquence orthologue végétale de celle-ci.

3. Gène chimère comprenant un promoteur exprimable végétal, un allèle de EIN5 fonctionnel mutant selon la revendication 1 ou 2 et une séquence terminatrice fonctionnelle.

4. Plante mutante, ou cellule, partie, graine ou descendance de celle-ci, ayant des gènes EIN5 knock-out, comprenant un gène chimère selon la revendication 3.

5. Plante mutante, ou cellule, partie, graine ou descendance de celle-ci, ayant ses gènes EIN5 de type sauvage remplacés par des allèles de EIN5 fonctionnels mutants dans un état homozygote, dans laquelle lesdits allèles sont définis selon la revendication 1 ou 2, dans laquelle la plante, partie, graine ou descendance n'est pas exclusivement obtenue au moyen d'un processus essentiellement biologique.

6. Plante selon la revendication 4 ou 5, dans laquelle le rendement est accru d'au moins 20 % par rapport à la plante ayant des allèles de EIN5 de type sauvage.

7. Plante selon la revendication 4 ou 5, dans laquelle la tolérance à la sécheresse est accrue par rapport à la plante ayant des allèles de EIN5 de type sauvage.


### SEQUENCE LISTING

<110> VIB VZW UNIVERSITEIT GENT
<120> Mutant alleles for yield increase
<130> DI/EIN5/502
<150> EP 14187614.4
   <151> 2014-10-03
<160> 18
<170> PatentIn version 3.5
<210> 1
   <211> 947
   <212> PRT
   <213> Arabidopsis thaliana
<400> 1
<210> 2
   <211> 6726
   <212> DNA
   <213> Arabidopsis thaliana
<400> 2
   ctaacaactt tggtgacagc taaagaaaaa ctaagttaga agaatattta gataaccttc      60
   aagctcgaga ccactcggat ctctctctaa tcgccgatcg gaatccgcca tgggagtacc     120
   ggcgttctac agatggctcg ctgaccgata cccgaagtca attagtgacg tcgttgaaga     180
   agaacctacc gatggaggtc gtggtgacct tattcctgtt gatatcacca gacctaaccc     240
   taacggcttc gaatttgata atttgtatct cgatatgaat ggtatcattc atccttgttt     300
   ccatcctgaa ggaaaggtat gttgtagctc agaaatgttt ggttagggtt tgagttgatt     360
   tgttgtatga gaaatgaaat caatgagttt agtagtttgg tggtggagaa gattattgtt     420
   gtctgtattc gtagtttagt acagttatga gagaaggaga agtatactag ttgttgcttt     480
   tcctcgattt tgcttcctgc acgtcaatgg aagcgatggc caacttggtg atactagacg     540
   tagggtatct aacttttctg aagtgtatga ttagagtttg agttgattgt attaggcagt     600
   aacaagtaga catggatcgt atgagaaatg aaatcactaa gtttggtatt ttggtgagaa     660
   gatgattttt ttttggtgtt cataatttag ctcttttatt ggggaaggga ggagtgtact     720
   agttgttgct ttccgactat ttttgcttac cgaaagtcaa tagaagcaat ggccattttt     780
   gtgatactag attttgggat ctaaagttca gttcagaaag tgatttagat atattatttg     840
   ggaaattgga tgcttatggc tgtgtgtatt ttgttcatgt atcacttatg cgagtctaaa     900
   gtcagttttt ttataaaaat agttgactag aaaattaaaa gacactgatg ttattagttt     960
   ttcattatag ttgaggagat attatcgaag ggagtgagtt tgcatagcaa tacgtgttat    1020
   ggtgatactt gatttccatt aaagatttta ctagctaatc gtactttgat tacaatgcag    1080
   cctgcacctg ccacgtacga tgatgtattt aaatcaatgt ttgagtacat tgaccatctt    1140
   tttactttag tgcgtccgag gaagattctt tatttggcta taggtgatct tgtgtttcct    1200
   ttttatcatc tgtctgtttt gctctgtgag atgtggttga ttaaatatac tgttgcagat    1260
   ggagttgcgc cgagagcaaa gatgaatcag cagcgttctc gacgtttcag agctgcaaag    1320
   gacgcagcag aggctgtatg ttctctttgt gtttctattt ttacatttac tcttggcttt    1380
   acatgggtca ctgttgctta tactatttct ggtttaatag gaagctgagg aagagaggtt 1440
   gagaaaagat tttgagatgg agggccaaat cttgtccgct aaagagaaag ctgaaacctg 1500
   tgattcaaat gtcattactc ctgggacccc ttttatggct atactgtcag tcgctcttca 1560
   gtattacatc caatctaggt taaaccataa ccctggctgg agatatgtta aggtgtgata 1620
   ctgtttcatc taacttagca gctatttcca aactgtacaa gacttttcgc attcttgcag 1680
   tttctctttt ttttttggtg ttgtcccttt acttttggtt ttaaaggtaa tttgtctccc 1740
   taagaagtcg tggattatta tttgaaggta attctttctg attcaaatgt tccgggagaa 1800
   ggagaacaca agataatgtc atacattcgt ctccaacgta atcttcctgg ttttgatcca 1860
   aatacacggc attgtctata tggtctggtt agtgtcaacc cataatgtac ttgttttatt 1920
   ttcttaaata aggtgttggt gcttcgtcca aaaatttgca cattacattt gccaactgta 1980
   tattattatg caggatgcag atttgataat gctctcctta gctacacatg aagttcactt 2040
   ttccatctta agagaggtag ctgatttcca cccctacgct ctatattttc tttgatattc 2100
   agttgatcaa atgatgggtc atctttttga ttccaggtta tcacttaccc tgggcagcaa 2160
   gagaaatgct ttgtgtgtgg acaaactggc cactttgctt cagattgccc tggaaaatca 2220
   ggtagcaata atgcagcagc tgatattcca atccacaaga aaaaatatca ggtttgtagt 2280
   tttccttgtt atctccaaga gatcttgctg cagtgaacaa cagaaaatta ttttatttta 2340
   cttttggagc tttctcttat ttgtctgatg taattttttt tttgaaaata gttcctgaac 2400
   atttgggtgt tgcgagaata tctgcaatat gaattggcca ttccagatcc tccttttatg 2460
   atcaactttg agagaataat agacgacttt gtattccttt gcttttttgt tggtaatgac 2520
   ttcctacctc atatgcctac actggaaatt cgagaggtat gtacatggta tagcttttta 2580
   ttccttaaag gattggctgt tcgtaaagtg atggttgtgt agattagaaa atatagtaat 2640
   agccttgtgg aagataaata tcctgtttca tgctctacaa taccactgag gcactgaaaa 2700
   attagtctgt ttctttaaac caagtttctt tgttacttca gggggctata aacttgctga 2760
   tgcatgttta cagaaaggag tttacagcta tgggaggcta tctaacagat tctggtgagg 2820
   taagttattg tttctttttt taatcttctc ccacatttca ttctcgcttg acattgctca 2880
   ataccctatt ttgtatgagc tgtagaaatg gtcagttatc ttgtggaaac tattttctcg 2940
   cttctattta gtctggctat atgattacta atgaaaatcg gaactgcatt atatctgtgg 3000
   gcttcatttc ttttaatcgt taacccattg acaacttctt tttttctcca ttgataggtt 3060
   ttactagata gggtagagca tttcattcaa gctgtcgctg taaatgaaga taaaatattt 3120
   cagaaaagaa cgcggataaa acaggtaatc ttaaccttta ccctattttc ctacacaacc 3180
   ctttggaggc tttaggaact gtgctcacac ccttatgagg ctaatggtgt gttgatgttg 3240
   gtatcagtca atggataaca atgaagaaat gaagcaaaga tctagaaggg acccatctga 3300
   ggtaccacct gaaccgatag acgataaggt aaaatagttt actgcaaaga actcttatat 3360
   gtgcctgtga gctggagaga ttcttatata tttattgaaa aggaaattag tgtaggagga 3420
   acgactaaaa ctgctctttc attttgggta gattaaatta ggagaaccag gctacaagga 3480
   gaggtattat gctgagaaat ttagtacgac caatccagag gaaactgagc aaatcaaaca 3540
   agacatggtg agttttacgt caattaacgc tctctgtcac tcttctgaat ctctaatgat 3600
   ttgttataat aagtctaaag ttggaaaaag gtatcttttg gcgatgtgct actttgatta 3660
   tgtgtgcatt tagacttttt aacatcctat taactccctc atgttgaaaa tttctgccca 3720
   cggaatttag ctcttaatat tttcacagtt cttttttttt cttttatgga ggcttatagc 3780
   gcgatatgaa ctacttcatt gaagtgactc tcaaaacagt ttggaattgg atgaacaata 3840
   actagttatc tattctgatc attcattacc tattttcgtt gtaggtactg aaatatgttg 3900
   aaggtttgtg ctgggtttgc cggtactact accaaggtgt atgctcttgg caatggtaag 3960
   tctgacttta caaaaactta atgtcatgtt attcccatga gagccatgca ttctaacaaa 4020
   aacttggtct cttctcggga aagaccttga tgttgatgtc acttactgag aaatactaag 4080
   ctgcaaattt taaatgtaat tttgtgtttt cttacacttt cttctaattg tttcaggttt 4140
   tacccatacc attatgctcc atttgcttca gatcttaaga atctacctga tttagaaatc 4200
   acatttttta ttggagagcc ctttaaacct tttgaccagt taatgggaac cctgccggct 4260
   gcaaggttct tccctttttt tggaacctta attattaata ttgtgggcct ctatggtgat 4320
   gtaaattgtg tttcttggtt gttgcagctc aaatgcgctg cctggagaat accggaagtt 4380
   gatgactgat ccctcatccc cgatacttaa attttaccct gctggtaatt gttgatactg 4440
   aatttgtatt gttgttttcc tatatattca tgctggatag ttgacactca taagctatca 4500
   ttctatttat agattttgag cttgacatga atggaaagcg cttcgcctgg caggttcatg 4560
   gttgtttgta ttctttggat atagtattga tgttatcatg ctgagactgt gtattttgtt 4620
   cttcagctgt tgatgttgtg tgttttgcgc ttgttgttat ctagggtatt gcaaaacttc 4680
   ctttcattga ggagaaatta ttgttagctg ctacaaggaa gcttgaagaa actctaacgg 4740
   tttgaactga accttgactc gaaatgcata atgtttgatc aaaactttat tctctccaaa 4800
   ggaaactata attctttctg aaagatgatt cttggacctg caggatggtt tatttgctag 4860
   ttataaagta gttttatagc ttagattgct ctagccatca gttttcatgg cagtttatga 4920
   ttaactacaa tatggatcca tctttcaggt ggaagaacaa caacgcaaca gtgtgatgct 4980
   tgatttgtta tacgtacacc ccgctcatcc actgggccag cggattctgc agtactatca 5040
   tttctaccag cacatgccac cacatgaatg tcttccatgg atgattgacc cgaattctag 5100
   gtttgttttt attggctaaa ttgataacta acaagctgtg tgtactctgg atatcgagct 5160
   tgttcttaat catcaattcg tttgtgtttg ttgcagccaa ggaatgaacg gttttctttg 5220
   gtttagtgaa aggaatggtt tccaaactag agtggattcc cctgtcaatg gtttgccatg 5280
   cattgagcag aaccgagcct tgtgcgtttc ttgtacttca caagttagtg ttcatgaaat 5340
   tgatgaagtc ttgatttact cacttatatt ttcctctaca ttttccagaa atgttacata 5400
   cctgtgtcct gccaaacact ctcatatctc agaaccaccc agaggagcga tcattccgga 5460
   caaggtatga ttcctcaaag gaattcgtca tgaacagtag tccatctcta ctgcatcaaa 5520
   ataaatagaa acttaggttt ttatagcgtt tatgagaatc tacttgcttg catctcaaat 5580
   gtctatgttt tcttaccagt ctatttatct gctgaatatt cattgcaaaa caagtgttct 5640
   tatgaaagtt caattggcag atcttgacgt cagtagatat aaagccattt cctcctttat 5700
   ggcatgaaga caatagcaac cgacgccgac aagcccgaga taggtaagga gattttactg 5760
   cagactgata ttttgaagat catagtgtga attatgttaa aagaaccata tggtatatct 5820
   attgtgcagg ccgcaagtgg ttggagctat agcagggcct tctctgggag aagcagctca 5880
   tcgtctgatc aaaaacaccc ttaacatgaa atcctctact ggtgcggctt cgggattaat 5940
   tgacccaaac ggatactacc gaaatgtacc gggtaattat tcttatggtg gagtcaatag 6000
   accaagagca ccgggtccgt ctccttaccg gaaagcttat gacgatgact cgagctatta 6060
   ctatggaaag tataacaata gcacacaggg aacgtttaac aatggtccaa gatatcctta 6120
   tccatcaaat gggtcacaag actataaccg gaactacaac tccaagatag tagcagaaca 6180
   gcacaaccgt ggtggacttg gagctggaat gtctggttta tcgatagaag ataacggcag 6240
   aagcaaacaa ttgtattcaa gttatacaga agctgctaat gcaaatttga acccgttgcc 6300
   atcacccccc acccaatgga tcggtacaca gccaggtggt aattttgtcg gtggatacta 6360
   tagggatgga gttgggtaca gtgaaacaaa tggaaaatcg gtaaagaaag taatttacca 6420
   ggccaagaca caaccaagtc atcgaggtgc aaacttgtga gatattagtt gaggcgcata 6480
   aggaggttct tctgtacagg tgagttcctt tcttttgctg caatggtctt atgtatggta 6540
   gcatcatctg ccatcagttc tcttaaattt ctggctaaag gcaaatgtat aatgtacctt 6600
   tggacatttc ttttgttata tatacttatt acattaaact tgtttagagt ccttaagaga 6660
   tctcttggca attggatgtt attcgactct ctgtttcttc taccgtgttt cgctattggt 6720
   tttaac 6726
<210> 3
   <211> 966
   <212> PRT
   <213> Glycine max
<400> 3
   Met Gly Val Pro Ala Phe Tyr Arg Trp Leu Ala Asp Arg Tyr Pro Leu
   1               5                   10                  15
   Ser Ile Ala Asp Val Val Glu Glu Glu Pro Ser Val Gly Ser Gly Ala
               20                  25                  30
   Val Asp Val Ser Lys Pro Asn Pro Asn Gly Met Glu Phe Asp Asn Leu
           35                  40                  45
   Tyr Leu Asp Met Asn Gly Ile Ile His Pro Cys Phe His Pro Asp Gly
       50                  55                  60
   Lys Ser Ala Pro Thr Thr Tyr Glu Asp Val Phe Lys Ser Ile Phe Asp
   65                  70                  75                  80
   Tyr Ile Asp His Ile Phe Ser Leu Val Arg Pro Arg Lys Leu Leu Tyr
                   85                  90                  95
   Leu Ala Ile Asp Gly Val Ala Pro Arg Ala Lys Met Asn Gln Gln Arg
               100                 105                 110
   Ser Arg Arg Phe Arg Ala Ala Lys Asp Ala Ala Glu Lys Asp Ala Ala
           115                 120                 125
   Glu Ala Glu Ala Glu Ile Glu Arg Leu Arg Glu Glu Phe Glu Gly Glu
       130                 135                 140
   Met Lys Leu Leu Ser Ser Lys Val Lys Pro Glu Thr Tyr Asp Ser Asn
   145                 150                 155                 160
   Val Ile Thr Pro Gly Thr Pro Phe Met Gly Val Leu Ser Val Ala Leu
                   165                 170                 175
   Gln Tyr Tyr Ile Gln Thr Arg Leu Asn Tyr Asn Pro Gly Trp Arg Asn
               180                 185                 190
   Thr Lys Val Ile Leu Ser Asp Ser Asn Val Pro Gly Glu Gly Glu His
           195                 200                 205
   Lys Ile Met Asp Tyr Ile Arg Leu Gln Arg Asn Leu Pro Gly Phe Asn
       210                 215                 220
   Pro Asn Thr Arg His Cys Leu Tyr Gly Leu Asp Ala Asp Leu Ile Met
   225                 230                 235                 240
   Leu Ser Leu Ala Thr His Glu Val His Phe Ser Ile Leu Arg Glu Val
                   245                 250                 255
   Ile Thr Phe Pro Gly Gln Gln Glu Lys Cys Phe Gln Cys Gly Gln Val
   260 265 270
   Gly His Phe Ala Ala Glu Cys Arg Gly Lys Pro Gly Glu Lys Ala Glu
   275 280 285
   Asp Trp Asn Pro Val Asp Asp Thr Pro Ile His Lys Lys Lys Tyr Gln
   290 295 300
   Phe Leu Asn Ile Trp Val Leu Arg Glu Tyr Leu Gln Tyr Glu Met Glu
   305 310 315 320
   Ile Pro Asn Pro Pro Phe Glu Ile Asp Phe Glu Arg Ile Val Asp Asp
   325 330 335
   Phe Val Phe Leu Cys Phe Phe Val Gly Asn Asp Phe Leu Pro His Met
   340 345 350
   Pro Thr Leu Glu Ile Arg Glu Gly Ala Val Asn Leu Leu Met His Ile
   355 360 365
   Tyr Arg Lys Glu Phe Thr Ala Met Gly Gly Tyr Leu Thr Glu Ala Gly
   370 375 380
   Glu Val Phe Leu Glu Arg Val Glu His Phe Ile Gln Ser Val Ala Val
   385 390 395 400
   His Glu Asp Gln Ile Phe Leu Lys Arg Val Arg Ile Gln Gln Ala Thr
   405 410 415
   Glu Ile Asn Glu Glu Met Lys Ala Arg Ala Arg Gly Glu Met Pro Gly
   420 425 430
   Glu Pro Arg Ala Ser Val Leu Asp Lys Val Lys Leu Gly Glu Pro Gly
   435 440 445
   Tyr Lys Glu Arg Tyr Tyr Ala Glu Lys Phe Gly Ala Leu Asp Leu Glu
   450 455 460
   Lys Ile Glu Lys Ile Lys Lys Asp Thr Val Leu Lys Tyr Val Glu Gly
   465 470 475 480
   Leu Cys Trp Val Cys Arg Tyr Tyr Tyr Gln Gly Val Cys Ser Trp Gln
   485 490 495
   Trp Tyr Tyr Pro Tyr His Tyr Ala Pro Phe Ala Ser Asp Leu Lys Asp
   500 505 510
   Leu Ala Asp Leu Glu Ile Thr Phe Phe Leu Gly Glu Pro Phe Lys Pro
           515                 520                 525
   Phe Asp Gln Leu Met Gly Thr Leu Pro Ala Ser Ser Ser Ser Ala Leu
       530                 535                 540
   Pro Glu Lys Tyr Arg Asp Leu Met Ile Asp Pro Ser Ser Pro Ile Leu
   545                 550                 555                 560
   Gln Phe Tyr Pro Ala Asp Phe Glu Ile Asp Met Asn Gly Lys Arg Phe
                   565                 570                 575
   Ala Trp Gln Gly Val Ala Lys Leu Pro Phe Ile Asp Glu Lys Lys Leu
               580                 585                 590
   Leu Ser Ala Thr Ser Lys Leu Glu Ala Thr Leu Thr Glu Glu Glu Gln
           595                 600                 605
   Leu Arg Asn Ser Lys Met Leu Asp Leu Leu Tyr Val Ser Ser Ala His
       610                 615                 620
   Asn Leu Ala Pro His Ile Leu Ser Tyr His Gln Tyr Ser Cys Gln Leu
   625                 630                 635                 640
   Pro Leu His Glu Arg Pro Ala Leu Pro Ile Asp Pro Arg Ala Ser Ser
                   645                 650                 655
   Asp Gly Met Asn Gly Tyr Leu Trp Leu Tyr Glu Arg Asn Val Leu Arg
               660                 665                 670
   Thr Thr Val Ser Ser Pro Ile Lys Gly Leu Gln Asp Ile Glu Phe Asn
           675                 680                 685
   Gln Val Leu Asn Ile Thr Tyr Leu Asn Pro Arg Lys His Arg His Ile
       690                 695                 700
   Pro Lys Pro Pro Asp Gly Val Leu Met Pro Lys Lys Ile Leu Lys Ala
   705                 710                 715                 720
   Ile Asp Ile Lys Pro Phe Pro Val Leu Trp His Glu Asp Asn Ser Gly
                   725                 730                 735
   Arg Arg Gln Gly Arg Glu Arg Gln Gln Val His Gly Ala Met Ala Gly
               740                 745                 750
   Pro Gln Leu Gly Glu Ala Ala His Arg Leu Val Lys Asn Ser Leu Asn
           755                 760                 765
   Ile Lys Ser Asn Asn Met Pro Tyr Gly Ser Pro Glu Gln Phe Pro Gly
       770                 775                 780
   His His Ala Met Asn Arg Leu Arg Ser Ala Gly Pro Ser Gly Ser Gly
   785                 790                 795                 800
   Lys Tyr Tyr Gly Glu Asp Thr Ser Gly Tyr Tyr Gly Gln His Tyr Asn
                   805                 810                 815
   His Gln Gly Ile Ile Thr Arg Pro Arg Tyr Pro Val Ser Ser Asn Gly
               820                 825                 830
   Gly His Asn Asp Lys Gln Asn Phe Arg Ile Gln Asp Arg Ser Tyr His
           835                 840                 845
   His Asp Gln Tyr Tyr Asn Gly Thr Thr Gly Phe Tyr Thr Met Ala Met
       850                 855                 860
   Glu Glu Gly Ala Arg Ala Arg Pro Tyr Ala Val Pro Leu Pro Arg Thr
   865                 870                 875                 880
   Pro Ala Val Thr Leu Ser Arg Pro Pro Asn Ser Gly Ser Thr Thr Asn
                   885                 890                 895
   Gly Gln His Gln Phe Val Gln Asn Met Gly Pro Pro Ile Pro Pro Pro
               900                 905                 910
   Asn Trp Ile Thr Arg Val Pro Asp Thr Asn Gly Ile Tyr Ala Gly His
           915                 920                 925
   Gln Glu Thr Ala Leu Gly Gly Thr Tyr Asp Lys Gln Asn Met Lys Val
       930                 935                 940
   Tyr Gln Pro Ile Gln Val Leu Gln Trp Ser Met Arg Gly Gln Gln Leu
   945                 950                 955                 960
   Cys Asp Thr Val Arg Gln
                   965
<210> 4
   <211> 11880
   <212> DNA
   <213> Glycine max
<400> 4
   aaggcggcca ccgcagccgc ccgtacatta aacggcaaca cagatctcct ctccatctct      60
   tttttatttt tgttttattt tttcccactc tttctcctcg tttccgaacc ggaccgaacc     120
   gagccggaaa catgggagtt ccagcgttct accggtggct cgcggatcgg tacccactct      180
   cgatcgccga cgtcgtggag gaggagccat ctgtcggctc cggcgccgtt gatgtgtcta      240
   aacctaatcc aaatggcatg gagttcgaca acttgtactt ggacatgaac ggcatcattc      300
   acccctgttt tcaccctgat ggcaaggtaa ccatatgctt cgcttcacct cactttcatt      360
   ctattcactt ttttctcgtg actcagaata ttgcaaccgg ttaacctatt attctgttaa      420
   tagtatcaac attttcttcg ttgtttgaga aaaaagccat tatgtacaat ctaatagaaa      480
   taagtaaaga gagcggaaaa taatgacagt gaacttctga tatttctgct tcaacttctc      540
   atttcgagta tttgtatgga tttctttatt ggatttagat atattgagat cgtatgttcc      600
   tgaattggtt gtcgaaaact cgttctagaa tttgttgcat tgttaaactg agttcctggt      660
   gctttttgaa gaaatggcat catacacgat tatgtgagta gtgtgaaacc ttatacaaag      720
   ctgactagac tgtggcaatt gggatctgta atttctttat tttgcgtcta aaaggaagtg      780
   attttttgta aatttacatt tctattctgc taagtgcttg ttgttgatta atctttaaaa      840
   tgcagtcagc acctactacc tacgaagatg tattcaaatc aatctttgac tatattgacc      900
   atatattttc attggttcgc ccaaggaagc ttctttatct tgcaattggt atgttgcttt      960
   ttcttgttat tgtatctctc gtctatggaa caccaagcat ttcccaatac tttatcttac     1020
   ttgtagatga tgctcatgga agaaataaga ttgacaccat tcctctttgt gagcaataag     1080
   cagaaaatta ggatggcaat gatttataat ttctattttg tttttagtaa tgtcccaaaa     1140
   gcatgttcat tgtttggatt tgagaaatgc atgaatcata tcctatatat tttaagagta     1200
   gtcataatgc cataccagat acatatcata tttgtgcgtc atagtttctc atcgtggagg     1260
   atttgcccaa ctgctccttt aagttataaa tccatttggt tcctaacttc tcattatgcc     1320
   ttctgttttt tttcgatgat tagtaacttt tggcacactc ctgtatggtt gtacattcaa     1380
   aatttagtgc aaatattttc aagtaaaaga ctcagataaa tgtttgtaaa acagaatagc     1440
   cagtaaggaa agttagaact tttaaatatc caatttttgg aagcctgtaa tttttttgtc     1500
   tgtaatgtta tgttattgta tgtttgattt aactttgaat cgcttcctca ttcctaccat     1560
   ttctttccat atgtactttt ctcgcccctt aagacggtgt tgcaccaaga gccaaaatga     1620
   atcagcaacg ttcacggcgt tttcgagccg caaaagatgc agctgagaaa gacgcagctg     1680
   aggctgtaag aatatgaaca tgctcctgtt tacaatgatc tcctgctatt tctgttacaa     1740
   agtttgaata caatgtgatg ttgtaggaag ctgaaattga aagacttagg gaagaatttg     1800
   agggtgagat gaaactcttg tcttctaaag tcaagccaga gacttatgac tcaaatgtca     1860
   ttacccctgg cactccattt atgggagtgc tgtcagtggc tcttcagtat tatatacaga     1920
   ctagattgaa ctacaaccct ggctggcgga atacaaaggt tgaccttatc ctatttattc     1980
   tttgccccct tgatccattc aatattggat attccttact tgtggagtaa tcctgtttat 2040
   tttctgttcc catattgtac atatatctgg ttttcagaat attttgtctc tatatattta 2100
   tttatggagc tgcatttggt attttttgaa ggttatactt tctgattcaa atgttcctgg 2160
   tgagggagaa cataaaataa tggattacat acgattacag cgtaaccttc ctgggtttaa 2220
   tccaaatact cgtcactgtt tgtatggatt ggtgagtttt tgtctgattt tgaaattttc 2280
   aatttatccg aggttggtgt atttaattat atatcttttc cttgattttt atatggtgtg 2340
   ataacattga tgtgatcttt aattcaggat gctgatttga ttatgttgtc cttagccaca 2400
   catgaggttc atttctcaat attgagggag gttaggttct attttatgaa tagtcatggt 2460
   gattttagtt cattttcaaa gagaaagata tcaaatatct gaagttcctt tttatatatt 2520
   atatggatga tccgatccgt aaggaccatg attgggaatt ttttttatca tctttctcca 2580
   agaaagaggc gaaacataat caaatgtcag aaaacttgga gaaaatgaca aatatttttt 2640
   agttttaact ctatggtata gaagaaatag aaaaggatca tataaaagtt ttttaaattt 2700
   aaaacggatc agtacacctg gtctacgaat atattctaat cccaatagat tgacctgatg 2760
   attaattaca tgtatgaaca ggtaatcact ttccctggac aacaggagaa gtgttttcag 2820
   tgtggccaag ttggccattt tgcagctgaa tgtcgtggta agccaggtga gaaagctgag 2880
   gactggaatc cagttgatga taccccaatt cacaagaaga aatatcaggt tggatattgg 2940
   aatccagcat gttaggctga atattcattt tcttctgtta ctttttaatt tcaaactaaa 3000
   accttgcatg tacagtttct taacatctgg gtgctgcgtg aatatttgca atatgaaatg 3060
   gagattccca accctccttt tgagattgac tttgaacgca ttgtggatga ttttgtattt 3120
   ctatgcttct ttgttggcaa tgactttctt ccacatatgc caacattaga gatccgtgag 3180
   gtaagcatct gggaggcacc atgttataaa tgtcaatgtt gtcctctatg ataaactttt 3240
   tactgtttca gggggctgta aatttattga tgcatattta tagaaaagaa ttcaccgcca 3300
   tgggtggtta tcttactgaa gcgggtgagg taaatttctt ttttttcttt cttaaattca 3360
   attaagttgt gtgtgtgcct ttttgtatgt ttttgtaaat ctgtgatgtg ttctggtatg 3420
   tgactgaatc ttttttagca atcatcttcc ttcaatttgc ttgtctcatc tctcaagtca 3480
   cccatctgga attttgtttc ttcaatgact gttgtaatat gttgaacact ctaggttttc 3540
   ttagagagag tagaacattt tattcagtct gttgctgttc atgaagatca aatatttctg 3600
   aaacgagttc ggattcaaca ggtattgtga tttctgtgtt ttgactatcc ctctttggaa 3660
   caaataatta tcattattcc ttcaacttct gaaccctctg gagttttgca ccaatcatat 3720
   tcaataatcg tcagagttct acttatcttc atggttctgt atttagagat gggatatctt 3780
   gcttaatgca ttgtaagaaa gttcttctgt caatattgat gtggaaaaat cttcaattca 3840
   ttccattctg ttttgcattc tgtgtatttt ggcccattgt ttctgttgaa aatttataac 3900
   tgaagcatca caactttatt ccctcatcca tgatttgtgc ttcctatttg aactttggaa 3960
   ttaagtgatt ttgtaagaag acttgaaacg acactgtttc gtatcattta aagtcatatt 4020
   gagcatgtat ggaggaacta aattggcttt ggctactcca cagattaaat aattataaaa 4080
   ggggtataaa ctgtagctga ggcatgaatt tgatatggct gtatcatact taatttggga 4140
   gtggagttct cctctaatgt tccggtgaat aaaggaaatg acttgtaagt agacttcagt 4200
   ggttaggcaa attttggtca gcatgtacag aattatcgag gatctttatc caatgaagtt 4260
   ccagaggaca attccttaat ccttgttcca ctgtactagg gtttaagtat agtgtctccc 4320
   tctaatacgt aactctcaac tttggtggtt acctgttact gacgtgcaag ttcccaagcc 4380
   aacaccaaca tgatactatt gtatttgtta ccatttgttt tcttttctct catatgcact 4440
   gttctgaaac aaattctgat gtggatgtat tgtgacatta gactttacat ccgggagatg 4500
   tcattcacaa gtccgtctaa ttagtttcat gaactgatat gattgagcta tatatttctt 4560
   taatatagtt ttaaacttta tttcctgata tgcaatactt gtgatttgat tgtttttcag 4620
   gctacagaaa ttaatgaaga gatgaaagct agggcaagag gagagatgcc tggagaacca 4680
   cgggcatcag ttttggacaa ggtttggtaa caagattttt tgcccaaatg ttttaatcat 4740
   ttcgcttgaa tttagtattt tctgcttaat tttattggac cctaaaccat agtaattcta 4800
   gtggcttccc aaacatacat tgagggcagc atgtctgtga gtttttgtgc tctgctggtc 4860
   tgtgggtgat cagtgatgcg gttcccactt cctgccactt aatttcagtt tttctcatat 4920
   taaaattgtt gtcaggaagc cttgtaaagc ttgtagtaac tttaaaaact tacgtctgtt 4980
   atgtattttg agaagatact tcaatagtag ttgtgcgtgt tattttgttg ttactattct 5040
   ttgtttcatt tttgtatgta tacatatatt atattattct atataatttt atatataaca 5100
   ggtaaactct acctcccttg tctgatccta ggtgggatct attgttttgg ctacctttgg 5160
   aagtaaggcc gcagagctct tgatgttact tatagtttgc cttgttgcat ttatggctaa 5220
   ccagttatgg ggcaatctga taatttttag tagccatgtt gaagggttta tacttgtact 5280
   tctgttttat cccccattgt tatataaaac atgaactgca tgggcagtgc tggtgccgga 5340
   accaacttca tcatctgcct acacaaaaag agtttgtgtc agtgtgtgtc tgttgatatc 5400
   aatttgaagt taattgctcc ctaatttaac tgtttggatg catgaagaaa atgttttctt 5460
   tgtctctctt ctgaaacaac ttggttacac ttttactttt ctcatgtgtt ttcttttagt 5520
   gggaacaact gatgataact ctgactttcc caactagaat ttgtctttct aaaattaaat 5580
   gttccttttg tcagttgaaa gtagctaaaa gatcctgggt tagaaggatt cataattttt 5640
   ttggttagat acaattcatg taagagtgat ccctgaatag ggacacttgg ccaatgtcag 5700
   accacatgaa atccaatagt gagaaaattt ataaactaca tattgtttgt gattttttca 5760
   ggttaagtta ggagagcctg gatacaagga aaggtattat gctgaaaaat ttggtgcatt     5820
   ggatctggag aagattgaaa aaatcaagaa agatacagta tgtcctgtca gtattcttgg     5880
   aattgtttac tgtcaccttc ctttctatac acattcatgg cttaaaatgg aaacaatttc     5940
   aaaagtacaa attcattgct aagtttttca atactaaaag tcaagtgtcg tattatcagt     6000
   gaaaatgatt gtatacaggg cctgtttact ttgtgaaaat gttttctatt ttcactttca     6060
   attacaagaa tgttgcctta ttttcacatg gtttcctgtt ttcaaaagtt tttttttcac      6120
   tatttcttat ataaactttt taaaacagga aacattgtac aaaaaggtga catttttgta     6180
   attaaaagtg aaagcagaaa acaattttct caaaccaaag aacccttttc tttttaaatg     6240
   atgttagaca catgctgtta gtctgtcttg aactcttgat cttactatct gactgttaca     6300
   caagagggag tgtgctcgta tagttcctca atctgttatg ctcatgagag aataaagacc     6360
   tcacactggt tgaaaattta tgttatgttt ggctcacgaa ttgataggtg gtttgttgga     6420
   ttttatttgt tttttccttc ttgtggatga ctattgtagc caacttcaat aatctatggc     6480
   tttcatattc atatatattg cattagaaga tagctgatag tcttggtttt ccttgtaggt     6540
   cttaaaatat gtagaaggcc tttgttgggt ttgtcggtac tactaccagg gtgtctgctc     6600
   atggcaatgg tttgtcgtgc ttttactagt taatagatta catttgaacc cattcctgct     6660
   attttctgtt actggattga taagctggta catttatgtt ggaagttgcc ttagttgtgg     6720
   ccacccttag cctgccttaa ttgcagcctc aaggtggtgt ttagttccat gttgactaga     6780
   tatataatta aatatagctt atatatatag gcttgggtaa ttgtcatctt acaagccatt     6840
   tttgtggggt ttgagttagg cctttttttt tttgctcagc aaaaataata tattataaaa     6900
   tagtaccaga ggtactaaat aatacagcta tagggacatg tcatacgaag ttaggataca     6960
   gaaatacaaa acacaaactg aaaaacccac agtacaataa ttgagcaaga aactagacta     7020
   tattcctatg ctggtgtcct aagaaatgca gctttgaggt ttgaggacca ctgattgaaa     7080
   tgcaaagaga agtctttttc caagcatctt agccatgtcc acataagaaa cactgcatct     7140
   tccattaatt tatgagcgtc aaaagtagca ttagagaaga tgatgctatt cctatgcttc     7200
   caaatagaaa aagtcagtgc taaccaccac cacagccatc tgatttcctg cattccttca     7260
   acaaccccat ggacatgctg aataaagtgc tgacccgggt gatatgggaa aacccccacc    7320
   atatttaccc aagattgaga ctcccaccaa agaggattaa ttttactgca gtgaaagaat     7380
   aaatgactcg cggtttgagt taggccttaa gccccaaatt ctaagaattt acatgcatga     7440
   tactataatg taaccagttt aggtgaaaaa tgcactttta aacttatgct gtgaaaatat     7500
   ccccactttt ggtccctcaa aggatcaatt gtctggaact atggatgctt agttgacttc     7560
   atgtgattgt taacctgata atatcctagg gcaatgggtg ttttgaaaaa tagatttact     7620
   atgtctgttg aatttagaga ggagacttta gagaaaaaaa gagaacttag aatgaaacaa     7680
   cttatttagt agattgtgta gtacataggt tctgcatatc atccataata tagtttctta 7740
   atagacttag tggagagtat ttcctaggct actattaatt aagtgcaact tcctagacac 7800
   taataccctt gggttctaaa tgcattttat catgcgatac atgaacatac aatttcctaa 7860
   gtaattaaat aacattattt ccaacaacgg gattagtaaa gttttgtcgg ttaactaaac 7920
   attcaattat agtgtgccaa aagcaatggg tacaagtggc ttttcatagt agaatgggta 7980
   caagtgactt ttcatcattt tgtcgcttga gtatgacagt tattcctcat ttgtgaataa 8040
   cactttcttg tgttccttct ccacattgtg gtatgatgat ccaaaaaatt tatgatactt 8100
   tgcatgaagg gtgataagac aaataaaata taaagggttg attattttct ttcaagatta 8160
   tcttgctcca catatgttct tttagcaagt ttctcctgtg ccttcatatt ctctttgaat 8220
   attgagcttt cattaaattc tgttattatt ccaaagatgg ttgcaagcag gaacttctaa 8280
   tcatgatttt ataaggagtg taacaagata taatatggac tggatgcctt gagggctggg 8340
   atacaggctt tgatgtttga gtggctttaa gatgattgac ataaaattct gagactattc 8400
   ttcattttgg tggagtctat cacttatgac tattcaattt tttcattatt gtttgttcat 8460
   tcaggtacta cccataccat tatgctcctt ttgcttctga tcttaaagat ctagctgatc 8520
   tggagataac tttttttttg ggagaaccat ttaaaccatt tgatcagcta atggggaccc 8580
   tacccgcttc aaggtttttt catctctcct gtcctcagat ctattatatc atgcatcctg 8640
   tgctgatgct ggttaagttc ttgttacagt tcaagtgcac ttcctgaaaa atatagggat 8700
   ttgatgattg atccttcatc acctattctt caattctacc ctgctggtga gatcacttgt 8760
   ctgttattga catattttgc tatatcttat agagtttcta gaattacttg tatcactttt 8820
   tgacagattt tgagattgat atgaatggaa aacgatttgc atggcaggta atatagtttt 8880
   tgtattgtgg tttgtgtgtg tgttttaatg ttttattaac actttataat gaagggggat 8940
   taaggttact gttggaaatc tttcagggtg ttgccaagtt gccattcatt gatgagaaga 9000
   aattgctttc tgccacaagc aagcttgaag ccacattaac ggtctggaaa tgtttcttat 9060
   ttttactttt ctgggggagg ggtgttggtt tttacgatat ttagttctgg tctattacac 9120
   tggtgactaa ctatataaga ttagtggtcc aaaattgttg gccttcaaat tgcatcaaag 9180
   aataatttgt caaagaagtt tactgctatg gtttatcaaa atagaagttg ttgaatagat 9240
   ctgttgagtg atacccctct gttaatgctg tttctttttg cacaattatc cccaactctt 9300
   tgctgtgcaa ttgctggaat ttttattttt attttggttt gagctggaaa tatttttagt 9360
   ccactagaag tttacagcag ttaatttgaa taatattttt gcatctgggt gctcaatttc 9420
   attcacattt gttatgtgca ggaggaagaa cagctccgaa atagcaagat gcttgatttg 9480
   ctttatgtca gcagtgcgca taatttggct ccacatatct tatcatatca tcaatattct 9540
   tgtcaattgc ctctacatga aagacctgct ctaccaattg acccaagagc taggttagtg 9600
   aatatgtttg gtaatggtca ccagtcgtta tcataaatat gtttggtaat tgtcatcagt 9660
   catgtgtgcc ttcttttctt tgttagcagt gatggtatga atggatatct ctggttgtac 9720
   gaaaggaatg tgctcagaac cactgtatct tctcccatca agggattgca agacattgag 9780
   tttaatcaag ttttgtaagt tttgttcttt agacttgcct aatattttaa aatcattgaa 9840
   tgaactcatg ttgagtgttt caaatttcag aaatattact tatctcaatc ctcgtaaaca 9900
   tagacatatt ccaaaacctc cggatggtgt actaatgcca aaaaaggttg gtatagactt 9960
   ctcttagcat ttgacattat caatggatac atcctgtaga aggagcttca tgattcattt 10020
   gtttaggctt aaggggggca tttgaaagag ttaatgtgga gttgatttag accatgaaaa 10080
   tagcttgact cccgtaaatt ctttttagct tatttcaata agctttttag tctagcttat 10140
   caaaataagc tctttttagc ttattcccat aagtttcatg ggcaaattta tggcataagc 10200
   ttttatgtga taggagctta ttaaacaaac acctaattaa gctttaccta agcacaccct 10260
   aacatgtgtt taagcccatc ccgatagttt tgatccatta agtggtgaga tcattgccac 10320
   atggcataac ctgatatgcc acatcatcac ttaacagaat acaactacca gcatggacta 10380
   caaacttgca tttccatgaa atcaatgatc taatatattt ttttagggac caaaaatgaa 10440
   aattggctaa tttttcaggg atgaaaaatt tacttaaccc catttattta tgtttagaat 10500
   cagattctcg cgtcaataac atagctcaga tagttgccat gactttattc atttaatcaa 10560
   atattatact ttcgttttag ttttttcaaa gaaaaattat actccttgtt ttcttctatg 10620
   gatagatctc atttactttt gacattggtt atgtttgcag atattaaaag ccattgacat 10680
   taaacctttt cctgtgttat ggcacgaaga caatagtggt cggcgacaag gcagggaaag 10740
   gtgagaatct attcctgagg ttgctatatt ggtgtagtat aattttcatg ttctttctgt 10800
   ctggtataca atccacaact gtttaaattt aatgttcaat gtggtagtgg tactgttatt 10860
   actcaccacc ttatgtggac aaattttttt tgaggaaatt gtcaatatta agtaattgtc 10920
   ctgtggtgaa tgtagagaat tccatgttga gtgggttgat aagttctcaa tgtctttcaa 10980
   atttagcaca tgaagttttt tatgcatgga atctggtatt tatgtgagtg ataagacata 11040
   tactttaaaa gagctcttac ctttcttgtg tgatgcatgt gacagacaac aagtacatgg 11100
   agcaatggct ggccctcaac tgggagaagc agcacatcgt cttgtcaaga actcccttaa 11160
   tattaaatca aataacatgc cgtatggatc accggagcag tttccaggcc atcatgcaat 11220
   gaaccggctc agatcagctg gaccttctgg atctgggaaa tattatggtg aggacacaag 11280
   cggttattac ggacaacatt acaatcacca ggggataata actaggccta gatatccagt 11340
   ctcatctaat ggtggacata atgacaaaca gaattttagg atacaggata ggtcatatca 11400
   tcatgatcaa tactacaacg ggacaactgg gttttatacc atggcaatgg aggaaggtgc 11460
   aagagctagg ccatatgcag tgcctttgcc aaggacacct gcagtgacgt tatcgaggcc 11520
   tcccaattca gggtcaacaa ctaatggaca acaccaattt gtgcagaaca tgggtcctcc 11580
   tataccacct ccaaattgga ttactagagt accggatacg aatggaatat atgctgggca 11640
   tcaagaaact gcattggggg gaacttatga taagcagaac atgaaggtgt accaagttaa 11700
   aactcgtcaa ccccaagata tgccagagta cgggaatcaa tgatgactgt tgtggatttt 11760
   attcgccttg tgacgaagtg ccctcttcca ttcctcgggc tgtttctcaa atcggagccc 11820
   atccaagttc tacaatggag tatgaggggg caacaattgt gtgataccgt aaggcaatag 11880
<210> 5
   <211> 962
   <212> PRT
   <213> Malus domestica
<220>
   <221> misc_feature
   <222> (148) .. (148)
   <223> Xaa can be any naturally occurring amino acid
<220>
   <221> misc_feature
   <222> (180) .. (180)
   <223> Xaa can be any naturally occurring amino acid
<220>
   <221> misc_feature
   <222> (218) .. (218)
   <223> Xaa can be any naturally occurring amino acid
<220>
   <221> misc_feature
   <222> (608) .. (608)
   <223> Xaa can be any naturally occurring amino acid
<220>
   <221> misc_feature
   <222> (645) .. (645)
   <223> Xaa can be any naturally occurring amino acid
<220>
   <221> misc_feature
   <222> (941) .. (941)
   <223> Xaa can be any naturally occurring amino acid
<400> 5
   Met Gly Val Pro Ala Phe Tyr Arg Trp Leu Ala Asp Arg Tyr Pro Gln
   1               5                   10                  15
   Ser Ile Ala Asp Val Val Glu Glu His Ala Arg Glu Asp Ser Asn Gly
               20                  25                  30
   Val Pro Val Pro Val Asp Val Ser Gly Pro Asn Pro Asn Gly Ser Glu
               35                  40                  45
   Phe Asp Asn Leu Tyr Leu Asp Met Asn Gly Ile Ile His Pro Cys Phe
       50                  55                  60
   His Pro Asp Gly Lys Pro Ala Pro Ala Thr Tyr Asp Asp Val Phe Lys
   65                  70                  75                  80
   Ser Ile Phe Asp Tyr Ile Asp His Leu Phe Thr Leu Val Arg Pro Arg
                   85                  90                  95
   Lys Leu Leu Phe Leu Ala Ile Asp Gly Val Ala Pro Arg Ala Lys Met
               100                 105                 110
   Asn Gln Gln Arg Ser Arg Arg Tyr Arg Ala Ser Lys Asp Ala Ala Glu
           115                 120                 125
   Ala Glu Ala Glu Glu Glu Arg Leu Lys Lys Glu Phe Glu Met Glu Gly
       130                 135                 140
   Thr Ser Leu Xaa Ser Lys Glu Lys Pro Glu Thr Leu Asp Ser Asn Val
   145                 150                 155                 160
   Ile Thr Pro Gly Thr Gln Phe Met Ala Val Leu Ser Val Ala Leu Gln
                   165                 170                 175
   Tyr Tyr Ile Xaa Ser Arg Leu Asn His Asn Pro Gly Trp Arg Ser Thr
               180                 185                 190
   Lys Val Ile Leu Ser Asp Ala Asn Val Pro Gly Glu Gly Glu His Lys
           195                 200                 205
   Ile Met Ser Tyr Ile Arg Leu Gln Arg Xaa Leu Pro Gly Phe Asp Pro
       210                 215                 220
   Asn Thr Arg His Cys Leu Tyr Gly Leu Asp Ala Asp Leu Ile Met Leu
   225                 230                 235                 240
   Ala Leu Ala Thr His Glu Val His Phe Ser Ile Leu Arg Glu Val Ile
                   245                 250                 255
   Thr Phe Pro Gly Gln Gln Glu Lys Cys Phe Leu Cys Gly Gln Val Gly
               260                 265                 270
   His Leu Ala Ala Glu Cys Arg Gly Ile Ser Asp Asp Asn Gly Asn Val
           275                 280                 285
   Val Asp Asp Thr Pro Ile His Gln Lys Lys Tyr Gln Phe Leu Asn Ile
       290                 295                 300
   Trp Val Leu Arg Glu Tyr Leu Gln Tyr Glu Leu Asp Ile Pro Asn Pro
   305                 310                 315                 320
   Pro Phe Gln Leu Asn Phe Glu Arg Ile Val Asp Asp Phe Val Phe Met
                   325                 330                 335
   Cys Phe Phe Val Gly Asn Asp Phe Leu Pro His Met Pro Thr Leu Glu
               340                 345                 350
   Ile Arg Glu Gly Ala Ile Asn Leu Leu Met His Ile Tyr Arg Thr Glu
           355                 360                 365
   Phe Thr Ala Met Gly Gly Tyr Leu Thr Asp Ala Gly Glu Val Leu Leu
       370                 375                 380
   Asp Arg Val Glu His Phe Ile Gln Ser Val Ala Val Phe Glu Ser Gln
   385                 390                 395                 400
   Ile Phe Gln Lys Arg Val Arg Ile Gln Gln Ala Ile Glu Asn Asn Glu
                   405                 410                 415
   Glu Arg His Arg Ala Arg Arg Glu Thr Ser Ala Glu Leu Pro Ala Pro
               420                 425                 430
   Val Met Asp Lys Val Lys Leu Gly Glu Pro Gly Tyr Thr Glu Arg Tyr
           435                 440                 445
   Tyr Ala Glu Lys Phe Gln Val Ser Thr Pro Glu Glu Ile Asp Lys Val
       450                 455                 460
   Lys Lys Asp Leu Val Leu Lys Tyr Val Glu Gly Leu Cys Trp Val Cys
   465                 470                 475                 480
   Arg Tyr Tyr Tyr Gln Gly Val Cys Ser Trp Ile Trp Tyr Tyr Pro Tyr
                   485                 490                 495
   His Tyr Ala Pro Phe Ala Ser Asp Leu Lys Asp Leu Asn Glu Leu Glu
               500                 505                 510
   Ile Thr Phe Phe Leu Gly Glu Pro Phe Lys Pro Phe Asp Gln Leu Met
           515                 520                 525
   Gly Thr Leu Pro Ala Ala Ser Ser Ser Ala Leu Pro Glu Lys Tyr Arg
       530                 535                 540
   Asn Leu Met Thr Asp Pro Arg Ser Pro Ile His Asn Phe Tyr Pro Ala
   545                 550                 555                 560
   Asp Phe Glu Ile Asp Met Asn Gly Lys Arg Phe Ala Trp Gln Gly Val
                   565                 570                 575
   Ala Lys Leu Pro Phe Ile Asn Glu Arg Lys Leu Val Thr Glu Thr Arg
               580                 585                 590
   Lys Leu Glu Ser Thr Leu Thr Glu Glu Glu Gln Ala Arg Asn Ser Xaa
           595                 600                 605
   Met Leu Asp Leu Leu Tyr Val Tyr Pro Ser His Pro Leu Ala Ala Gln
       610                 615                 620
   Ile Ser Met Tyr Tyr Gln Gln Phe Tyr Gln Thr Pro Leu His Glu Arg
   625                 630                 635                 640
   Phe Leu Trp Arg Xaa Asp Pro Ile Ala Ser Gly Gly Met Asn Gly Phe
                   645                 650                 655
   Leu Gly Leu Ser Glu Arg Asn Gly Leu Arg Tyr Val Ile Pro Ser Pro
               660                 665                 670
   Ile Arg Gly Tyr Pro Asp Leu Asp Cys Asn Gln Val Leu Asn Ile Thr
           675                 680                 685
   Tyr Leu Asn Pro Ala Asp His Lys His Ile Pro Glu Pro Pro Lys Gly
       690                 695                 700
   Val Ile Leu Pro Arg Lys Val Val Arg Pro Thr Asp Ile Lys Pro Phe
   705                 710                 715                 720
   Pro Thr Leu Trp His Asp Glu Gly Pro Arg Arg Gln Gly Arg Glu Arg
                   725                 730                 735
   Pro Gln Ile Pro Gly Ala Ile Ala Gly Pro Met Leu Gly Glu Ala Ala
               740                 745                 750
   His Arg Leu Val Lys Asn Thr Leu Asn Ile Lys Pro Asn Gly Ser Ser
           755                 760                 765
   Pro Gly Leu Trp Asp Gln Pro Pro Leu Arg Asn Ser Gly Asn Tyr Ala
       770                 775                 780
   Gly Asn Tyr Pro Val Asn Arg Pro Arg Pro Ala Gly Pro Ser Gly Tyr
   785                 790                 795                 800
   Glu Arg Val Phe Arg Glu Glu Ala Thr Tyr Gly Asn Ser Phe Asn Pro
                   805                 810                 815
   Gln Val Met Met Ala Arg Pro Arg Ile Pro Ala Ser Asn Gly Met Gln
               820                 825                 830
   Gly Asp Arg Gln Asn Phe Arg Ala Gln Glu Arg Val Gln His Gln Glu
           835                 840                 845
   Gln Asn Phe Arg Thr Gln Asp Arg Val Gln Asp Gln Glu Gln Tyr Asn
       850                 855                 860
   Leu Arg Ser Val Met Ser Ser Leu Thr Val Gln Asp Ser Gly Arg Thr
   865                 870                 875                 880
   Arg Ser Pro Ala Ala Ala Pro Pro Gly Met Pro Asn Ser Gly Tyr Gln
                   885                 890                 895
   Ala Asn Arg Lys His Gln Phe Val Gln Asn Met Gly Ala Leu Pro Ser
               900                 905                 910
   Pro Pro Thr Lys Trp Ile Ser Lys Glu Pro Thr Gly Asn Gly Gly Leu
           915                 920                 925
   His Val Arg Lys Gln Glu Thr Gly Tyr Gly Gly Ala Xaa Glu Gln Gln
       930                 935                 940
   Gln Val Lys Lys Val Tyr Gln Val Lys Thr Arg Ala Pro Gln Asp Val
   945                 950                 955                 960
   Lys Pro
<210> 6
   <211> 8757
   <212> DNA
   <213> Malus domestica
<400> 6
   atgggcgttc ctgcgtttta ccgctggcta gcggatcggt acccacagtc catcgccgac      60
   gtcgtggagg agcatgccag agaggactcc aatggcgtcc ctgtgccagt cgacgtctcc     120
   ggaccgaacc ctaatgggtc cgagttcgac aacttgtacc ttgacatgaa cggcattata     180
   catccctgtt ttcatcccga tgggaaggtt cgctctctct ytttcctctg caatttccaa     240
   tttccgttcg ttttgttgta aaatgttgat tttggtgctc tcagaggtag aatttagctt     300
   aatttgtgtc taatggcaat tttttgttgt acaaaactat gggttttgga ggtgtttgtt     360
   tgggttgctt caacaatttc gtactttttg ttgcagtcgg tgagaattga gttccagatt 420
   gattgtttgc atgttatttc tgtcgtgttt gcgtttgatg atcgaagtga ttgattttgg 480
   tagtctttag aggaaaaatt tagtcttcag ttatacacat atacatatac atatgtggct 540
   tgacaatact taaatttagt gcctttttct tgtgggaagt acgtgactcc gttgtttatc 600
   ttgcatatgt tgacttaaat attgacttgg actgtcagat tgggtaaaag ttatcttgtt 660
   gtttatgtgg gtgtttgaaa tgtgggsatt taccaggtta ttatgtgctt tgagtgacct 720
   ctcgatgagg aaatgccttt gcttaaagtt gtattgtgat gacatgttaa ttttgtgtgt 780
   gtgtgcgtgt gtgcagcctg ccccygctac ctatgatgat gtattcaaat caatatttga 840
   ttacattgat catctattca cattggttcg cccaagaaag cttctttttt tggcaattgg 900
   taagatttaa cttaagacat gttaagtagt ttatctataa tgttttttct tgcgctaaat 960
   ttagtacttc atatatttct aatcttgtag atggggttgc tccaagagcc aaaatgaatc 1020
   agcaacgttc tcgacgttac agagcttcaa aagatgcggc tgaagcggta acatcttact 1080
   cactgtgcat aattagttcc tatccaggga ggcagtgaac ttattgttgt ttttcttaaa 1140
   tccttatgtt aggaagctga agaggaaaga cttaagaaag aatttgagat ggaggggaca 1200
   tctttgsctt ctaaagagaa acctgaaact ttagactcaa atgtcataac cccgggcact 1260
   caatttatgg cagtgctctc ggtggcactt caatattaca tacawagtag gttgaaccac 1320
   aaccctggtt ggcggtctac gaaggttggt ttgacatcat tgcctgtatt gatttgcaat 1380
   tgcctccctc agtcccttca agatcagatg gttctctgtc trtggagtaa tccattctct 1440
   atatttttac catgtacttc tggttttcag ggtactttgt ctctctaaat ttatttatgg 1500
   ggctgcattg ggtggttatt tgaaggttat actttctgat gcaaatgttc ctggcgaggg 1560
   agagcacaaa ataatgtcat acatccgatt gcaacgcarc ctccctggtt ttgatcctaa 1620
   cactcgacay tgtctttatg gtttggtgag tgtttattat ttaggccttt ttttgcgagt 1680
   cttttatttg ctgttatcat tatcaggctt ttgtaaataa tttttttttg ccgtgtgtgt 1740
   atgcatgttt tatgtccttt gaatgggaag ccattttgta tttcctttgc tgcattgttt 1800
   agttctaaaa tgggwatttg gatcatcata atcaggatgc agatctaatt atgctggctt 1860
   tagctacaca tgaggttcac ttctcaatat taagagaggt agttgctctt tcttcagaac 1920
   attttctaag ttgtttatta caagtggttg atcttataga ttgtttacca aaattgtaca 1980
   ggtgattact tttccaggtc aacaagaaaa atgttttctt tgtggacaag tkggtcatct 2040
   ggcagctgag tgtcgtggta tatctgatga taatggaaac gtggttgatg atacrccaat 2100
   tcaccagaag aaatatcagg ttgaagaaat ggatcctgct tctcgttgct accctttttg 2160
   aattcctctt gatatcattc tcatattatt cctaaattat attttgtttg tgcagttcct 2220
   caacatatgg gtattgcgtg aatatttgca atatgagctg gatattccca accctccttt 2280
   tcagctcaat tttgaaagga tagtggatga ttttgttttt atgtgctttt ttgttggcaa 2340
   tgactttctt ccacatatgc caacattgga gatcagagag gtacgttatt atagctagca 2400
   gtctgttgct ttgattttca gaagtytgcc aattttaggg gtttaggtac taagtttatt 2460
   rcattctcgt caaattaaag tgtctaagat ttgttaactt ttaccgtatc aaacacatga 2520
   ttgttcactg ctccttctat tggttgttca cttttctttt ttacttgatg aaaggataaa 2580
   gttttggccc tttcacatgc aagcccttga taacattgca ctttagataa aagcacttca 2640
   aacgcaatgc tttagaaaaa gacctctgac tttgtaatgg aagaaggcca ccaaaggaat 2700
   ttcacagcta ataatcaatt aaagcatgac accaaaggaa tttcacagct aataatcatt 2760
   aaagcatgaa atatgtatrg attaggtttt ttttccctca agttgatctt ttgaggtttt 2820
   ttgtataact tgtagttgtt ggkgtatttt ctaatttact attaactttt tactgcctca 2880
   gggtgctatt aatttattga tgcatattta tagaacggag ttcactgcta tgggaggtta 2940
   tcttactgat gcgggtgagg taaattgctc ttctttacat gtctgttact cttgtatctc 3000
   attgcatttg ccataaatgc gttctgatgt gtcaattgca gtttcttatg tatgatgttt 3060
   gtaatgggtg ctacatcaag tttctcttgt tttttatatt tgaaaaagta tcaactgttt 3120
   ggatgttaac attttttaag tgagtgaaag atcttaaatg cacccatgaa tgagggttaa 3180
   tgaatgccat ttctatcatt gctactataa caatattggg aagacctact tttgtaatgt 3240
   aggagactgg tgcacttaaa tcttattatc gatctaatac attagctcac cgtagtagac 3300
   tgtaacttta attattggag agccggtcct atagatctgt ggatgactgc agtattgcgt 3360
   gtaaatggga tacaatgcct gatattgaaa cacaatcaag atagcaatga aaggaaagag 3420
   tggaacaggg ttattcatat ttgtcagatg tgttcattat gtgtaaaata ttttaatctc 3480
   tctctctcac ccccttgcac ttaaaaatgt ggatgcttgc tctgttattg cttttgtttg 3540
   ttttgaatat gtttctatga agaacttgtg cataacattt taatatatta tcttttggca 3600
   aaaatctttt tgatctatat ttaaccctag ttgattttag tgratattgg taccatgcat 3660
   gtgcttatcc atttgagttt ctctcacagg tattgctaga cagagtagag cattttattc 3720
   agtctgttgc tgtttttgaa agccaaatct ttcaaaagcg ggttcgaatt cagcaggtct 3780
   tcckcttctt tctgatttaa tgtgtactat cagttcattt gtttgtcaat ttactggagc 3840
   atcatgaaac caagggatga tgacaaattt aagcatgata aagaatttac tcctttcact 3900
   gcaaaattgt tgttcgtcca ttagtttctt agtcttattt ttgtgggaag ttttttgtca 3960
   atactgatac ctagtggact cttttaagtt tcttttggtg attacttgat tacaacggaa 4020
   ttcatctggt cttaaatatt ttcccattag aatgagtgag atgtttggtt aaacaaaaat 4080
   gaaaacttcc tatgagatgt aaatgatggt agttaaataa gcctttctag caaaaggcaa 4140
   gaaatttrta ggtataagct tttgtagggg crtttgtkca cttgatattt ctttgatgaa 4200
   aggaaatttg aacacgatga taaatcataa ttatgtagtg acttatgtgt tatgtcctgg 4260
   csttatgtgg atccatttga cagaaaagtt aatccactca catttagcac tatgaatttc 4320
   gctwcatgat rtgtgaacca aaggcttaga gcctttcttt tataaccata tttgagccat 4380
   ggaagtgaac ttttgttctg ggggttaatt gtgggattat ataggtcact taatcatgct 4440
   aygggtatcc aaaactattt attggtyrga aatttgctcc tttagtcaat catacatgtc 4500
   tttcttcatr ctgatgatga ttatattgca ggctattgag aataatgaag agagacatag 4560
   agctagaaga gaaacttcag cagagctacc ggctccggtg atggayaagg tctgcaatat 4620
   aattgcaact gtttgttcaa attttaaaat aatacagtga taaaataact tagctttttg 4680
   tgccatctat tgtacatttg ttcactcatt tcactggaaa gtcatgaaga gactgaaagc 4740
   ttctttgaag ggcaggccaa gtaattaagg aatgtatggt gtttgtgcgt ctgattttat 4800
   tttgtttctc aggttaaatt gggagagcct ggatatacag aaaggtatta tgctgagaaa 4860
   tttcaagtat caacaccaga agagattgat aaagttaaaa aggacttggt aagctctctc 4920
   aggcgaraca ttttagttat attatttggc tgtggatatg gttttgtttt tggatattac 4980
   actacttatt tttgcttttc atgtgaacgg atatctttat tgaatcatct gccatatata 5040
   ttttggattg aacctkggag gtttaagctg tgcgtggggt ctgtagctct acccatctat 5100
   agttggcttt ttttttcttw ggagaaaaaa aaawagtgat cgaggaaaaa ggttaaawca 5160
   ctgtgatcag gtctgtactt agstatgaac agtcggtaat ttaggagatc agaagatttc 5220
   atatgttttg agtttaggag attcttgttg ggttcagttg ctgccttcat gccattctaa 5280
   tgaacttaat ctaatttaaa agcttttcgt ctttttgctg ttgtttcttc ctggtttgat 5340
   aggacaagta ctaagttact ctatgctaaa atatttttgt ttctttgcag gttttaaaat 5400
   atgtagaagg cttatgttgg gtttgccgat attattacca aggagtctgc tcatggatat 5460
   ggttagtttg actttaacct tttgcttctt ttacctgctt tgcctcatct tattgctgat 5520
   ctggaggtct gactwctcta tctggaagtt ggatgggtga catgttgagc caccgtgttg 5580
   actggattga tatccagttc agtttcaata actttgcttg tatctgtaat cgtttttttt 5640
   ttcaggtatt atccatacca ttatgctcct tttgcttctg atctaaaaga tttaaatgaa 5700
   ctggaaataa catttttctt gggagagcca tttaagccct tcgatcagct aatgggaacc 5760
   ctaccagctg caaggtctct ctcatttgtg gctttgaatt tattcaaata agttatttat 5820
   atatagaaga tttcatcttc tagatatgat aatgctcagt atttctattt tacagctcta 5880
   gtgctcttcc ggaaaaatac agaaacttaa tgactgatcc aagatcacct atccataatt 5940
   tctatcctgc tggtaataat tgtctagttg tatggatctt tttatactgt gaagttgtat 6000
   cctatctgat aaatattttc cttttgaaat atctcactcc agattttgaa attgacatga 6060
   atggcaagcg ctttgcatgg caggtatgat cttctgtagt gttgttgttt twctttattt 6120
   tttaaatttg tgtttgtttt tccttttgga ggggtaaact aatggtagwt tgatcacttt 6180
   atgtgtctgt gtgtgtgtgt gtgtttggaa gtcagggtgt tgccaaattg ccgttcatta 6240
   acgagaggaa attggttact gagacaagga agcttgaaag cactttaacg gtatggaaga 6300
   atattcattt tcttcctaat aatttttgca tcaattatac ttcatgttca tatgtttact     6360
   tgtcaaattt atacccctta aaatggattt tgtataccaa tacttatgta aagcattggt     6420
   ttttacaagt tctaggttag cttgcaagag ttatatttca gtttttacaa tcagggtttt 6480
   tagggaaaca tatggcacca tgttctgata gactgaattt tggggttgtg agaatgacat 6540
   gagatattaa tccatcctat catgcctatc tmttctaggt ctgaacaaat agtattgaaa 6600
   ttattggcta tgtrtatgta ttgataattt gagattgaaa tgcctgaaaa gttgatggtc 6660
   tttggaatca tggattacat ttgggtcaga gtaggagtta aagatcttgt atggtgggct 6720
   gcaatgagcc tacgacttat ctaggattct ctatcttacc gtagggctat tttggacatc 6780
   ttttaactgc aataaccacg ataagttggc cctcatttta ctactacatt ttgagggttc 6840
   atactctttg tcatatgcag gaagaagagc aggctcggaa tagcrtgatg cttgatttgc 6900
   tttatgttta tccttctcat cctttggcag cacaaatcag tatgtactac caacagtttt 6960
   atcagacacc cctacatgaa agatttttat ggcggrtaga cccaattgct aggtaggtta 7020
   ttttagtagt aatttatggg gactcatggt ctaattgtca ctgtggaatt tgcttgactt 7080
   gtgaccctct tgtttctttt tctttttttt tcagtggygg aatgaatgga ttccttgggt 7140
   tatctgagag aaatggctta agatatgtaa tcccttcccc tattcgtggt tatcctgacc 7200
   ttgattgtaa ccaagtcttg taagtagagc ctctactatt gttgtttaag tatttaaaga 7260
   gaatacagtg ttttcatggt gggacttttc gtatgcagaa atattacata tcttaatccg 7320
   gcagatcata aacacatccc agaacctccc aagggtgtga tcctgcccag aaaggtatct 7380
   ggattcttta aagttcttgt ccaagtctta cagaaatttt ttcgttgtag tccatgtcat 7440
   tacgttttac atggcatgtc aaatacctat tgctcatacc cccctgtgaa gacattcgtt 7500
   cttcccrtta aagctgggat atgtgatata tcaggttcct gtggttggtg tttctttggt 7560
   tcatggtttg catacatgaa aatattacat ccttgtggct ttkttcaatg tgaattttct 7620
   aatgaaaaat attatgttat ttctattttc ttatggttaa aaaaatgtga gtagctgcat 7680
   ataagttgag cttttcatta tcacttcatg tgttgattta tcgtcacatt gctctgtgyt 7740
   tggagatgct aagacagttg ggaatatgct tcaatttctt gtcctctttt tctgttatct 7800
   tttctattac acttgtttat cagggggatc taatttggat tgtcctttta aatgtcttta 7860
   ggttgtaaga cctacggaca tcaaaccatt ccctacgtta tggcacgacg aaggtcctcg 7920
   acgccaaggc agagagaggt gagttgaatt ccatatgtcc ggattttaca tgttttttgc 7980
   ttagtaacaa gatctatatc tgaggatatt tatcttataa ctgcaactcg gggctctgga 8040
   ttgtggtttc tgaacctgat gcgtttgttt tataggccac aaatacctgg agcaattgct 8100
   ggacccatgt tgggagaagc agctcaccgc ctggtcaaga acacccttaa tattaaacct 8160
   aatggttcat cccctggatt atgggatcaa ccgccattac gaaattccgg aaattatgca 8220
   ggaaattatc cagtcaacag accaaggcca gctgggccct ctgggtatga aagggtcttc 8280
   cgcgaagaag caacgtatgg aaattccttc aatcctcagg tcatgatggc taggcctaga 8340
   attcctgcat caaatggtat gcaaggtgac agacagaatt ttagggcgca agaaagagta 8400
   caacatcaag aacagaattt taggacacaa gatagagtac aagatcaaga acagtacaac 8460
   ctgagatccg tgatgtckag tttaacagtr caagacagtg ggagaaccag gtcacctgca 8520
   gcggcgccac ctgggatgcc aaattcagga taccaagcaa accgtaagca ccagtttgtg 8580
   cagaacatgg gtgctcttcc ttcgccacct accaagtgga ttagcaaaga accaactgga 8640
   aatggtggat tacatgtaag gaaacaggaa actggatatg gtggagcatr tgagcaacag 8700
   caggtcaaga aggtctatca agttaagacg cgggctcccc aggatgtcaa accgtag 8757
<210> 7
   <211> 1068
   <212> PRT
   <213> Oryza sativa japonica
<400> 7
   Met Gly Val Pro Ala Phe Tyr Arg Trp Leu Ala Glu Lys Tyr Pro Met
   1               5                   10                  15
   Val Val Val Asp Val Val Glu Glu Glu Ala Val Glu Ile Glu Gly Val
               20                  25                  30
   Lys Val Pro Val Asp Thr Ser Lys Pro Asn Pro Asn Gly Leu Glu Phe
           35                  40                  45
   Asp Asn Leu Tyr Leu Asp Met Asn Gly Ile Ile His Pro Cys Phe His
       50                  55                  60
   Pro Glu Asp Arg Pro Ser Pro Thr Thr Phe Ala Glu Val Phe Gln Cys
   65                  70                  75                  80
   Met Phe Asp Tyr Ile Asp Arg Leu Phe Val Met Val Arg Pro Arg Lys
                   85                  90                  95
   Leu Met Tyr Met Ala Ile Asp Gly Val Ala Pro Arg Ala Lys Met Asn
               100                 105                 110
   Gln Gln Arg Ser Arg Arg Phe Arg Ala Ala Lys Asp Ala Ala Asp Ala
   115 120 125
   Ala Ala Glu Glu Glu Arg Leu Arg Glu Glu Phe Glu Arg Glu Gly Arg
   130 135 140
   Lys Leu Pro Pro Lys Gln Gln Ser Gln Thr Cys Asp Ser Asn Val Ile
   145 150 155 160
   Thr Pro Gly Thr Glu Phe Met Ala Val Leu Ser Ile Ala Leu Gln Tyr
   165 170 175
   Tyr Ile His Leu Arg Leu Asn Tyr Asp Pro Gly Trp Lys Gln Val Lys
   180 185 190
   Val Ile Leu Ser Asp Ala Asn Val Pro Gly Glu Gly Glu His Lys Ile
   195 200 205
   Met Ser Tyr Ile Arg Gly Gln Arg Asn Leu Pro Gly Phe Asn Pro Asn
   210 215 220
   Thr Arg His Cys Leu Tyr Gly Leu Asp Ala Asp Leu Ile Met Leu Ala
   225 230 235 240
   Leu Ala Thr His Glu Val His Phe Ser Ile Leu Arg Glu Val Val Tyr
   245 250 255
   Thr Pro Gly Gln Gln Asp Lys Cys Phe Leu Cys Gly Gln Val Gly His
   260 265 270
   Leu Ala Ala Asn Cys Glu Gly Lys Val Lys Arg Lys Ala Gly Glu Phe
   275 280 285
   Asp Glu Lys Gly Glu Ala Ile Val Pro Lys Lys Pro Tyr Gln Phe Leu
   290 295 300
   Asn Ile Trp Thr Leu Arg Glu Tyr Leu Glu Tyr Glu Phe Arg Met Gln
   305 310 315 320
   Asn Pro Pro Phe Pro Ile Asp Phe Glu Arg Ile Val Asp Asp Phe Ile
   325 330 335
   Phe Met Cys Phe Phe Val Gly Asn Asp Phe Leu Pro His Met Pro Thr
   340 345 350
   Leu Glu Ile Arg Glu Gly Ala Ile Asn Leu Leu Met Ala Val Tyr Lys
   355 360 365
   Lys Glu Phe Pro Ser Met Gly Gly Tyr Leu Thr Asp Ala Cys Thr Pro
   370 375 380
   Asp Leu Asn Lys Val Glu His Phe Ile Gln Ala Val Gly Ser Tyr Glu
   385 390 395 400
   Asp Lys Ile Phe Gln Lys Arg Ala Arg Leu His Gln Arg Gln Ala Glu
   405 410 415
   Arg Ile Lys Arg Glu Lys Ala Gln Ala Lys Arg Gly Asp Asp Leu Asp
   420 425 430
   Pro His Val Arg Asp Asp Leu Ile Val Pro Val Ala Arg Phe Gln Gly
   435 440 445
   Ser Arg Leu Ala Ser Gly Pro Val Pro Ser Pro Tyr Glu Gln Asn Gly
   450 455 460
   Ser Asp Lys Asn Asn Gly Gly Lys Asn Ser Arg Ala Arg Lys Ala Ala
   465 470 475 480
   Arg Val Ser Ser Ser Gly Ser Ser Ile Ala Ala Ala Ile Val Glu Ala
   485 490 495
   Glu Asn Asp Leu Glu Ala Gln Glu Arg Glu Asn Lys Glu Asp Leu Lys
   500 505 510
   Thr Met Leu Lys Asp Ala Leu Arg Glu Lys Ser Asp Val Phe Asn Ser
   515 520 525
   Glu Asn Pro Glu Glu Asp Lys Ile Lys Leu Gly Glu Pro Gly Trp Arg
   530 535 540
   Glu Arg Tyr Tyr Glu Glu Lys Phe Gly Ala Arg Thr Pro Gly Gln Ile
   545 550 555 560
   Glu Glu Ile Arg Arg Asp Val Val Leu Lys Tyr Thr Glu Gly Leu Cys
   565 570 575
   Trp Val Met His Tyr Tyr Tyr Glu Gly Val Cys Ser Trp Gln Trp Phe
   580 585 590
   Tyr Pro Tyr His Tyr Ala Pro Phe Ala Ser Asp Leu Ser Gly Leu Gly
   595 600 605
   Gln Leu Asn Ile Thr Phe Glu Leu Gly Ser Pro Phe Lys Pro Phe Asp
   610 615 620
   Gln Leu Met Gly Val Phe Pro Ala Ala Ser Ser His Ala Leu Pro Val
   625 630 635 640
   Gln Tyr Arg Gln Leu Met Thr Asp Ala Asn Ser Pro Ile Ile Asp Phe
   645 650 655
   Tyr Pro Thr Asp Phe Glu Val Asp Met Asn Gly Lys Arg Tyr Ser Trp
   660 665 670
   Gln Gly Ile Ala Lys Leu Pro Phe Ile Asp Glu Ala Arg Leu Leu Ala
   675 680 685
   Glu Ile Lys Lys Val Glu His Thr Leu Thr Pro Glu Glu Ala Arg Arg
   690 695 700
   Asn Ser Ile Met Phe Asn Met Leu Phe Val Asn Gly Ser His Pro Leu
   705 710 715 720
   Ser Pro Tyr Ile Tyr Ser Leu Asn Ser Lys Phe Gly His Leu Pro Asp
   725 730 735
   Arg Glu Arg Asn Glu Ile Lys Glu Lys Ile Asp Pro Ser Ser Ser Gly
   740 745 750
   Gly Met Asn Gly Tyr Ile Ser Leu Cys Ser Gly Asp Pro Cys Pro Pro
   755 760 765
   Val Phe Arg Ser Pro Val Asp Gly Leu Glu Asp Ile Met Asp Asn Gln
   770 775 780
   Val Ile Cys Thr Ile Tyr Lys Leu Pro Asp Ser His Lys His Ile Ala
   785 790 795 800
   Arg Pro Pro Val Gly Val Ile Ile Pro Lys Lys Thr Val Glu Ala Thr
   805 810 815
   Asp Leu Lys Pro Pro Pro Val Leu Trp His Glu Asp Ser Gly Arg Arg
   820 825 830
   Pro His Asp Asn Asn Asn Arg Arg Pro Tyr Glu Asn Ser Asn Arg Gln
   835 840 845
   Asn Pro Ala Gly Ala Ile Ser Gly Arg Gln Leu Gly Glu Ala Ala His
   850 855 860
   Arg Leu Val Val Asn Ser Leu Asn Ala Arg Ser Gly Gly Gln Tyr Asn
   865                 870                 875                 880
   Thr Pro Ser Met Pro Tyr Gln Thr Ile Met Asn Gly Met Pro Tyr Pro
                   885                 890                 895
   Asn Gly Ile Pro Pro Arg Met Glu Gln Pro Ala Pro Gly Trp His Val
               900                 905                 910
   Pro Gly Asp Leu Pro Asn Gly Gln Val Pro Pro Ala Tyr Ala Ser Ser
           915                 920                 925
   Ser Gly His Tyr Gln Asn Asp Arg Ser Gly Pro Ser Gln Tyr Gly Arg
       930                 935                 940
   Asp Asn His Gly Arg Tyr Pro Tyr Ala Arg Asp Asn His His Asp Ser
   945                 950                 955                 960
   Arg Gly Arg Val Pro Pro Tyr His Gln Ser Gly Gly Asn Ser Tyr Gln
                   965                 970                 975
   Ser His Ser Ala Pro Ser Ala Gly Pro Gly Arg Tyr Ala Gln Pro Pro
               980                 985                 990
   Pro Tyr Ala Gly Gly Tyr Gly Arg  Ser Tyr Gln Pro Ala  Pro Tyr Gly
           995                 1000                 1005
   Gly Gly  Gln Gln Trp Gln Gln  Gln Gln Gln Gln Pro  Tyr Gly Ser
       1010                 1015                 1020
   Tyr Ala  Gly Ser Gly Pro Tyr  Gly Gly Gly Ala Pro  Pro Ala Arg
       1025                 1030                 1035
   Pro Asn  Ser Arg Pro Gln Gln  Ser Gln Asn Arg Tyr  Asn Thr Leu
       1040                 1045                 1050
   Asp Arg  Asn Ser Asn Arg Arg  Pro Pro Pro Gly His  Gly Arg His
       1055                 1060                 1065
<210> 8
   <211> 7877
   <212> DNA
   <213> Oryza sativa japonica
<400> 8
   atataccccc ctcccctccc cgcccgcgtg gcctaccccc ttcgttaggg tttcgcgccc        60
   tcttccctct ctcccttccc ctcggccgtc ggcccggctc ctcgcggcgg cggcggcggc       120
   ggctcgcttt ctagggttag ggttagggtt ccggcggcgg aggaggagga gatgggtgtg     180
   ccggcgttct accggtggct ggcggagaag tacccgatgg tggtggtgga cgtggtggag     240
   gaggaggcgg tggagatcga gggcgtcaag gtgcccgtcg acacctccaa gcccaacccc     300
   aacggcctcg agttcgacaa cctctacctc gacatgaacg ggatcattca cccctgcttc     360
   caccccgagg atagggtacg tcgatgtttt ttttttgggc gtttctgttt gcgtagctct     420
   ggggtttgtt tgggtgttcg tatggtgtcc ctccgctgta caggtgatgg atttgtgtat     480
   gcctgattga tgtacgacgg agctgtggca gaggagatag tttggtgaaa tgattttgta     540
   gcttcctgtt ttgtaattta cctgagcgtt aaacataatt ttgcaaataa ccacaaactg     600
   ctaaattgcc acatgagcca tcttaggatg aggatgctgc tgtagtttaa tcttcaaact     660
   ctgcatcgca aatttgttaa tttgcctttt tgagttatgt ttattttcat tcggtggacg     720
   caccagttta gactgcatga tgctactctg tcattgccct gcatttctct tcgaacaaat     780
   attttaatgt ttactgattg ctcaagtgct ttctgatttc tctgctgacc atgatttctt     840
   gctattgtgt tatcttttac agccctctcc gacaacattc gctgaggttt tccagtgcat     900
   gtttgattat atcgataggc tatttgtcat ggttcgccct cggaagctta tgtatatggc     960
   cattggtacg tataacatct gatgtgtagg aattctctta ctaccttttt ccttcctaaa    1020
   ggattaggag tgtttagtgt cattgctctt ttacctgttg gtcatcatca tagaacagct    1080
   tgccatactg ccaactaatg cctttgttga agttagctat atactatgac gagtcattta    1140
   catgagtttt gaatcaggca taatcctcac ccgtaaacta tttatttggc agcatagtac    1200
   ctgtgcttgt aaacttttgc tgtccagggt cctgtttttt accacatatg ttttgttctt    1260
   gtcgatggtt tcttgttgat attttgtatt atcttacaat ggttttacag atggtgttgc    1320
   tcctcgtgcc aagatgaacc agcaacgctc tagacgattc agagctgcaa aagatgctgc    1380
   tgatgcggtc agtattctag tgaaattatc ttcctttatg cagtgtatga taatatgtcg    1440
   acatttaaat tcccattcat cgacggcttc aaggggtaga attgcattta tggcttatta    1500
   tttttctatc tccaggccgc agaagaagag aggttgcgtg aagagtttga gagggaaggc    1560
   agaaaacttc caccaaaaca gcaatctcaa acatgtgatt ctaacgttat tactccagga    1620
   acagagttta tggctgtttt atcgattgct ttgcagtact acatccatct caggttgaat    1680
   tatgatcctg gatggaaaca agttaaagta agcatggttt taagcctatc agttttgttt    1740
   tggcattaca ttctgtcagt atgttccata tgttaacttc acaaactcta aggtttacat    1800
   tgattggaaa ggcaagtatg aaaaagtcat gtgtgttctg gttaaattct tttgaaggtt    1860
   attctttctg atgccaatgt tcctggtgaa ggggaacata aaattatgtc ctacattcgc    1920
   ggtcaacgga atctccctgg atttaaccca aatactcgcc attgtttgta tggcctggta    1980
   tgttctataa acctattttt tatatatttt aattgggcaa tgtttcttct gttggtcctg    2040
   ctaatgcatt tttttatata tactcttgaa tgcaggatgc agacctgatc atgttagctc 2100
   ttgcaacaca cgaagtacat ttctctatac tgagagaggt tagctctcct caggacctag 2160
   aatcaatgcc tttttgtgta aatacatttt accatgttat gctaactaaa agagcggatt 2220
   agttccttat gctttcttgt ttatgttatt tgttttctct ctcttctgac tatattttgg 2280
   tttcttgcag gtagtttata cacctggaca gcaggacaaa tgcttcttgt gtggtcaggt 2340
   tggacatcta gcagcaaact gtgaaggaaa ggttaagaga aaagctgggg agtttgatga 2400
   aaaaggtgaa gctattgttc caaagaaacc ttaccaggta atattgtctc tttcctttgt 2460
   tgcaatttgt ggtctaataa gtacacttta tgaatttgct gaggaccttg ttttatttta 2520
   attgctcctt gtgtgacatt gcagttcttg aatatatgga ccctccggga gtacttagaa 2580
   tatgagttca gaatgcagaa tccacctttc ccgattgact ttgaacgcat cgttgatgat 2640
   ttcatcttca tgtgtttttt tgttggcaat gattttcttc cacatatgcc aacattagag 2700
   attcgtgagg tttgttacct gttgtaaagc taaatgtttt actggatatc ttcgtttgaa 2760
   gctagtagct atgaaattat cagattacta cttactagta aattggatat ttattttatt 2820
   cctgcttaca gttctcttgt agatttgtta ttgcatgatg ttaacttggc ttttctgcag 2880
   ggagctatta acttgctaat ggctgtatac aagaaggaat ttccttccat gggtggctat 2940
   ctaactgatg cttgcacggt aattaaaaag ccactcctat tgaagttacc catttgtgtg 3000
   tgtgtgtgtg tgtgtgtgtt tgtttgcaga aagtagtatt ttattccaac tgatccaagt 3060
   tggtatttca gtattaacat gtttttcatc tgttacatta gccggatctg aataaagttg 3120
   aacactttat tcaagcagtt ggctcctacg aggataaaat atttcagaaa agagctcgtt 3180
   tgcatcaggt gattttattt tattttactc cttagccttt tcaattccct tgttatcatg 3240
   ttatcatctg gcacctaatc tttattttta ctccatgcca ctatgcgcca aggggttcac 3300
   cattagctat cagttgaaat ttctgtttgt atttggcaat gtaatttcct tatttgcaaa 3360
   gttcattgag tgtttatttt gatatactgg taatttcagc ctgatgtact gcgttatatc 3420
   ttctatgaca tgttgtctat aaacttcctt caaattggag gtagtgattg tgaatttgcc 3480
   acctgccacc aatagtccaa cacactgcat ttcttactaa ttgctccgtg tattgtttta 3540
   atattacacc gtatatcttc tctatgctgc tttcttctta gctagccttg taaattcaca 3600
   atataacatg gaaaggttca caatgaccag ataatccttg acatgtaggt tagtgttttt 3660
   ttttttacaa tacctctaaa gaagttaatt ctacaattag catgtgtgaa cactgccaat 3720
   ccttgaaaag aagtaaatgc tatgctccgt agaaatcaca ttatgttttg aacaatattg 3780
   agacattttt gattcccatt tagctagtaa aatcagaaaa accagttgat tgttcaagta 3840
   tcttctaaac tagttatttg ttcaaggttt tctcccccat actttcccct agaatttaaa 3900
   tgttacccat ggaaaacttc tgcatgcagc gtaccacgtc cgtatggctt aggcatgttc 3960
   catcattatt tatccttagt tatgcaaagc cttctgatat gatcaacatg aactcttgag 4020
   aaataaatgt ttgcaacact attctatata tctggcggtc aaatattcaa caagtcattg 4080
   atgttaagtt catatccttg tgtagcgtca agctgaaagg ataaaacgag aaaaggccca 4140
   agcaaaacga ggggatgacc tggatcccca tgtcagggat gatcttattg tacctgttgc 4200
   acgcttccaa gggtctcgcc ttgcatctgg acctgtgcct tcaccatatg aacaaaatgg 4260
   atctgacaaa aacaatgggg gaaaaaatag tcgtgctcgg aaagctgcac gggtatcatc 4320
   gtcaggttca agcattgcgg ctgctattgt tgaagctgaa aatgaccttg aagcacaggt 4380
   agctcatttc atctggctcc aggagaattt atatttgttt ttgaagaaac atttttgtga 4440
   ttttatctga cacttgttcc tgtatctttc ttaggaacgc gaaaacaagg aagatttgaa 4500
   gacaatgctt aaagatgcac ttcgtgagaa atctgatgtt tttaattctg aaaatccaga 4560
   ggaggacaag gttagtgttt acacacaagt catgccactg ctgtgctgga ccttattata 4620
   cgtttaatta aagtgtttac tttcctttta ttttaactca tttactttct attagctatt 4680
   tgtttaaatg ttttcaattt agtcataaca ttgtttgtct tctcagataa aacttggaga 4740
   accaggatgg agggaaaggt actatgaaga gaagtttgga gcaagaacac ccgggcagat 4800
   tgaagaaata cgcagagatg tcgtaagtgt cttgaatacc tttgaattac tgcattgcct 4860
   gataatggtg tcatgatgat ctattgctgt taatatatag ttgtacaaac ttccataaaa 4920
   gatgccattt catcgctatt tgagcatgta ctcatttatt atcttatatg tatgatgtgc 4980
   cctattcatc ttcttcaggt tctgaaatat actgaaggtt tgtgttgggt aatgcactac 5040
   tattatgaag gtgtctgctc gtggcaatgg taatttagct tgagttggtc aatataggtt 5100
   gaattgtgta aaggtttatt ttcaactctt tgtcaatggc cttaaattgt tatctgtcta 5160
   cctcaccatg cacaggtttt acccttacca ttatgctcct tttgcttcgg atttgagtgg 5220
   tctaggtcaa ctcaatataa cttttgaact tgggtcacca ttcaaaccgt ttgatcaact 5280
   tatgggagtt ttcccagctg ctaggtgaat acactaagat aatgttccct agatcccagt 5340
   tccttaatgt tgtggatttt ttgtaaaggt tgtggcatta catttcagct cccatgcact 5400
   ccctgtacag tatcggcaat tgatgacaga tgcaaattca ccaataattg atttttatcc 5460
   aactggtatg cttatatttg tgatgtttga tatatttttt attacctttc cactttataa 5520
   ttttacactg ttttcaccca tttgtgatgc agattttgaa gtagatatga atgggaagag 5580
   gtattcatgg caggtaaatt gattataata gggcatatat ttatgtaatg taagtatacc 5640
   ttcatgttta atctatatac ttgaatttgg caggggatag caaaactgcc cttcattgat 5700
   gaagcccggt tgctagctga aattaaaaaa gttgagcata ctttgacggt atggcaacaa 5760
   aacagattgt cattcagttg tgcttttata atccatggaa taggagatct tgttctaaga 5820
   tatgcatcgc ttttcttacc ttctgtacag cctgaagaag caaggagaaa cagtatcatg 5880
   ttcaatatgc tttttgtgaa tggctcccat ccactttcgc cttatatcta ctccctcaac 5940
   agcaaatttg gacacctacc tgacagagag cgaaatgaaa ttaaagaaaa gattgatcct 6000
   tcttctaggt tagctaaccc tattaggatc cttgatttac tgcaaatgct atttttatta 6060
   gtttaaaagc atatggcatt tgcactgacc agtgttgtag attctgtaat gccttctgtg 6120
   tcaggcaaaa gttctggcta gcataggcca ctgaattgtt tttttcttct tattatttcc 6180
   ttaacgcagc tgccttgttt tgtgcagtgg aggaatgaat ggttacatct cactttgtag 6240
   tggggatccg tgccctcctg tcttcaggtc tcctgttgat gggttggagg atatcatgga 6300
   caatcaagtg atgtaagttc aatcccttct gggaaattaa gataatatga tttattggca 6360
   atctaattta ccatcatgtc atctgtctta attagatgta caatctacaa gcttccggat 6420
   tctcataagc acatcgctcg tccacctgtt ggtgtaatca tacccaagaa ggtacatctc 6480
   tatcattttt gtctagtatc aacactagta acatctttcc acttgagaaa tggtaataac 6540
   aaataagtaa actctgcaga ctgttgaagc tactgacctg aaaccaccac ctgtgctatg 6600
   gcatgaagac agtggtagga ggcctcatga taataacaac aggaggcctt atgaaaacag 6660
   caacaggtac actatctacc ttttgtgtta ttttgtcaat ccttgcaacg tcattgaaaa 6720
   tcctaacctt ctggtgaaat tgtttaggca aaacccagct ggagcaatat ctggccgcca 6780
   acttggggaa gctgcccacc gacttgtggt gaacagcttg aatgctcgga gtggcggaca 6840
   atacaatacc ccatcgatgc catatcaaac cataatgaat ggcatgcctt atccaaatgg 6900
   gattccacca aggatggagc agcctgctcc tggctggcat gttcctggtg atctaccaaa 6960
   tggtcaggta ccaccagctt atgcatcatc atcaggtcat tatcagaacg ataggtctgg 7020
   gccttctcag tatggacgag ataaccatgg aaggtaccca tatgcaagag acaaccacca 7080
   tgattcaaga ggaagggttc caccatacca tcagagcggt ggaaactcgt atcaatcaca 7140
   ttctgctcca tcagcaggtc ctggacggta tgcgcaaccc ccaccatatg ctggggggta 7200
   tggtaggagc taccagcctg caccgtatgg aggtggtcag cagtggcagc agcagcagca 7260
   acaaccttat ggctcctatg ctgggtcagg gccttatggg ggtggggcac ctcctgcaag 7320
   gcctaattca cgaccacagc agtcgcagaa ccgttacaat accttagata ggaattctaa 7380
   caggagacct ccaccaggtc atgggcgaca ctaagcccaa cctctgtttg ttgtacctgt 7440
   ggttttgttg ttagaaaacc tgcgtactaa aatagagaac ctcagcctag taaaacagag 7500
   aacctcagct tagagatata tatagagcaa tttgcatagt ctcgtgatcg acaagctgtt 7560
   atagcatcaa aggttctaag aggcatgtgt tgtacaatag aattgtcagt tcatcagata 7620
   ctggtggctg actgccgttt ttcatttgtg gcactcatta ttttagcccc ctaaggttgg 7680
   gaatatgttt aacaagtttt gtatttttaa gataataatg ttctaggtta aatttcagtt     7740
   ttggtggata tatgtcaaaa tcttgtattc ttttcttatt tataatggaa ttgtggtttt     7800
   ccctctaacc tgtcgtggtt gtcaattgag aacaagtggt tattttttgc tggcttataa     7860
   aatttttctg ttaatat                                                    7877
<210> 9
   <211> 1068
   <212> PRT
   <213> Oryza sativa indica
<400> 9
   Met Gly Val Pro Ala Phe Tyr Arg Trp Leu Ala Glu Lys Tyr Pro Met
   1               5                   10                  15
   Val Val Val Asp Val Val Glu Glu Glu Ala Val Glu Ile Glu Gly Val
               20                  25                  30
   Lys Val Pro Val Asp Thr Ser Lys Pro Asn Pro Asn Gly Leu Glu Phe
           35                  40                  45
   Asp Asn Leu Tyr Leu Asp Met Asn Gly Ile Ile His Pro Cys Phe His
       50                  55                  60
   Pro Glu Asp Arg Pro Ser Pro Thr Thr Phe Ala Glu Val Phe Gln Cys
   65                  70                  75                  80
   Met Phe Asp Tyr Ile Asp Arg Leu Phe Val Met Val Arg Pro Arg Lys
                   85                  90                  95
   Leu Met Tyr Met Ala Ile Asp Gly Val Ala Pro Arg Ala Lys Met Asn
               100                 105                 110
   Gln Gln Arg Ser Arg Arg Phe Arg Ala Ala Lys Asp Ala Ala Asp Ala
           115                 120                 125
   Ala Ala Glu Glu Glu Arg Leu Arg Glu Glu Phe Glu Arg Glu Gly Arg
       130                 135                 140
   Lys Leu Pro Pro Lys Gln Gln Ser Gln Thr Cys Asp Ser Asn Val Ile
   145                 150                 155                 160
   Thr Pro Gly Thr Glu Phe Met Ala Val Leu Ser Ile Ala Leu Gln Tyr
                   165                 170                 175
   Tyr Ile His Leu Arg Leu Asn Tyr Asp Pro Gly Trp Lys Gln Val Lys
               180                 185                 190
   Val Ile Leu Ser Asp Ala Asn Val Pro Gly Glu Gly Glu His Lys Ile
           195                 200                 205
   Met Ser Tyr Ile Arg Gly Gln Arg Asn Leu Pro Gly Phe Asn Pro Asn
       210                 215                 220
   Thr Arg His Cys Leu Tyr Gly Leu Asp Ala Asp Leu Ile Met Leu Ala
   225                 230                 235                 240
   Leu Ala Thr His Glu Val His Phe Ser Ile Leu Arg Glu Val Val Tyr
                   245                 250                 255
   Thr Pro Gly Gln Gln Asp Lys Cys Phe Leu Cys Gly Gln Val Gly His
               260                 265                 270
   Leu Ala Ala Asn Cys Glu Gly Lys Val Lys Arg Lys Ala Gly Glu Phe
           275                 280                 285
   Asp Glu Lys Gly Glu Ala Ile Val Pro Lys Lys Pro Tyr Gln Phe Leu
       290                 295                 300
   Asn Ile Trp Thr Leu Arg Glu Tyr Leu Glu Tyr Glu Phe Arg Met Gln
   305                 310                 315                 320
   Asn Pro Pro Phe Pro Ile Asp Phe Glu Arg Ile Val Asp Asp Phe Ile
                   325                 330                 335
   Phe Met Cys Phe Phe Val Gly Asn Asp Phe Leu Pro His Met Pro Thr
               340                 345                 350
   Leu Glu Ile Arg Glu Gly Ala Ile Asn Leu Leu Met Ala Val Tyr Lys
           355                 360                 365
   Lys Glu Phe Pro Ser Met Gly Gly Tyr Leu Thr Asp Ala Cys Thr Pro
       370                 375                 380
   Asp Leu Asn Lys Val Glu His Phe Ile Gln Ala Val Gly Ser Tyr Glu
   385                 390                 395                 400
   Asp Lys Ile Phe Gln Lys Arg Ala Arg Leu His Gln Arg Gln Ala Glu
                   405                 410                 415
   Arg Ile Lys Arg Glu Lys Ala Gln Ala Lys Arg Gly Asp Asp Leu Asp
               420                 425                 430
   Pro His Val Arg Asp Asp Leu Ile Val Pro Val Ala Arg Phe Gln Gly
           435                 440                 445
   Ser Arg Leu Ala Ser Gly Pro Ala Pro Ser Pro Tyr Glu Gln Asn Gly
       450                 455                 460
   Ser Asp Lys Asn Asn Gly Gly Lys Asn Ser Arg Ala Arg Lys Ala Ala
   465                 470                 475                 480
   Arg Val Ser Ser Ser Gly Ser Ser Ile Ala Ala Ala Ile Val Glu Ala
                   485                 490                 495
   Glu Asn Asp Leu Glu Ala Gln Glu Arg Glu Asn Lys Glu Asp Leu Lys
               500                 505                 510
   Thr Met Leu Lys Asp Ala Leu Arg Glu Lys Ser Asp Val Phe Asn Ser
           515                 520                 525
   Glu Asn Pro Glu Glu Asp Lys Ile Lys Leu Gly Glu Pro Gly Trp Arg
       530                 535                 540
   Glu Arg Tyr Tyr Glu Glu Lys Phe Gly Ala Arg Thr Pro Gly Gln Ile
   545                 550                 555                 560
   Glu Glu Ile Arg Arg Asp Val Val Leu Lys Tyr Thr Glu Gly Leu Cys
                   565                 570                 575
   Trp Val Met His Tyr Tyr Tyr Glu Gly Val Cys Ser Trp Gln Trp Phe
               580                 585                 590
   Tyr Pro Tyr His Tyr Ala Pro Phe Ala Ser Asp Leu Ser Gly Leu Gly
           595                 600                 605
   Gln Leu Asn Ile Thr Phe Glu Leu Gly Ser Pro Phe Lys Pro Phe Asp
       610                 615                 620
   Gln Leu Met Gly Val Phe Pro Ala Ala Ser Ser His Ala Leu Pro Val
   625                 630                 635                 640
   Gln Tyr Arg Gln Leu Met Thr Asp Ala Asn Ser Pro Ile Ile Asp Phe
                   645                 650                 655
   Tyr Pro Thr Asp Phe Glu Val Asp Met Asn Gly Lys Arg Tyr Ser Trp
               660                 665                 670
   Gln Gly Ile Ala Lys Leu Pro Phe Ile Asp Glu Ala Arg Leu Leu Ala
           675                 680                 685
   Glu Ile Lys Lys Val Glu His Thr Leu Thr Pro Glu Glu Ala Arg Arg
   690 695 700
   Asn Ser Ile Met Phe Asn Met Leu Phe Val Asn Gly Ser His Pro Leu
   705 710 715 720
   Ser Pro Tyr Ile Tyr Ser Leu Asn Ser Lys Phe Gly His Leu Pro Asp
   725 730 735
   Arg Glu Arg Asn Glu Ile Lys Glu Lys Ile Asp Pro Ser Ser Ser Gly
   740 745 750
   Gly Met Asn Gly Tyr Ile Ser Leu Cys Ser Gly Asp Pro Cys Pro Pro
   755 760 765
   Val Phe Arg Ser Pro Val Asp Gly Leu Glu Asp Ile Met Asp Asn Gln
   770 775 780
   Val Ile Cys Thr Ile Tyr Lys Leu Pro Asp Ser His Lys His Ile Ala
   785 790 795 800
   Arg Pro Pro Val Gly Val Ile Ile Pro Lys Lys Thr Val Glu Ala Thr
   805 810 815
   Asp Leu Lys Pro Pro Pro Val Leu Trp His Glu Asp Ser Gly Arg Arg
   820 825 830
   Pro His Asp Asn Asn Asn Arg Arg Pro Tyr Glu Asn Ser Asn Arg Gln
   835 840 845
   Asn Pro Ala Gly Ala Ile Ser Gly Arg Gln Leu Gly Glu Ala Ala His
   850 855 860
   Arg Leu Val Val Asn Ser Leu Asn Ala Arg Ser Gly Gly Gln Tyr Asn
   865 870 875 880
   Thr Pro Ser Met Pro Tyr Gln Thr Ile Met Asn Gly Met Pro Tyr Pro
   885 890 895
   Asn Gly Ile Pro Pro Arg Met Glu Gln Pro Ala Pro Gly Trp His Val
   900 905 910
   Pro Gly Asp Leu Pro Asn Gly Gln Val Pro Pro Ala Tyr Ala Ser Ser
   915 920 925
   Ser Gly His Tyr Gln Lys Asp Arg Ser Gly Pro Ser Gln Tyr Gly Arg
   930 935 940
   Asp Asn His Gly Arg Tyr Pro Tyr Ala Arg Asp Asn His His Asp Ser
   945                 950                 955                 960
   Arg Gly Arg Val Pro Pro Tyr His Gln Ser Gly Gly Asn Ser Tyr Gln
                   965                 970                 975
   Ser His Ser Ala Pro Ser Ala Gly Pro Gly Gln Tyr Ala Gln Pro Pro
               980                 985                 990
   Pro Tyr Ala Gly Gly Tyr Gly Arg  Ser Tyr Gln Pro Ala  Pro Tyr Gly
           995                 1000                 1005
   Gly Gly  Gln Gln Trp Gln Gln  Gln Gln Gln Gln Pro  Tyr Gly Ser
       1010                 1015                 1020
   Tyr Ala  Gly Ser Gly Pro Tyr  Gly Gly Gly Ala Pro  Pro Ala Arg
       1025                 1030                 1035
   Pro Asn  Ser Arg Pro Gln Gln  Ser Gln Asn Arg Tyr  Asn Thr Leu
       1040                 1045                 1050
   Asp Arg  Asn Ser Asn Arg Arg  Pro Pro Pro Gly His  Gly Arg His
       1055                 1060                 1065
<210> 10
   <211> 7239
   <212> DNA
   <213> Oryza sativa indica
<400> 10
   atgggtgtgc cggcgttcta ccggtggctg gcggagaagt acccgatggt ggtggtggac 60
   gtggtggagg aggaggcggt ggagatcgag ggcgtcaagg tgcccgtcga cacctccaag 120
   cccaacccca acggcctcga gttcgacaac ctctacctcg acatgaacgg gatcattcac 180
   ccctgcttcc accccgagga tagggtacgt cgatgttttt tttttgggcg tttctgtttg 240
   cgttgctctg gggtttgttt gggtgttcgt atggtgtccc tccgctgtac aggtgatgga 300
   tttgtgtatg cctgattgat gtacgacgga gctgtggcag aggagatagt ttggtgaaat 360
   gattttgtag cttcctgttt tgtaatttac ctgagcgtta aacataattt tgcaaataac 420
   cacaaactgc taaattgcca catgagccat cttaggatga ggatgctgct gtagtttaat 480
   cttcaaactc tgcatcgcaa atttgttaat ttgccttttt gagttatgtt tattttcatt 540
   cggtggacgc accagtttag actgcatgat gctactctgt cattgccctg catttctctt 600
   cgaacaaata ttttaatgtt tactgattgc tcaagtgctt tctgatttct ctgctgacca 660
   tgatttcttg ctattgtgtt atcttttaca gccctctccg acaacattcg ctgaggtttt 720
   ccagtgcatg tttgattata tcgataggct atttgtcatg gttcgccctc ggaagcttat 780
   gtatatggcc attggtacgt ataacatctg atgtgtagga attctcttac tacctttttc       840
   cttcctaaag gattaggagt gtttagtgtc attgctcttt tacctgttgg tcatcatcat      900
   agaacagctt gccatactgc caactaatgc ctttgttgaa gttagctata tactatgacg      960
   agtcatttac atgagttttg aatcaggcat aatcctcacc cgtaaactat ttatttggca     1020
   gcatagtacc tgtgcttgta aacttttgct gtccagggtc ctgtttttta ccacatatgt     1080
   tttgttcttg tcgatggttt cttgttgata ttttgtatta tcttacaatg gttttacaga     1140
   tggtgttgct cctcgtgcca agatgaacca gcaacgctct agacgattca gagctgcaaa     1200
   agatgctgct gatgcggtca gtattctagt gaaattatct tcctttatgc agtgtatgat     1260
   aatatgtcaa catttaaatt cccattcatc gacggcttca aggggtagaa ttgcatttat     1320
   ggcttattat ttttctatct ccaggccgca gaagaagaga ggttgcgtga agagtttgag     1380
   agggaaggca gaaaacttcc accaaaacag caatctcaaa catgtgattc taacgttatt     1440
   actccaggaa cagagtttat ggctgtttta tcgattgctt tgcagtacta catccatctc     1500
   aggttgaatt atgatcctgg atggaaacaa gttaaagtaa gcatggtttt aagcctatca     1560
   gttttgtttt ggcattacat tctgtcagta tgttccatat gttaacttca caaactctaa     1620
   ggtttacatt gattggaaag gcaagtatga aaaagtcatg tgtattctgg ttaaattctt     1680
   ttgaaggtta ttctttctga tgccaatgtt cctggtgaag gggaacataa aattatgtcc     1740
   tacattcgcg gtcaacggaa tctccctgga tttaacccaa atactcgcca ttgtttgtat     1800
   ggcctggtat gttctataaa cctatttttt atatatttta attgggcaat gtttcttctg     1860
   ttggtcctgc taatgcattt ttttatatat actcttgaat gcaggatgca gacctgatca     1920
   tgttagctct tgcaacacac gaagtacatt tctctatact gagagaggtt agctctcctc     1980
   aggacctaga atcaatgcct ttttgtgtaa atacatttta ccatgttatg ctaactaaaa     2040
   gagcggatta gttccttatg ctttcttgtt tatgttattt gttttctctc tcttctgact     2100
   atattttggt ttcttgcagg tagtttatac acctggacag caggacaaat gcttcttgtg     2160
   tggtcaggtt ggacatctag cagcaaactg tgaaggaaag gttaagagaa aagctgggga     2220
   gtttgatgaa aaaggtgaag ctattgttcc aaagaaacct taccaggtaa tattgtctct     2280
   ttcctttgtt gcaatttgtg gtctaataag tacactttat gaatttgctg aggaccttgt     2340
   tttattttaa ttgctccttg tgtgacattg cagttcttga atatatggac cctccgggag     2400
   tacttagaat atgagttcag aatgcagaat ccacctttcc cgattgactt tgaacgcatc     2460
   gttgatgatt tcatcttcat gtgttttttt gttggcaatg attttcttcc acatatgcca     2520
   acattagaga ttcgtgaggt ttgttacctg ttgtaaagct aaatgtttta ctggatatct     2580
   tcgtttgaag ctagtagcta tgaaattatc agattactac ttactagtaa attggatatt     2640
   tattttattc ctgcttacag ttctcttgta gatttgttat tgcatgatgt taacttggct 2700
   tttctgcagg gagctattaa cttgctaatg gctgtataca agaaggaatt tccttccatg 2760
   ggtggctatc taactgatgc ttgcacggta attaaaaagc cactcctatt gaagttaccc 2820
   atttgtgtgt gtgtgtgtgt gtgtgtgtgt gtttgtttgc agaaagtagt attttattcc 2880
   agctgatcca agttggtatt tcagtattaa catgtttttc atctgttaca ttagccggat 2940
   ctgaataaag ttgaacactt tattcaagca gttggctcct acgaggataa aatattccag 3000
   aaaagagctc gtttgcatca ggtgatttta ttttatttta ctccttagcc ttttcaattc 3060
   ccttgttatc atgttatcat ctggcaccta atctttattt ttactccatg ccactatgcg 3120
   ccaaggggtt caccattagc tatcagttga aatttctgtt tgtatttggc aatgtaattt 3180
   ccttatttgc aaagttcatt gagtgtttat tttgatatac tggtaatttc agcctgatgt 3240
   actgcgttat atcttctagg acatgttgtc tataaacttc cttcaaattg gaggtagtga 3300
   ttgtgaattt gccacctgcc accaatagtc caacacactg catttcttac taattgctcc 3360
   gtgtattgtt ttaatattac accgtatatc ttctctatgc tgctttcttc ttagctagcc 3420
   ttgtaaattc acaatataac atggaaaggt tcacaatgac cagataatcc ttgacatgta 3480
   ggttagtgtt tttttttttt acaatacctc taaagaagtt aattctacaa ttagcatgtg 3540
   tgaacactgc caatccttga aaagaagtaa atgctatgct ccgtagaaat cacattatgt 3600
   tttgaacaat attgagacat tttcgattcc catttagcta gtaaaatcag aaaaaccagt 3660
   tgattgttca agtatcttct aaactagtta tttgttcaag gtttcctccc ccatactttc 3720
   ccctagaatt taaatgttac ccatggaaaa cttctgcatg cagcgtacca cgtctgtatg 3780
   gcttaggcat gttccatcat tatttatcct tagttatgca aagccttctg atatgatcaa 3840
   catgaactcc tgagaaataa atgtttgcaa cactattcta tatatctggc ggtcaaatat 3900
   tcaacaagtc attgatgtta agttcatatc cttgtgtagc gtcaagctga aaggataaaa 3960
   cgagaaaagg cccaagcaaa acgaggggat gacctggatc cccatgtcag ggatgatctt 4020
   attgtacctg ttgcacgctt ccaagggtct cgccttgcat ctggacctgc gccttcacca 4080
   tatgaacaaa atggatctga caaaaacaat gggggaaaaa atagtcgtgc tcggaaagct 4140
   gcacgggtat catcgtcagg ttcaagcatt gcggctgcta ttgttgaagc tgaaaatgac 4200
   cttgaagcac aggtagctca tttcatctgg ctccaggaga atttatattt gtttttgatg 4260
   aaacattttt gtgattttat ctgacacttg ttcctgtatc tttcttagga acgcgaaaac 4320
   aaggaagatt tgaagacaat gcttaaagat gcacttcgtg agaaatctga tgtttttaat 4380
   tctgaaaatc cagaggagga caaggttagt gtttacacac aagtcatgcc actgctgtgc 4440
   tggaccttat tatacgttta attaaagtgt ttactttcct tttattttaa ctcatttact 4500
   ttctattagc tatttgttta aatgttttca atttagtcat aacattgttt gtcttctcag 4560
   ataaaacttg gagaaccagg atggagggaa aggtactatg aagagaagtt tggagcaaga 4620
   acacccgggc agattgaaga aatacgcaga gatgtcgtaa gtgtcttgaa tacctttgaa 4680
   ttactgcatt gcctgataat ggtgtcatga tgatctattg ctgttaatat atagttgtac 4740
   gaacttccat aaaagatgcc atttcatcgc tatttgagca tgtactcatt tattatctta 4800
   tatgtatgat gtgccctatt catcttcttc aggttctgaa atatactgaa ggtttgtgtt 4860
   gggtaatgca ctactattat gaaggtgtct gctcgtggca atggtaattt agcttgagtt 4920
   ggtcaatata ggttgaattg tgtaaaggtt tattttcaac tctttgtcaa tggccttaaa 4980
   ttgttatctg tctacctcac catgcacagg ttttaccctt accattatgc tccttttgct 5040
   tcggatttga gtggtctagg tcaactcaat ataacttttg aacttgggtc accattcaaa 5100
   ccgtttgatc aacttatggg agttttccca gctgcgaggt gaatacacta agataatgtt 5160
   ccctagatcc cagttcctta atgttgtgga ttttttgtaa aggttgtggc attacatttc 5220
   agctcccatg cactccctgt acagtatcgg caattgatga cagatgcaaa ttcaccaata 5280
   attgattttt atccaactgg tatgcttata tttgtgatgt ttgatatatt ttttattacc 5340
   tttccacttt ataattttac actgttttca cccatttgtg atgcagattt tgaagtagat 5400
   atgaatggga agaggtattc atggcaggta aattgattat aatagggcat atatttatgt 5460
   aatgtaagta taccttcatg tttaatctat atatttgaat ttggcagggg atagcaaaac 5520
   tgcccttcat tgatgaagcc cggttgctag ctgaaattaa aaaagttgag catactttga 5580
   cggtatggca acaaaacaga ttgtcattca gttgtgcttt tataatccat ggaataggag 5640
   atcttgttct aagatatgca tcgcttttct taccttctgt acagcctgaa gaagcaagga 5700
   gaaacagtat catgttcaat atgctttttg tgaatggctc ccatccactt tcgccttata 5760
   tctactccct caacagcaaa tttggacacc tacctgacag agagcgaaat gaaattaaag 5820
   aaaagattga tccttcttct aggttagcta accctattag gatccttgat ttactgcaaa 5880
   tgctattttt attagtttaa aagcatatgg tatttgcact gaccagtgtt gtagattctg 5940
   taatgccttc tgtgtcaggc aaaagttctg gctagcatag gccactgaat tgtttttttc 6000
   ttattatttc cttaacgcag ctgccttgtt ttgtgcagtg gaggaatgaa tggttacatc 6060
   tcactttgta gtggggatcc gtgccctcct gtcttcaggt ctcctgttga tgggttggag 6120
   gatatcatgg acaatcaagt gatgtaagtt caatcccttc tgggaaatta agataatatg 6180
   atttattggc aatctaattt accatcatgt catctgtctt aattagatgt acaatctaca 6240
   agcttccgga ttctcataag cacatcgctc gtccacctgt tggtgtaatc atacccaaga 6300
   aggtacatca tttttgtcta gtatgaacac tagtaacatc tttccacttg agaaatggta 6360
   ataacaaata agtaaactct gcagactgtt gaagctactg acctgaaacc accacctgtg 6420
   ctatggcatg aagacagtgg taggaggcct catgataata acaacaggag gccttatgaa 6480
   aacagcaaca ggtacactat ctaccttttg tgttattttg tcaatccttg caacgtcatt 6540
   gaaaatccta accttctggt gaaattgttt aggcaaaacc cagctggagc aatatctggc 6600
   cgccaacttg gggaagctgc ccaccgactt gtggtgaaca gcttgaatgc tcggagtggc 6660
   ggacaataca ataccccatc aatgccatat caaaccataa tgaatggcat gccttatcca 6720
   aatgggattc caccaaggat ggagcagcct gctcctggct ggcatgttcc tggtgatcta 6780
   ccaaatggtc aggtaccacc agcttatgca tcatcatcag gtcattatca gaaagatagg 6840
   tctgggcctt ctcagtatgg acgagataac catggaaggt acccatatgc aagagacaac 6900
   caccatgatt caagaggaag ggttccacca taccatcaga gcggtggaaa ctcgtatcaa 6960
   tcacattctg ctccatcagc aggtcctgga cagtatgcgc aacccccacc atatgctggg 7020
   gggtatggta ggagctacca gcctgcaccg tatggaggtg gtcagcagtg gcagcagcag 7080
   cagcaacaac cttatggctc ctatgctggg tcagggcctt atgggggtgg ggcacctcct 7140
   gcaaggccta attcacgacc acagcagtcg cagaaccgtt acaatacctt agataggaat 7200
   tctaacagga gacctccacc aggtcatggg cgacactaa 7239
<210> 11
   <211> 965
   <212> PRT
   <213> Populus balsamifera subsp. trichocarpa
<400> 11
   Met Gly Val Pro Ala Phe Tyr Arg Leu Leu Ala Asp Arg Tyr Pro Leu
   1               5                   10                  15
   Ser Ile Ser Asp Val Ile Glu Glu Glu Pro Gln Glu Asp Ser Asn Gly
               20                  25                  30
   Asn Ser Lys Pro Ile Asp Val Ser Lys Pro Asn Pro Asn Gly Ile Glu
           35                  40                  45
   Phe Asp Asn Leu Tyr Leu Asp Met Asn Gly Ile Ile His Pro Cys Phe
       50                  55                  60
   His Pro Glu Gly Lys Pro Ala Pro Ala Thr Tyr Asp Asp Val Phe Lys
   65                  70                  75                  80
   Ser Ile Phe Ala Tyr Ile Asp His Leu Phe Ala Leu Val Arg Pro Arg
                   85                  90                  95
   Lys Leu Leu Phe Met Ala Ile Asp Gly Val Ala Pro Arg Ala Lys Met
               100                 105                 110
   Asn Gln Gln Arg Ser Arg Arg Phe Arg Ala Ala Lys Asp Ala Ala Gln
   115 120 125
   Ala Glu Ala Glu Glu Glu Arg Leu Arg Lys Glu Phe Glu Ala Glu Gly
   130 135 140
   Glu Leu Leu Ser Val Lys Glu Lys Pro Glu Thr Phe Asp Ser Asn Val
   145 150 155 160
   Ile Thr Pro Gly Thr Gln Phe Met Ala Ala Leu Ser Thr Ala Leu Gln
   165 170 175
   Tyr Tyr Ile Gln Ser Arg Leu Asn His Asn Leu Gly Trp Gln Asn Thr
   180 185 190
   Lys Val Ile Leu Ser Asp Ser Asn Val Pro Gly Glu Gly Glu His Lys
   195 200 205
   Ile Met Ser Tyr Ile Arg Leu Gln Arg Asn Leu Ser Gly Phe Asn Pro
   210 215 220
   Asn Thr Arg His Cys Leu Tyr Ser Leu Asp Ala Asp Leu Ile Met Leu
   225 230 235 240
   Ser Leu Ala Thr Arg Glu Val His Phe Ser Ile Leu Arg Glu Ile Val
   245 250 255
   Thr Leu Pro Gly Gln Gln Asp Lys Cys Phe Leu Cys Gly Gln Ala Gly
   260 265 270
   His Leu Ala Ala Glu Cys Arg Gly Lys Gln Gly Asp Asp Ala Leu Asp
   275 280 285
   Trp His Val Val Asp Asp Thr Pro Ile His Lys Lys Lys Tyr Gln Phe
   290 295 300
   Leu Asn Ile Trp Val Leu Arg Glu Tyr Leu Gln Tyr Asp Leu Asp Ile
   305 310 315 320
   Leu Asn Pro Pro Phe Ala Ile Asp Phe Glu Arg Ile Val Asp Asp Phe
   325 330 335
   Val Phe Leu Cys Phe Phe Val Gly Asn Asp Phe Leu Pro His Met Pro
   340 345 350
   Thr Leu Glu Ile Arg Glu Gly Ala Ile Ser Leu Leu Met His Ile Tyr
   355 360 365
   Arg Arg Glu Phe Ser Ala Met Gly Gly Tyr Leu Thr Leu Ala Gly Glu
       370                 375                 380
   Val Phe Leu Asp Lys Val Glu His Phe Ile Gln Cys Val Gly Val Tyr
   385                 390                 395                 400
   Glu Glu Gln Ile Phe Gln Lys Arg Thr Arg Ile Gln Gln Ala Phe Asp
                   405                 410                 415
   Asn Asn Glu Glu Met Lys Leu Lys Ala Arg Arg Glu Ser Ser Glu Val
               420                 425                 430
   Ile Gln Ala Pro Val Val Asp Lys Val Lys Leu Gly Glu Pro Gly Tyr
           435                 440                 445
   Lys Glu Arg Tyr Tyr Ala Glu Lys Phe Glu Leu Ser Asn Gln Glu Glu
       450                 455                 460
   Ile Asp Lys Val Arg Lys Glu Val Val Leu Lys Tyr Val Glu Gly Leu
   465                 470                 475                 480
   Cys Trp Val Cys His Tyr Tyr Phe Gln Gly Val Cys Ser Trp Gln Trp
                   485                 490                 495
   Phe Tyr Pro Phe His Tyr Ala Pro Phe Ala Ser Asp Leu Lys Asp Leu
               500                 505                 510
   Gly Glu Val Glu Leu Asn Phe Phe Ile Gly Glu Pro Phe Lys Pro Phe
           515                 520                 525
   Asp Gln Leu Met Gly Thr Leu Pro Ala Ala Ser Ser Asn Ala Leu Pro
       530                 535                 540
   Lys Glu Tyr Arg Lys Leu Met Thr Asn Pro Ser Ser Pro Ile His Arg
   545                 550                 555                 560
   Phe Phe Pro Ser Asp Phe Glu Ile Asp Met Asn Gly Lys Arg Phe Ala
                   565                 570                 575
   Trp Gln Gly Ile Ala Lys Leu Pro Phe Ile Asp Glu Arg Lys Leu Leu
               580                 585                 590
   Ala Gln Thr Lys Lys Leu Glu Ser Thr Leu Thr Glu Glu Glu Gln Ile
           595                 600                 605
   Arg Asn Arg Val Met Leu Asp Leu Leu Tyr Ile His Pro Val His Pro
   610 615 620
   Leu Ala Gln Leu Val Ile Ser Tyr Tyr Gln Gln Asn Asp His Leu Ser
   625 630 635 640
   Glu Gly Glu Arg Phe Ala Trp Glu Ile Asp Thr Arg Ala Ser Gly Gly
   645 650 655
   Met Asn Gly Cys Leu Trp Leu Tyr Glu Arg Asn Val Arg Arg Ser Val
   660 665 670
   Val Pro Ser Pro Ile Leu Gly Leu Pro Ala Leu Glu Gly Asn Gln Val
   675 680 685
   Leu Asn Ile Thr Phe Leu Asn Pro Lys Asn Arg Ala His Ile Pro Glu
   690 695 700
   Ile Pro Glu Gly Val Val Met Pro Glu Lys Ile Val Lys Pro Val Asp
   705 710 715 720
   Leu Lys Pro Phe Pro Thr Leu Trp His Glu Asp Asn Gly Arg Arg Gln
   725 730 735
   Gln Gly Arg Glu Arg Pro Gln Val Gln Arg Ala Ile Ala Gly Pro Phe
   740 745 750
   Leu Gly Asp Ala Ala His Arg Leu Val Lys Asn Thr Leu Asn Ile Lys
   755 760 765
   Pro Asn Gly Ser Ser Ser Arg Val Phe Asp Gln Gln Leu Tyr His Asn
   770 775 780
   Ile Pro Gly Asn Tyr Thr Phe Tyr Arg Pro Arg Pro Ala Gly Pro Ala
   785 790 795 800
   Gly Tyr Gly Arg Gly Tyr Trp Asp Asp Pro Asn Tyr His Tyr Ala Gln
   805 810 815
   His Ser Asn Gln Gln Gly Leu Met Ser Asn Pro Arg Tyr Arg Ser Leu
   820 825 830
   Ser Asn Gly Val Gln Ser Asn Arg His Asn Phe Arg Thr Gln Asp Gly
   835 840 845
   Val Gln Tyr His Gln Gln Tyr His Asn Leu Ser Thr Gly Val Ser Ala
   850 855 860
   Leu Thr Val Glu Glu Asn Ile Arg Ser Arg Ala Pro Ala Val Ile Ser
   865                 870                 875                 880
   Pro Arg Met Pro Asn Pro Gly Asn Thr Pro Asn Leu Gln Asn Gln Ala
                   885                 890                 895
   Glu Gln Asn Thr Gly Leu Leu Ser Ser Pro Pro Thr Asn Trp Ile Asn
               900                 905                 910
   Lys Thr Ala Ala Gly Asn Thr Gly Met Tyr Phe Lys Gln Lys Ser Thr
           915                 920                 925
   Ser Ile Gly Pro Asn Glu Lys Gln Val Lys Gln Val Tyr Gln Val Lys
       930                 935                 940
   Thr Gln Val Ala Gln Glu Thr Pro Asp Ile Gln Ala Gln Glu Thr Pro
   945                 950                 955                 960
   Asp Leu Lys Gln Gln
                   965
<210> 12
   <211> 10955
   <212> DNA
   <213> Populus balsamifera subsp. trichocarpa
<400> 12
   atgggtgtac cggcgttcta caggcttctc gcagaccgat acccgctatc aatttcggat      60
   gtgatcgaag aggaacctca agaggattcg aatggaaact ccaaaccaat cgatgtttca     120
   aagcctaacc ctaacggtat tgagtttgac aatctgtatt tggatatgaa cgggattatc     180
   catccctgct tccatcctga aggcaaggta cagtttacca acttatactt gtatagtttt     240
   ctggaatctt agcttcttag ggtttaaaag gaggtttttt gtatttgtaa ttgaattgct     300
   gttaaatttt gatgcaaatg caattgactg tcgttaaatt ttgatggttt ttgctcgctg     360
   caaagctata cgtatacaga ttgtaaagtt ttagctaggg tttaaaagag gggttttttt     420
   gtgtgttgtt ggatgtagtt gttgattttg atggatagtg tttaaaatga acattcattt     480
   ataattttac acatgttata gggttttgtt aaggtttaaa agattgaaag tttttgtgtg     540
   ttatttaaat gctgtccatt tttcatggtt agtattgaat ggaagcatat atttacgcaa     600
   gttgtagaat tttatattgt aagatttaaa agaggaaatc tttgattttg tttgtgtgaa     660
   ttaattgttg tcaattttga tggtttgggc ttaaaagagg tgtttttttt ttatatatag     720
   tttctgtgtg taattgaata agttgaatgg tttcttactt ttgattttat ttacagcctg     780
   cacctgctac ttatgatgat gtgttcaagt ccatatttgc ttacattgat catttgtttg     840
   ctttggttcg tccaaggaaa ctacttttta tggcaattgg tagggttctt gttttattga     900
   atttgttttt ttaatcggtt ttggtatttg ttgaatttat aatcatttaa agttcattgc 960
   ttttttcctg ggataattgt agatggggtt gctcctagag ctaaaatgaa ccagcaacgg 1020
   tcgcgacggt ttagagctgc aaaagatgct gctcaagcag taagattgag ataatttaat 1080
   ggttttttta gtggatcctc ttttgtagaa gcgattcatt ttatttatga tgtatgtagg 1140
   aagctgaaga agaaagattg aggaaagaat ttgaggctga gggggagctt ttatccgtta 1200
   aagaaaagcc tgaaacattt gattctaatg ttatcactcc tggcactcaa tttatggctg 1260
   ctctttcgac tgcgcttcag tattatatac aaagtagatt gaatcacaat ctgggctggc 1320
   agaataccaa ggtgtgatgg atgttctcct ttcacttagt ttgttataga tcggatcatt 1380
   ccttgtcttt ggagtaatcc cttctgtatc ttttgaatat actttcggct tttcaggata 1440
   ctttgtgttc tctaaactta tttatggggc tgcattgggt gattatttga aggttattct 1500
   ttctgattca aatgttcctg gtgagggaga gcacaaaatc atgtcttata taagattgca 1560
   acgcaacctt tccggtttca atccaaacac acgccattgt ctgtatagtc tggtgagcgt 1620
   gtttctgatt tgaggcattt cagattttat tgttttatta ttgttttggc atctaactgc 1680
   taatcctatg aaaatatttt gtttgtggtc aggatgcaga tttgattatg ctgtcattag 1740
   ccacgcgtga ggttcacttt tccatattaa gagaggtttg cagctcatat cctaaagttg 1800
   gcatcatttc tccatcgatt attggtcagt cataaatttt atgtgcgttg attgtacaga 1860
   tagttacact tcccgggcag caggataaat gttttctctg cggtcaagct ggtcatcttg 1920
   cagctgaatg tcgtggcaag caaggagatg atgctttaga ttggcatgtg gtagatgata 1980
   ccccaattca caagaagaaa tatcaggttg aagaacagac atcaactgtt tccatttgtt 2040
   tccatctcgt cgtagtattt ctaagcagtt tccttatttg aacagttcct caacatctgg 2100
   gtgctgcgag aatatttgca atatgatctg gacattctga acccaccatt tgctatcgac 2160
   tttgaacgaa tagtggatga ctttgtgttc ctgtgttttt ttgttggcaa tgacttcctt 2220
   ccgcatatgc caacattgga aattagggag gtatgtaagt agcaagcaat ggttatatga 2280
   atgagtttgg gactgaacaa caggctgagt tccatttgtt tagtttttca gtgatcaact 2340
   cttgacaaac aattattatg agatgaggtg gtttttgttt tgtagcttgt ttgtggtgct 2400
   cagcttctag ttcgtttgga tgctttttag ctaatattaa gtgacattga tctgcttttg 2460
   tcctccaatc tctcctctac ttcttactat gaaatctttt cttcttttgt caatgaaaaa 2520
   gaatgatatt gatgttttta tttatattgg ccatagtttt gttttgaagt tctgttgctt 2580
   catggttttc actttgctgt tcttcctggt gctatctaca ctatatattg actcttcagt 2640
   tttatcaact tcatcatcac gtcctttcct gattcagggt gctataagct tactgatgca 2700
   tatttataga agggagttct ctgcaatggg tggttatctt actcttgcgg gtgaggtaat 2760
   ttctcttttt tatgttctct acaatacttg tgcctttgac tatactctgg aattaattta 2820
   tgtttctcat tatatgaaat ctgttaaaga tttggaatac gacattagtc taattttata 2880
   tcatgcatcc atttttttca tttatcacaa ttatgatcag gtggaaactg tgcgcaacac 2940
   ttgaaaattt agattaacat tgttaatgat ggtctgttca ttcttgttgt tttcctccca 3000
   cctttgctct aactgttcaa aaccaagttt tttttctcat tttagtttag ataacttctc 3060
   cttttaatca agtggattta ttcaataaaa tctgctaata aaaattaata aataatttac 3120
   aaagttatag acaagaactt ctgttagagt ttgtagtgag aggtttttgt tgtttatgag 3180
   caatgatgtt ttaagacttc tggtgagtgt agtgtttcta aaattactca gattcatcta 3240
   agttttctaa ctttggactt gatttcgaac tcatttagcc cttccatttc atcctatagc 3300
   catctgaatt aatgtggacc acttgagaca agaggatact agcgataaca tgggattttt 3360
   gctttatgtt tgctggaaga tatcattgta aatattagaa agttcataca ttatcaatgc 3420
   atttggttgt ctccaaatag aattagatct tagctgccca attccaccct ttcctctgta 3480
   tctcttttat cacacttatt tgtttggaaa ttgaattcag gcttggctca ttttatatgc 3540
   ttgaatttct agaatgactt gcttgctaaa catgtgctat aatattcagt gggttctagc 3600
   caagatatag acatgttata gcctgtcttg acccctagtt attctaaaat acttttttta 3660
   tttctccttt tctctcaaag aagttccttt ttggctctga aagtcttact tggttagata 3720
   atctttttat tagttattgc tctttgtcac ccatatcatg aaatagttac atctatctgc 3780
   tggatttgcc aaataaactt gttcttcagt gcttctttcc ccctttaaat caccatggct 3840
   tgtatttcat actttttatg ctgctttgtt ggttactttc agggaatgat tttgctccac 3900
   tattgcgagc atgagttcta atgaatgttc tgatttcaat tagcttctgt atacaggttt 3960
   tcctagataa agtagaacac tttattcagt gtgttggtgt ttacgaggag caaatctttc 4020
   agaagaggac tcgtatacaa caggtgcttt ctttttccat gcatggactt atagtcttca 4080
   gttctcatcc ttgggtctaa tgcaaacatc tgcatttatt tttactattt attgctggta 4140
   atagcatgtt cacttgttgg ctagaaactt tttatgttta gatgacaatt tatgtaaaat 4200
   cttggcaatg atttaactta tctgtaactg taaatttgct agatacagca ggtaggatgt 4260
   tggccctaaa tgaaacccca tatgctgctc ctgatttact ttattagtag aagtattagg 4320
   ttctgtaaac cattacaaaa tttaagaact ccgaaaattg gtctagtgat aagatttttg 4380
   ctttgtgtgc tagagctgaa gaaatttagg ggattccctt atcttgtttg gaatgcttag 4440
   ttctctaaga tttacgtcaa atttttatac acacattgca aagcagactg tattcttctt 4500
   tctatttttc atctgtggca aattttgagt tttggagtca tatacatact gtcttctctg 4560
   gttatctttt tgtttgtact tgtgtggata atccgttttc aggctttcga taataatgaa 4620
   gagatgaaac tcaaagcaag aagagagtcc tctgaagtga tacaagcacc agttgtagat 4680
   aaggtttgag cctataatat tttcatttga tgatatggat ttaaataaat gtgttgtgtt 4740
   ctgaatttgc ctgcgaaaaa ttgtgtagga tctatattaa acaaaaatga accaattatc 4800
   aattccttgc cagttagttt ttgacagaaa cctcaatcct ctctcatttt tttggacttc 4860
   cttctccatg tatagacgaa agctcgttag aggagttgat gcttaatatt gtttctaatt 4920
   tctcaataca ggatgttggt cataactctt cgagagaaag gaatataaag ctaattttgt 4980
   atgattccca tgttgtgttt ggtgacctaa ggatttttct tttcttaata gggtttttat 5040
   gatttaaaaa ataataattg cttttgcttt ctaactggta aatctgtaac ttacggttgt 5100
   gccttaagtt ccttgtcatc agaggtagat tttattttgt agataaacag cgaagattat 5160
   tttgattcag tagtgtctta atattgaaca tccatttact ctagcaggct cgttttctgt 5220
   ttgatatttg caagtttgct tatccatggc cttgcatctt gtgggccaac tgaggacaat 5280
   gtcagcccat ttgaaggcta gtctggttgt ccatttttcc tggtataaaa tttggttcat 5340
   ttttcttctt gtcaggttaa attaggggaa cctggataca aagagagata ctatgctgag 5400
   aaatttgagt tgtcaaatca agaggagatt gacaaagtta gaaaggaagt ggtaagttgt 5460
   tttttcctaa tcatgcaatt agcaccttag catatccata tagattatta tctgcatttt 5520
   ctcttggtag caaaattttg tgtgtacaca gtagttcatg acaaccgtct gtctccccca 5580
   accagtcctt cctctcctta agaaccccaa ggccccaaca tcaaagctgc aaggttgctt 5640
   gtacatattt tttttcctgt agttatatgg tcttcattct gagatccctt ggcatgatga 5700
   agtacattaa atgagtcttt caatcaccct ttttaattga tacaccatgg ggtggttttc 5760
   gttatagtta ttagtttatc tgggtgacct tgagtgctac ttgtctcact gttgtgctat 5820
   caacttgctg ttggcttctc atttccagtt cctaagagtt catttattag tttatctgga 5880
   tgatctttgg gtgtcattag tcttattgct gtgctatcag catgctattg cttcttgttt 5940
   ccagcctcaa agagttcaca tttttttcaa caaatgaata tgttcttcat tgtgattgta 6000
   tttgttttat tatgatctca agttccatta ttttctccaa cttttcattt tgagatttct 6060
   taatttatat tgactttagt ttgaaggtgt aatataataa ggtgaacttc acagatttac 6120
   atgaagaaaa taaggctgct tttgaaaaat caaggagttt tgcatgtttt tccttgaatg 6180
   ggaacactat tatgtactat cttaaccaaa ggaaaaaaaa agagaataat gaatagatag 6240
   agaaaaagtc agttgcttgt catcatataa caaatagaaa tctggtgtga cagaagattt 6300
   ggatgtagga agtatgccgg atatggtttc tggagtataa ttttttttta tgttgctaat 6360
   gattcctttt tggtctcccc tgtccctgtc acttgttttc tgtacaacca atcatgtatc 6420
   tgaagaaaaa agaaaataac atttgatgtt gttcatgcag gtattgaagt atgtggaagg 6480
   tttgtgttgg gtctgtcatt attacttcca aggtgtttgc tcttggcaat ggtcagttca 6540
   ataaaggcag aaactagaaa gtcattatac ttcttttcac tggctattta aattttttta 6600
   ttggagtttc aagcacaagc atgtgcccaa aaatacaaac ctatatatga atgcataata 6660
   tcttgaagct ctatacatga gtgaagaaat aatggaaaag aggacagtaa gttcattgaa 6720
   agttgagaac tatagttcta tccgtttcga aggtggtttt gaccctgagc tcttaagcat 6780
   atgacctgcc aaacaatcaa atttatatgg cttgtgacac ctaaatttaa ctttgccatc 6840
   actctgctca tcaaatggac aaaaataggc ttccattcac tctataagct tatatgatac 6900
   ggttccttac tgcaattagt tgaagttgga attctctgtt gtctgtttca ggttttatcc 6960
   gttccattat gctccatttg cttcagatct taaggatctg ggtgaggtgg aattaaactt 7020
   ttttattgga gagccattca aaccctttga tcaacttatg ggaaccctac cagctgcaag 7080
   gttttttttc tttctttttt atgtgagctc cttgcatttt caattttact ttggatactc 7140
   tgtctgatta ttctttctgt gctgtgtgtc atcctttggg catactacag ttctaatgct 7200
   ttgccaaaag aatacaggaa attgatgacc aatccatctt caccaattca tagattcttt 7260
   ccctcaggta aaagaattga ttgaaggata ttgattaatt tacctcaagg aagtgtttat 7320
   cttcttcaca agattaatgg ggtttcttta ttgtggacag attttgaaat tgacatgaat 7380
   ggcaagcgct ttgcttggca ggtaaattat tctgtattgg ttatcaatac atgtgtttta 7440
   actatacctt ttctttttca actgattata tttaccttat atttattgga agctagggta 7500
   ttgccaaatt gccattcatt gatgagagga aattgcttgc ccagacaaag aagcttgaaa 7560
   gcactttaac ggtatttagg ttttttaaag tagtatccaa taatttcatc catcgtaaac 7620
   tatattttaa tttctcttgt atgtgttggc cattttatta gaggctttca ctctgcacat 7680
   gttcttgtaa atataaactg ctacctgctc cttgctctct tggatgttga gctattcttt 7740
   tggaggcttt tactctgata ttttcagaga tttgaaaaag aaaagtatgc aattaatatt 7800
   tgggaaaaca tatttaatgg agaaatgcca ctgtctgaga gatcaatttt gtgcaaagag 7860
   aaggattcgt gatgtgatat tttaaaaaac ctgttgagat tcctagttat aatgcatttc 7920
   tactgattta gattctagga aacaatatca attcaaatac tgatttcgga gaaaaaatat 7980
   ttatttgtta caaatgcttt tgtcaccaaa ggttcctaaa gtttatttct tatgttactg 8040
   taatggaggt tgggctgtta acataaactc gtattcattt atcttattac ttgtgtaact 8100
   ttttaatagt caattatttg attgtgtgat ctgatagttt tttgctaagt tttgtatttg 8160
   aataaatcca tgttgacttt tgagtgattt ccataccaga tggaaaagtt ttttagtttc 8220
   tgatatatcc tcctgtactt aaatttctcc tgaaatttaa tttgtaagca ccaagggtag 8280
   tttagagttg gcagtcagtt gtgttcacac ccctataatg ggtttgatcc ttcagaagct 8340
   aaaccttgct gttttgtggc atatcagtct tctttttagt ttggatgtca agtatgttaa 8400
   tctttgattt gctatttgca ggaggaagag cagatcagga atagagtgat gcttgatttg 8460
   ctatatattc atcctgttca tccattagct caactagtta tttcatacta tcaacagaac 8520
   gatcatttat ctgaaggtga aagatttgct tgggaaattg acacaagagc caggttagtt 8580
   ggtcttagtg ttgattgatc ttttagtttg aagttgtcaa atcattattt atgtgtgctg 8640
   gctttattat ctcattttac actaaccttt tacagtgggg ggatgaatgg ttgtctttgg 8700
   ttatatgaga gaaatgtgcg gagaagtgtg gtcccctctc ctatacttgg attgccagcc 8760
   ttagagggta accaagtttt gtaagttcca actttttcca gtgctgtttt cataagctga 8820
   ttgatccttg gtgtaatgtt gtgtactttt tctatgcaga aatatcacat ttctaaatcc 8880
   taaaaaccgt gcacacatcc cagaaattcc tgagggtgta gtcatgcctg aaaaggtgtg 8940
   tgagctctag atgcctgtaa atttggttta ttttgttata cgcagaaaag caaggacatg 9000
   aaatttctgt caactaatga tgtggcagtg attcaattca tgcataaaat aatgtaatag 9060
   tgttattggg tttcatagtt tagagtttac tggagcagta aactgggatt cttctgtgaa 9120
   aattaattgg ccatgcctga aaaggtatgt gagctctaga tgcatgtttt tcgaaggtat 9180
   tgttgggcag taaactggag gcagtccttg tgttcaagga aattgagttt ggtcatggtt 9240
   tctgtttctg gaagttcccc cctgcatatg ggtggaagtg ctgatgcaat tgtaatataa 9300
   tggttatgtt aagtttgcag gtgcaaggat tctcatatgt tgtttccttg aaggcatttt 9360
   ctttggataa tgtgttgtgc cgtcacaggg tttgatgtta gttaatgatt tgatttcaca 9420
   aatgtccatg gctaatttga ggcatgttct tgctatttgc agatcgtaaa acctgtagat 9480
   cttaaaccat tccccacatt atggcatgaa gataatggcc ggagacagca aggcagggaa 9540
   aggtacgaat ggatttccac atctttgcaa tgattgattt gcattggttt gtaatgtttg 9600
   ggtggttagg ttgaatatgg gtgacatttg ttcatgacct gctattcttg acagacaatg 9660
   catctcgctc aatttatatg tggagaaagg aagggtttaa ttgatatcct cttttttttc 9720
   tctgaaaaac atagattgta gggtttagaa cttactgctt cgtcttctct taaatagcct 9780
   aattcacatg tgctgtctct gtattacagg ccacaagtac aacgagcaat agctggcccc 9840
   ttcctggggg atgcagctca tcgtcttgtc aagaacaccc ttaatatcaa accaaatggt 9900
   tcatcctcta gagtgtttga ccagcaacta tatcataata tccccggcaa ttacacattc 9960
   tacagaccaa gaccagctgg accagctggt tatggaaggg gctattggga tgatccaaac 10020
   tatcattatg cacagcacag taaccagcaa ggcctgatga gtaatcctag gtatcgatct 10080
   ttgtcaaatg gtgtgcaaag caaccgacac aactttagga ctcaagacgg agtacagtac 10140
   catcaacagt atcataactt gagtactggg gtgtctgctc tgactgtaga agaaaatatc 10200
   agaagcaggg cacctgcagt gatctcacct cgaatgccga atccaggaaa cactccaaac 10260
   ttacaaaatc aggccgagca gaacacaggt ctactttcat caccacccac caattggatc 10320
   aacaaaacag ctgcaggaaa cactggaatg tacttcaaac aaaaatcaac atcgataggt    10380
   ccaaatgaga agcaggtgaa gcaggtttat caggttaaga cacaagtagc tcaggagacg    10440
   ccagatattc aagctcagga gacgccagat ttaaaacagc agtaacattt tatatttgaa    10500
   gaatcttgca tgtttttgcc acactcacat tgcagtggca atgcatgttc atttgaatct    10560
   ttgcataagc gcaagttttt ggcgaattta ctcggtttgg gcaacagtca cagaaactat    10620
   tcctggatgg tatgcagctg caagagctcg tggatgttaa aagagtataa tatatgaaca    10680
   gtggcctttt tgagatccac ttttgatttg gtgcgagtta gcatactgca tctcacctca    10740
   ttcatgttgc agctgctttt ttccagattt tcctatttgt ttaccagatt gtcgtagaaa    10800
   actgtatcat acaaatgttt ggttgtaatc gggcatctat ctcagaaatg ttgtcagtat    10860
   gtaccgtatt ttggatgatg taattcttga tgtaattcta tgcagtgtcc tttttagtga    10920
   ctcggaactt tttgcttcag gatttctgtg caaat                               10955
<210> 13
   <211> 1240
   <212> PRT
   <213> Zea mays
<400> 13
   Asp Gly Ser Pro Thr Arg Pro Pro Ala Arg Thr His Ala Arg Gly His
   1               5                   10                  15
   Arg Pro Ser Phe Pro Phe Pro Arg Thr His Ala Pro His Leu Ser Ser
               20                  25                  30
   Leu Arg Pro Tyr Arg Ser Asn Gln Gly Gly Ser Asn Gln Arg Thr Pro
           35                  40                  45
   Leu Arg Arg Glu Leu Ala Glu Ala Pro Pro His Glu Arg Glu Ser Arg
       50                  55                  60
   Ala Arg Lys Arg Ser Gly Gly Arg Ala Gly Arg Ser Glu Met Gly Val
   65                  70                  75                  80
   Pro Ala Phe Tyr Arg Trp Leu Ala Asp Arg Tyr Pro Leu Thr Val Ser
                   85                  90                  95
   Asp Ala Glu Glu Glu Glu Pro Val Glu Leu Glu Pro Gly Ala Phe Val
               100                 105                 110
   Pro Val Asp Leu Arg Arg Pro Asn Pro Asn Gly Leu Glu Phe Asp Asn
           115                 120                 125
   Leu Tyr Leu Asp Met Asn Gly Ile Ile His Pro Cys Phe His Pro Glu
           130                 135                 140
   Gly Arg Pro Ala Pro Thr Thr Tyr Asp Glu Val Phe Lys Ser Ile Phe
   145                 150                 155                 160
   Asp Tyr Ile Asp His Leu Phe Gly Leu Ile Arg Pro Arg Lys Leu Leu
                   165                 170                 175
   Tyr Met Ala Ile Asp Gly Val Ala Pro Arg Ala Lys Met Asn Gln Gln
               180                 185                 190
   Arg Ser Arg Arg Phe Arg Ala Ala Lys Asp Ala Ala Asp Ala Ala Ala
           195                 200                 205
   Glu Glu Glu Arg Leu Arg Lys Glu Phe Glu Ala Glu Gly Arg Thr Leu
       210                 215                 220
   Ala Gln Lys Glu Lys Ser Glu Ala Ile Asp Ser Asn Val Ile Thr Pro
   225                 230                 235                 240
   Gly Thr Glu Phe Met Phe Val Leu Ser Thr Ala Leu Gln Tyr Tyr Ile
                   245                 250                 255
   Gln Leu Arg Leu Asn His Thr Leu Gly Trp Gln Ser Val Lys Ile Ile
               260                 265                 270
   Leu Ser Asp Ser Asn Val Pro Gly Glu Gly Glu His Lys Ile Met Ser
           275                 280                 285
   Tyr Ile Arg Leu Gln Arg Asn Leu Pro Gly Phe Asp Pro Asn Thr Arg
       290                 295                 300
   His Cys Leu Tyr Gly Leu Asp Ala Asp Leu Ile Met Leu Ala Leu Ala
   305                 310                 315                 320
   Thr His Glu Val His Phe Ser Ile Leu Arg Glu Val Ile Ser Met Pro
                   325                 330                 335
   Gly Gln His Glu Lys Cys Phe Leu Cys Gly Gln Val Gly His Leu Ala
               340                 345                 350
   Ala Glu Cys Arg Gly Pro Ser Gln Pro Asp Asn Ser Val Glu Leu Pro
           355                 360                 365
   Pro Ile His Lys Lys Lys Tyr Gln Phe Leu Asn Ile Trp Val Leu Arg
       370                 375                 380
   Glu Tyr Leu Ala Lys Asp Leu Glu Ile Ile Asp Ala Pro Phe Lys Ile
   385 390 395 400
   Asn Phe Glu Arg Leu Ile Asp Asp Phe Val Phe Met Cys Phe Phe Val
   405 410 415
   Gly Asn Asp Phe Leu Pro His Met Pro Thr Leu Glu Ile Arg Glu Gly
   420 425 430
   Ala Ile Asn Leu Leu Met Ser Ile Tyr Arg Ser Glu Phe Thr Ser Met
   435 440 445
   Gly Gly Tyr Leu Thr Asp Val Gly Glu Val Ile Leu Asp Arg Val Glu
   450 455 460
   His Phe Ile Gln Ser Val Ala Val Asn Glu Glu Gln Ile Phe Gln Lys
   465 470 475 480
   Arg Ala Arg Ile Gln Gln Ala Arg Glu Asn Asn Glu Glu Lys His Lys
   485 490 495
   Met Gln Arg Glu Asn Ser Glu Glu Asp Gln Tyr Val Asp Lys Val Lys
   500 505 510
   Leu Gly Glu Pro Gly Tyr Arg Glu Arg Tyr Tyr Ala Asp Lys Phe Lys
   515 520 525
   Glu Glu Ala Glu Ser Lys Pro Ile Val Gln Val Arg Arg Asp Val Val
   530 535 540
   Gln Lys Tyr Val Glu Gly Leu Cys Trp Val Met Arg Tyr Tyr Tyr Gln
   545 550 555 560
   Gly Val Cys Ser Trp Gln Trp Phe Tyr Pro Tyr His Tyr Ala Pro Phe
   565 570 575
   Ala Ser Asp Leu Lys Gly Leu Ala Glu Leu Glu Ile Thr Phe Phe Leu
   580 585 590
   Gly Gln Pro Phe Lys Pro Phe Asp Gln Leu Met Gly Thr Leu Pro Ala
   595 600 605
   Ala Ser Ser Asn Ala Leu Pro Lys Tyr Tyr Gly Asp Leu Met Ser Asp
   610 615 620
   Pro Asp Ser Pro Leu Lys Ser Phe Tyr Pro Lys Asp Phe Glu Ile Asp
   625 630 635 640
   Met Asn Gly Lys Arg Phe Ala Trp Gln Gly Val Ala Lys Leu Pro Phe
                   645                 650                 655
   Ile Asp Glu Arg Arg Leu Leu Ala Glu Thr Arg Lys Leu Glu Asp Thr
               660                 665                 670
   Leu Thr Glu Glu Glu Lys Phe Arg Asn Arg Thr Met Leu Asp Ile Ile
           675                 680                 685
   Tyr Val Arg Asp Thr His Pro Leu Ile Thr Gln Ile Ile Phe Leu Tyr
       690                 695                 700
   Gln Asn Tyr Tyr His Leu Ser Arg Thr Asp Pro Tyr Val Ile Pro Ile
   705                 710                 715                 720
   Glu Pro Ala Ala Ser Gly Gly Met Asn Gly Phe Leu Cys Leu Ser Glu
                   725                 730                 735
   Arg Asn Trp Tyr Ser Val Thr Val Thr Ser Pro Val Lys Gly Phe Asn
               740                 745                 750
   Gly Ile Ala Gln Asn Arg Val Leu Asn Ala Ala Tyr Leu Asn Pro Gln
           755                 760                 765
   Tyr His Lys His Ile Pro Glu Pro Pro Ala Gly Val Ile Ile Pro Ala
       770                 775                 780
   Lys Ile Leu Lys Pro Ser Asp Phe Lys Pro Phe Pro Val Leu Trp His
   785                 790                 795                 800
   Gln Asp Asn Ser Arg Arg Gln Val Arg Glu Arg Pro Gln Val Ser Gly
                   805                 810                 815
   Ala Leu Ser Gly Ser Leu Leu Gly Glu Ala Ala His Arg Leu Val Lys
               820                 825                 830
   Asn Ser Leu Gln Ile Arg Ser Gly Asn Ala Ala Gly Leu Leu Tyr Met
           835                 840                 845
   Pro Tyr Arg Gly Ala Pro Tyr Gly Pro Gly Asn Arg Pro Arg Pro Ala
       850                 855                 860
   Gly Pro Leu Gly Tyr Glu Arg Gly Phe Val Asp Asn Pro Tyr His Ala
   865                 870                 875                 880
   His Met Ser Arg Ser Val Pro Asn Pro His Ala Gln Phe Phe Gly Asp
                   885                 890                 895
   Ser Gln Ala Asn Arg Gln Pro Met Arg Ile Leu Glu Arg Pro Asp Ser
   900 905 910
   Arg Ser His Asp Ala Gly Ile Arg Ala Ser Met Ser Lys Leu Thr Ile
   915 920 925
   Gln Glu Gly Pro Arg Pro His Gln Asn Asn Arg Met Gln Asn Ser Gly
   930 935 940
   Tyr Trp Pro Asn Gln Pro His Pro Thr His Phe Thr Gly Pro Pro Ala
   945 950 955 960
   Gln Arg Pro Met Gln Asn Ile Ser Phe Thr Pro Gln Arg Pro Phe Gln
   965 970 975
   Thr Gly Gly Phe Pro Gln Gln Arg Pro Val Asn Gly Ala Pro Pro Pro
   980 985 990
   Leu Pro Pro Ser Asn Trp Ile Gly Lys Gln Pro Ser Gly Cys His Met
   995 1000 1005
   Ser Val Ser Pro Ala Lys Leu Asp Pro Arg Thr Ala Pro Asp Arg
   1010 1015 1020
   Gln Pro Lys Gln Asp Asn Pro Arg Ser Gln Gln Asp Lys Arg Gln
   1025 1030 1035
   Gln Ala Thr Lys Val Tyr Arg Val Lys Thr Gln Ala Thr Asn Val
   1040 1045 1050
   Asn Gly Leu Ser Glu Pro Gly Lys Gln Glu Glu Pro Glu Gly Leu
   1055 1060 1065
   Gly Cys Cys Lys Ser Thr Arg Gln Val Cys Ala Trp Arg Glu Tyr
   1070 1075 1080
   Ala Gly Ser Trp Gln Gln Arg Val Ser Met Tyr Pro Ser His Asn
   1085 1090 1095
   Leu Pro Phe Asp Val Glu Leu Phe Val Val Leu Pro Gly Lys Glu
   1100 1105 1110
   Ser Ala Lys Phe Val Arg Gly Phe Arg His Ser Phe Cys Leu Val
   1115 1120 1125
   Asp Arg Thr Trp Tyr Gly His Ser Leu Leu Val Val Lys Leu Leu
           1130                1135                1140
   Val Gly Leu Lys Leu Phe Cys Ala Gly Leu Leu His Ile Gln His
           1145                1150                1155
   Arg Pro Val Arg Arg Leu Ala Gln Leu Pro Cys Ser Cys Ser Ile
           1160                1165                1170
   Ser Trp Lys Leu Gly Ala Asp Val Leu Val Val Phe Lys Leu Ser
           1175                1180                1185
   Ser Leu Cys Leu Glu Arg Arg Lys His Ser Glu Phe Phe Ile Val
           1190                1195                1200
   Pro Leu Asn Gln Val Glu Ser Leu Thr Leu Lys Arg Thr Cys Ser
           1205                1210                1215
   Ser Arg Ala Arg Val Pro Leu Gly His Gly Ile Ile Tyr Ile Phe
           1220                1225                1230
   Phe Phe Ser Gly Met Gln Thr
           1235                1240
<210> 14
   <211> 3754
   <212> DNA
   <213> Zea mays
<400> 14
   agacggaagc ccaacccgcc cgcctgcccg cacacacgcg cgtggccatc gcccctcctt 60
   ccccttcccc cgcacacacg cgcctcacct ttcgtccctt cgcccctaca gatcgaacca 120
   aggaggcagc aaccagcgca cgccactgcg gcgctaggag ctcgcagaag cgccgcccca 180
   cgagagggag tccagagcaa ggaagagatc cggtggcaga gcaggtcggt cggagatggg 240
   agtaccggcg ttctaccggt ggctggcgga ccgctacccg ctgacggtgt ctgacgcgga 300
   ggaggaggag cccgtggagc tcgagcccgg cgccttcgtc cccgttgacc tccgccgccc 360
   caatcccaac ggcctcgagt ttgacaacct ctacctcgac atgaatggta tcatccaccc 420
   atgcttccac cccgagggcc ggccggcgcc caccacctac gacgaggtgt tcaagtcgat 480
   ttttgattac atcgaccacc tcttcggcct catccgcccc aggaagcttc tctacatggc 540
   cattgatggt gttgctccaa gggcaaaaat gaaccagcag aggtccaggc gattccgggc 600
   tgctaaggac gcggctgatg cggcagcgga ggaagagagg ctgaggaagg agtttgaggc 660
   agagggtagg actctagccc agaaagagaa gtcggaggca attgactcca atgtcattac 720
   tccagggacg gagttcatgt ttgtactatc tacggcgctt cagtactaca tacagctgag 780
   gctgaatcac acgcttggat ggcagtctgt aaagataata ctgtctgatt ccaatgtccc 840
   tggagaggga gagcacaaaa ttatgtcata catccgcctg cagcgcaatc ttcctggctt 900
   tgatcccaat acacgccatt gcttatatgg cctggacgct gatttgatta tgcttgctct 960
   agcgactcac gaggttcact tctcaatttt aagagaggtg atctccatgc cagggcaaca 1020
   tgaaaaatgt tttctttgtg gtcaagttgg gcatttggct gctgaatgca gaggtcctag 1080
   tcagcctgat aattctgtgg agctacctcc tattcataaa aagaaatacc agtttcttaa 1140
   catatgggtg ttgcgtgaat acctggcaaa ggatttggaa attatagatg ctcccttcaa 1200
   gataaacttt gaacgtctta ttgatgattt tgtgtttatg tgtttctttg ttggcaatga 1260
   ctttcttcct catatgccaa ctttggaaat tcgtgagggt gccattaatc ttctcatgag 1320
   tatctacagg tcggagttca catcaatggg tggttaccta actgatgtgg gtgaggttat 1380
   attggaccga gtggaacatt tcatccagtc tgttgcggtc aatgaagaac agattttcca 1440
   gaaacgggca cgcattcaac aggcacgtga gaataatgaa gagaaacata aaatgcagag 1500
   agagaattct gaggaggatc aatatgtgga taaggtgaag ttaggagagc caggatacag 1560
   ggagcgttat tatgctgata agtttaaaga agaggcagaa tcaaaaccca ttgttcaagt 1620
   tcggagagat gttgtccaga aatacgtgga aggtctttgt tgggttatga gatactacta 1680
   tcaaggtgtt tgttcatggc agtggtttta tccctatcat tatgcgcctt tcgcatctga 1740
   tctaaaaggc ttggctgaat tggaaataac cttttttttg ggtcaacctt tcaagccatt 1800
   tgatcaacta atgggaaccc taccagctgc aagctccaat gcactgccaa aatactatgg 1860
   ggatttgatg agtgatccag attcaccgtt gaagtctttc tacccaaaag attttgagat 1920
   agacatgaat gggaaacgtt ttgcatggca gggtgttgca aaattgcctt ttattgatga 1980
   aaggcgtctg cttgcggaaa caagaaaact tgaagatact ttgacagaag aagagaaatt 2040
   caggaatagg actatgcttg acataattta cgttagagat actcatccat tgatcactca 2100
   gattatcttc ctgtatcaga actattacca tctatcaaga acagaccctt atgttattcc 2160
   cattgagcct gctgctagtg gtggaatgaa tggattccta tgtttatctg aaaggaactg 2220
   gtatagtgtc actgtcactt ctccagttaa ggggttcaat ggcattgctc agaacagagt 2280
   tttgaatgct gcctatctca atcctcagta tcacaaacac atcccagagc ctccagcggg 2340
   ggtcatcata cctgcgaaga tactgaagcc tagtgatttc aaaccctttc ctgtgttgtg 2400
   gcatcaagat aatagtcgtc gacaagtaag agaaaggcct caggtttctg gagccctgtc 2460
   cggttctctc ttaggagaag ctgcacaccg actggtgaaa aactccctcc agatcagatc 2520
   tggcaatgct gctgggttac tttatatgcc atacagaggt gcaccctatg gccctggaaa 2580
   cagaccaagg cctgctgggc cattggggta tgagaggggt tttgtggata atccatacca 2640
   tgcacacatg tctagaagtg ttccaaaccc tcacgctcaa ttctttggtg attctcaagc 2700
   taacagacag cccatgcgga tactagaacg gccagactcc cgaagtcatg atgctggtat 2760
   ccgtgcatca atgtctaaac tgacaatcca agaagggcca aggcctcatc aaaataacag 2820
   gatgcagaat tccgggtact ggcccaatca accacatcct actcacttta ctggaccccc 2880
   agcccagcgg cctatgcaga acatcagttt tacacctcag cgacctttcc aaaccggggg 2940
   atttccgcag cagagacctg taaatggggc tccaccacca ttacctccca gtaactggat 3000
   tggtaagcaa ccaagtggat gccacatgag tgtatcacct gcaaaactcg atccaaggac 3060
   agctccagac agacagccca agcaggataa cccaagatct caacaggata agaggcagca 3120
   ggcgaccaag gtataccgtg tcaaaactca agctactaat gttaatggtt tgtcagagcc 3180
   aggtaaacag gaagaacctg aagggtaact gggttgctgc aaatccacac gccaagttta 3240
   atgtgcgtgg agagaatacg ctggctcttg gtgatagcag cagagggttt ccatgtatcc 3300
   ctcccacaat ttacctttcg atgttgaatt gtttgttgta ttacccggaa aggagtcggc 3360
   taaattttag gtcaggggat ttagacacag cttttgttta gttgatagga catggtatgg 3420
   gcattcactg cttgttgtaa agctgttagt tggatagctg aaactgtttt gtgctggctg 3480
   actgctgcat attcaacata gacccgtgcg gaggctggct caactgccct gctcctgctc 3540
   tatctcatgg aagttggggg cagatgtgct tgtggtcttc aaactgagca gtttgtgtct 3600
   ctgagaaagg aggaagcatt ccgaattttt cattgtaccg ttgaaccaag ttgagtcttt 3660
   aacactgaaa agaacatgtt cctctcgagc ttaacgagtg ccataattag gacatggaat 3720
   aatatatata tttttctttt ctgggatgca aaca 3754
<210> 15
   <211> 16
   <212> PRT
   <213> Arabidopsis thaliana
<400> 15
   Asp Gly Val Ala Pro Arg Ala Lys Met Asn Gln Gln Arg Ser Arg Arg
   1 5 10 15
<210> 16
   <211> 9
   <212> PRT
   <213> Arabidopsis thaliana
<400> 16
   Gly Leu Asp Ala Asp Leu Ile Met Leu
   1 5
<210> 17
   <211> 5
   <212> PRT
   <213> Zea mays
<400> 17
   Gly Asp Val Ala Pro
   1               5
<210> 18
   <211> 19
   <212> DNA
   <213> Zea mays
<400> 18
   gatggtgttg ctccaaggg 19